# EUROPÄISCHE PATENTSCHRIFT

(11) **EP 0 983 255 B1**
(45) Veröffentlichungstag und Bekanntmachung des Hinweises auf die Patenterteilung: **29.01.2003**
(21) Anmeldenummer: 98922776.4
(22) Anmeldetag: 24.04.1998
(51) Int. Cl.: C07D 305/12, C07D 305/10, C07D 305/08, C07D 303/32, C07F 9/655, C07D 493/10, C07F 7/08, A01N 43/04

(54) **SUBSTITUIERTE 2-BENZOYL-CYCLOHEXAN-1,3-DIONE**
SUBSTITUTED 2-BENZOYL-CYCLOHEXANE-1,3-DIONES
2-BENZOYLE-CYCLOHEXANE-1,3-DIONES SUBSTITUEES

(30) Priorität: 07.05.1997 DE 19726711
(43) Veröffentlichungstag der Anmeldung: 08.03.2000
(73) Patentinhaber: BASF AKTIENGESELLSCHAFT, 67056 Ludwigshafen (DE)
(72) Erfinder: ENGEL, Stefan, D-55286 Wörrstadt (DE); BAUMANN, Ernst, D-67373 Dudenhofen (DE); VON DEYN, Wolfgang, D-67435 Neustadt (DE); HILL, Regina, Luise, D-67346 Speyer (DE); KARDORFF, Uwe, D-68159 Mannheim (DE); MAYER, Guido, D-67433 Neustadt (DE); OTTEN, Martina, D-67069 Ludwigshafen (DE); RHEINHEIMER, Joachim, D-67063 Ludwigshafen (DE); WAGNER, Oliver, D-67061 Ludwigshafen (DE); WITSCHEL, Matthias, D-67061 Ludwigshafen (DE); MISSLITZ, Ulf, D-67433 Neustadt (DE); WALTER, Helmut, D-67283 Obrigheim (DE); WESTPHALEN, Karl-Otto, D-67346 Speyer (DE)
(86) Internationale Anmeldenummer: EP9802448
(87) Internationale Veröffentlichungsnummer: WO98050377

(56) Entgegenhaltungen:
- EP-A- 0 278 742
- US-A- 5 451 578

## Beschreibung

Die vorliegende Erfindung betrifft substituierte 2-Benzoyl-cyclohexan-1,3-dione der Formel I 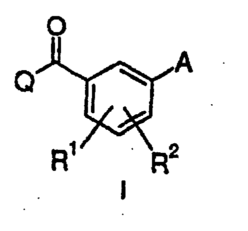 in der die Substituenten folgende Bedeutung haben:
- R¹, R²: Wasserstoff, Mercapto, Nitro, Halogen, Cyano, Rhodano, C₁-C₆-Alkyl, C₁-C₆-Halogenalkyl, C₁-C₆-Alkoxy, C₁-C₆-Alkyl, C₂-C₆-Alkenyl, C₂-C₆-Alkinyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ oder -NR¹⁰COR¹⁰;
- Q: ein gegebenenfalls substituierter, in 2-Stellung verknüpf ter Cyclohexan-1,3-dion-Ring;
- A: eine Gruppe der Formel IIa, IIb oder III 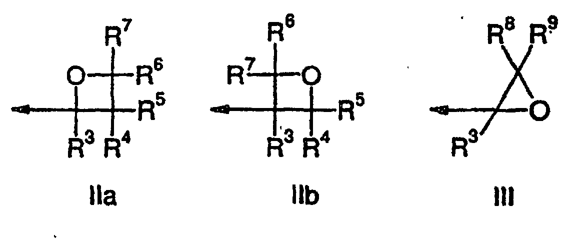 in der die Substituenten folgende Bedeutung haben:
- R³: Wasserstoff, C₁-C₆-Alkyl oder Rxenyl,
wobei der genannte Alkyl und Phenylrest partiell oder vollständig halogeniert sein kann und/oder eine bis drei der folgenden Gruppen tragen kann: Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴ - R⁷: können gleich oder verschieden sein und stehen unabhängig voneinander für:
Wasserstoff, Hydroxy, Mercapto, Amino, Halogen, C₁-C₆-Alkyl, C₃-C₆-Cycloalkyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰; wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig ha- logeniert sein können und/oder eine bis drei der folgen- den Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR^{10,} -CONR⁸R^{10,} C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴, R⁵: können zusammen eine gegebenenfalls durch ein Stickstoffoder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ stehen kann;
- R⁶, R⁷: können zusammen eine gegebenenfalls durch ein Stickstoffoder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ stehen kann;
- n: null, eins, zwei;
- R⁵, R⁶: können darüber hinaus, wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen;
- R⁸, R⁹: können gleich oder verschieden sein und stehen unabhängig voneinander für: Wasserstoff, Nitro, Halogen, Cyano, C₁-C₆-Alkyl, C₃-C₆-Cycloalkyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünfoder sechsgliedriges Hetaryl, wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/ oder eine bis drei der folgenden Gruppen tragen können: Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁸, R⁹: können darüber hinaus zusammen eine gegebenenfalls durch ein Stickstoff- oder ein Sauerstoffatom ein- oder zweifach unterbrochene C₂-C₅-Alkylen- oder C₂-C₅-Alkenylenkette bilden;
- R¹⁰: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/oder eine bis drei der folgenden Gruppen tragen können: Hydroxy, Mercapto, Amino, Cyano, R¹⁰, -OR¹⁰, -SR¹⁰, -NR³R¹⁰, =NOR¹⁰, -OCOR¹⁰. -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus EP-A 278 742, EP-A 298 680, EP-A 320 864 und WO 96/14285 sind 2-Benzoylcyclohexan-1,3-dione bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 2-Benzoyl-cyclohexan-1,3-dione der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon umfaßt.

Die Verbindungen der Formel I können ebenso je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschstenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Hervorzuheben sind die erfindungsgemäßen Verbindungen der Formel I, wobei die Variable Q einen in 2-Stellung verknüpften Cyclohexan-1,3-dionring der Formel IV darstellt, 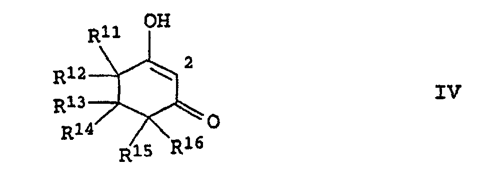 wobei IV auch stellvertretend für die tautomeren Formeln IV' und IV" steht, 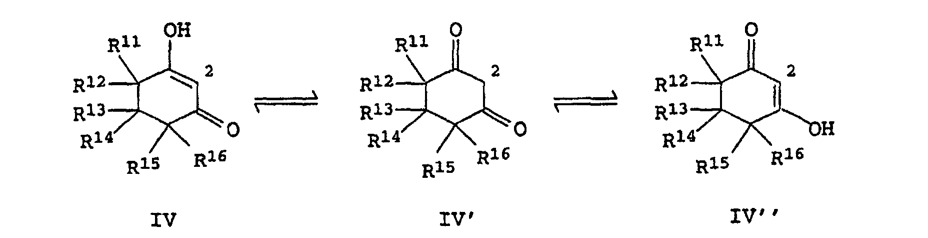 wobei
- R¹¹, R¹², R¹⁴ und R¹⁶: für Wasserstoff odet C₁-C₄-Alkyl stehen;
- R¹³: für Wasserstoff, C₁-C₄-Alkyl oder C₃-C₄-Cycloalkyl steht, wobei die beiden letztgenannten Gruppen einen bis drei der folgenden Substituenten tragen können: Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch ein bis drei C₁-C₄-Alkylreste substituiert sein können;
- R¹⁵: für Wasserstoff, C₁-C₄-Alkyl oder C₁-C₆-Alkoxycarbonyl steht;
oder
- R¹³ und R¹⁶: gemeinsam eine π-Bindung oder einen drei- bis sechsgliedrigen carbocyclischen Ring bilden;
oder
die CR¹³R¹⁴-Einheit durch C=O ersetzt sein kann.

### Verfahren A:

Umsetzungen von Cyclohexan-1,3-dion der Formel IV mit einer aktivierten Carbonsäure Va oder einer Carbonsäure Vb, die vorzugsweise in situ aktiviert wird, zu dem Acylierungsprodukt VII und anschließende Umlagerung zu den erfindungsgemäßen Verbindungen der Formel I.

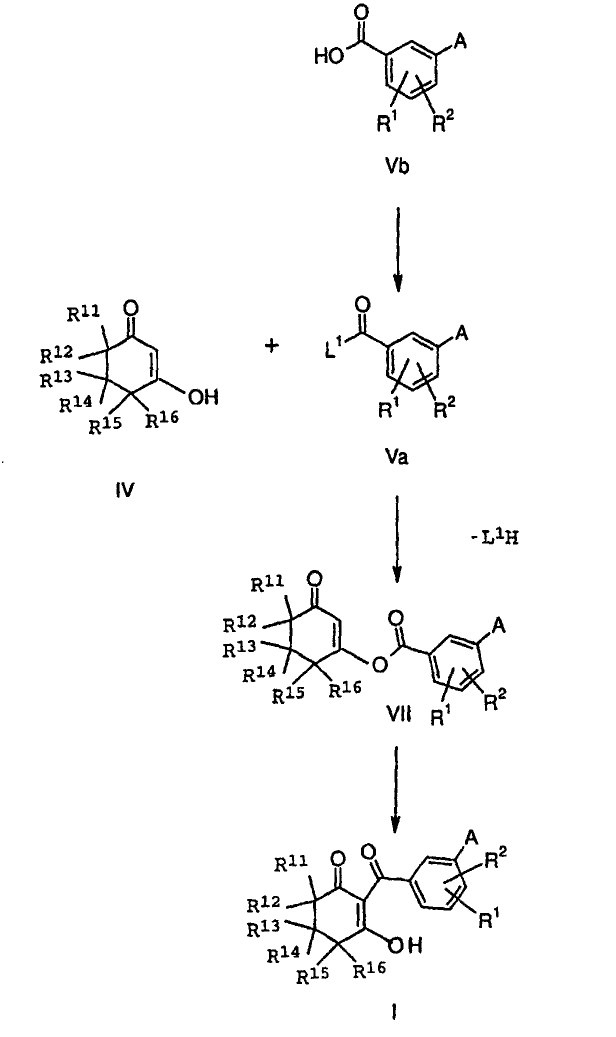

L¹ steht für eine nucleophil austauschbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäure - ester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel VII vorzugsweise durch Chromatographie gereinigt. Es ist aber auch möglich, den rohen Enolester der Formel VII ohne weitere Reinigung zur Umlagerung einzusetzen.

Die Umlagerung der Enolester der Formel VII zu den Verbindungen der Formel I erfolgt zweckmäßigerweise bei Temperaturen von 20 bis 40°C in einem Lösungsmittel und in Gegenwart einer Base sowie gegebenenfalls in Gegenwart einer Cyanoverbindung.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische cyanide, wie Natriumcyanid, Kaliumcyanid und organische Cyanoverbindungen, wie Acetoncyanhydrin, Trimethylsilycyanid in Betracht. Sie werden üblicherweise in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 oder US 4 643 757 genannt).

Die Benzoesäuren der Formel V sind neu, 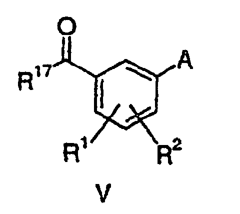 wobei die Substituenten folgende Bedeutung haben:
- R¹, R²: Wasserstoff, Mercapto, Nitro, Halogen, Cyano, Rhodano, C₁-C₆-Alkyl, C₁-C₆-Halogenalkyl, C₁-C₆-Alkoxy C₁-C₆-Alkyl, C₂-C₆-Alkenyl, C₂-C₆-Alkinyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ oder -NR¹⁰COR¹⁰;
- A: eine Gruppe der Formel IIa, IIb oder III 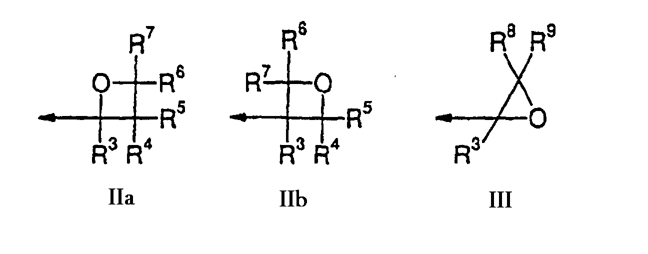
in der die Variablen folgende Bedeutung haben:
- R³: Wasserstoff, C₁-C₆-Alkyl oder Phenyl,
wobei der genannte Alkyl und Phenylrest partiell oder vollständig halogeniert sein kann und/oder eine bis drei der folgenden Gruppen tragen kann:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴- R⁷: können gleich oder verschieden sein und stehen unabhängig voneinander für:
Wasserstoff, Hydroxy, Mercapto, Amino, Halogen, C₁-C₆-Alkyl, C₃-C₆-Cycloalkyl, C₄-C₆-Cycloalkenyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰,
wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/ oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴, R⁵: können zusammen eine gegebenenfalls durch ein Stickstoffoder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰, NNR³R¹⁰ oder NOR¹⁰ stehen kann;
- R⁶, R⁷: können zusammen eine gegebenenfalls durch ein Stickstoffoder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰, NNR³R¹⁰ stehen kann;
- n: null, eins, zwei;
- R⁵, R⁶: können darüber hinaus, wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen;
- R⁸, R⁹: können gleich oder verschieden sein und stehen unabhängig voneinander für:
Wasserstoff, Nitro, Halogen, Cyano, C₁-C₆-Alkyl, C₃-C₆-Cycloalkyl, C₄-C₆-Cycloalkenyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünf- oder sechsgliedriges Hetaryl,
wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁸, R⁹: können darüber hinaus zusammen eine gegebenenfalls durch ein Stickstoff- oder ein Sauerstoffatom ein- oder zweifach unterbrochene C₂-C₅-Alkylen- oder C₂-C₅-Alkenylenkette bilden;
- R¹⁰: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl;
wobei die genannten Alkylreste partiell oder vollständig halogeniert sein können und/oder eine bis drei der folgenden Gruppen tragen können: Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R¹⁷: Hydroxy oder ein hydrolysierbarer Rest.

Beispiele für hydrolysierbare Reste sind Alkoxy-, Phenoxy-, Alkylthio-, Phenylthioreste, die substituiert sein können, Halogenide, Hetarylreste, die über Stickstoff gebunden sind, Amino-, Iminoreste, die substituiert sein können, etc.

Bevorzugt sind Benzoesäurehalogenide Va mit L = Halogen (=̂ V mit R¹⁷ = Halogen), 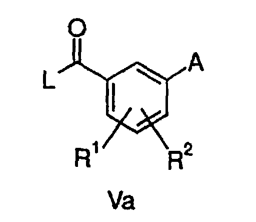 wobei die Variablen R¹, R², und A die unter Formel V genannte Bedeutung haben und
- L: Halogen, insbesondere Chlor oder Brom, bedeuten.

Ebenso bevorzugt sind Benzoesäuren der Formel Vb (=̂ V mit R¹⁷ = Hydroxy), 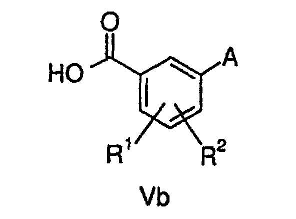 wobei wobei die Variablen R¹, R², und A die unter Formel V genannte Bedeutung haben.

Ebenso bevorzugt sind Benzoesäureester der Formel Vc (=̂ V mit R¹⁷ = C₁-C₆-Alkoxy), 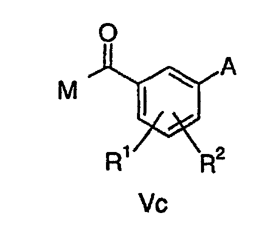 wobei die Variablen R¹, R², und A die unter Formel V genannte Bedeutung haben und
- M: C₁-C₆-Alkoxy
bedeutet.

Die Verbindungen der Formel Va (mit L = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel Vb mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel Vb können u.a. durch Verseifung der Benzoesäureester der Formel Vc (mit M = C₁-C₆-Alkoxy) erhalten werden.

Die erfindungsgemäßen Benzoesäureester der Formel Vc sind nach verschiedenen literaturbekannten Methoden (z.B. a. G. Dittus in Houben-Weyl, Methoden der Organischen Chemie, Band VI/3, Sauerstoff-Verbindungen I, 4. Aufl., S. 493 ff., Georg Thieme Verlag, 1965; b. T. L. Gilchrist, Heterocyclenchemie, 2. Aufl., Verlag Chemie, 1995) darstellbar, wie in den nachfolgenden Beispielen illustriert.

### Verfahren A:

Cyclisierung von 1,3-Halogenhydrinen der Formel VIIIa oder VIIIb unter alkalischen Reaktionsbedingungen zu den erfindungsgemäßen Benzoesäureestern der Formel Vc, in der die Variablen R¹, R² und M die unter Formel V genannte Bedeutung haben, A für eine Gruppe der Formel IIIa oder IIIb und L² für eine nucleophil austauschbare Abgangsgruppe, vorzugsweise Iod, Brom oder Chlor steht.

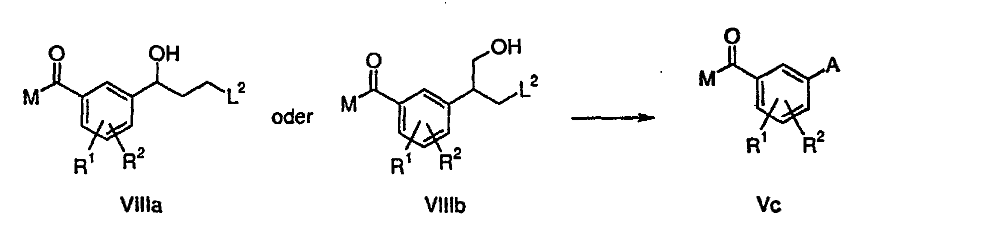

Als Abspaltungsmittel werden vor allem Alkali- und Erdalkalihydroxide, z.B. Kaliumhydroxid oder Natriumhydroxid oder organische Basen, z.B. Alkoholate, wie z.B. Natriummethanolat oder sekundäre Amine, wie z.B. Diethylamin verwendet.

Die Abspaltung von Halogenwasserstoff kann bereits durch alkalisch reagierende Salze, z.B. Kaliumfluorid erfolgen (E. Gryszkiewics-Trochimowski, O. Gryszkiewics-Trochimowski, Bulletin de la Société Chimique de France, Mémoires 123 (1953)).

Die Abspaltungsmittel können sowohl in Lösung als auch in Substanz, vorzugsweise in Lösung, z.B. methanolische Natriummethanolat-Lösung eingesetzt werden.

Die Durchführung der Cyclisierung erfolgt in indifferenten Lösungsmitteln, z.B. in Alkoholen, wie z.B. Methanol oder Ethanol.

### Verfahren B:

Photochemische Cycloadditionen von Aldehyden der Formel IX oder Ketonen der Formel X mit Olefinen XI, vorzugsweise mit Enolethern der Formel XI (mit R⁷ = OR¹⁰) zu den erfindungsgemäßen Benzoesäureestern der Formel Vc, in der die Variablen R¹, R², R³, R¹⁰ und M die unter Formel V genannte Bedeutung haben und A für eine Gruppe der Formel IIIa steht.

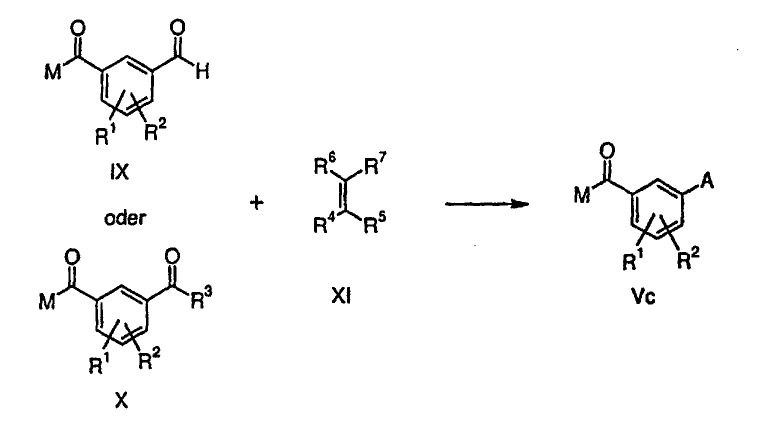

Die Durchführung der photochemischen Cycloaddition erfolgt in indifferenten Lösungsmitteln, die im eingesetzten Spektralbereich keine signifikante UV-Absorption aufweisen. So können z.B. Acetonitril oder aliphatische oder aromatischen Kohlenwasserstoffe, wie z.B. n-Hexan oder Toluol.

Als Strahlungsquellen werden geeignete UV-Strahler, bevorzugt Niederdruck- oder Mittel- bzw. Hochdruck-Quecksilber-Lampen eingesetzt (A.M. Braun, M.T. Maurette, E. Oliveros, Photochemical Technology, John Wiley & Sons Ltd. 1991). Niederdruck-Quecksilber-Lampen emittieren bei 189 und 253 nm, wobei die Emission bei 189 nm fast vollständig durch Sauerstoff, Wasser oder Lösungsmittel absorbiert wird, so daß Niederdruck-Quecksilber-Lampen praktisch eine monochromatische Strahlung bei 253 nm emittieren.

Mittel- bzw. Hochdruck-Quecksilber-Lampen werden bei Drücken zwischen 1 und 100 atm betrieben und emittieren je nach Druck und Temperatur in einem Wellenlängenbereich zwischen 200 und 600 nm,
wobei Mitteldruck-Quecksilber-Lampen eine dominante Emissionslinie bei 366 nm und Hochdruck-Quecksilber-Lampen zwei dominante Emissionen bei 436 und 546 nm aufweisen.

Darüber hinaus können mit Metallsalzen, wie z.B. Thallium-, Indium-, Natrium oder Galliumhalogenide dotierte Mittel- bzw. Hochdruck-Quecksilber-Lampen eingesetzt werden. Die Dotierung bewirkt eine Modifikation des Emissionsspektrums der Mittel- bzw. Hochdruck-Quecksilber-Lampen und führt zur Emission zusätzlicher für die jeweilige Dotierung charakteristischer Emissionslinien.

Zusätzlich können noch geeignete Filter eingesetzt werden, die nicht gewünschte Wellenlängenbereiche unterdrücken.

Geeignete Photoreaktoren sollten nicht im eingesetzten UV-Bereich absorbieren, sondern für die durch einen geeigneten UV-Strahler emittierten Wellenlängen transparent sein.

Dafür eignen sich insbesondere Borosilikat-Glas, z.B. Borosilikat BK7 von Schott oder Corning 7740 von Pyrex oder Quartz-Glas, das im UV-Bereich eine noch bessere Durchlässigkeit als Borosilikat-Glas aufweist.

Bevorzugte Photoreaktoren sind Falling Film-Reaktoren.

### Verfahren C:

Intramolekulare Cyclisierung von β-Halogenfettsäuren bzw. deren Alkalisalzen der Formel XIIa oder XIIb zu den erfindungsgemäßen Benzoesäureestern der Formel Vc, in der die Variablen R¹, R² und M die unter Formel V genannte Bedeutung haben, A für eine Gruppe der Formel IIIa oder IIIb und L² für eine nucleophil verdrängbare Abgangsgruppe, vorzugsweise Iod, Brom oder Chlor steht.

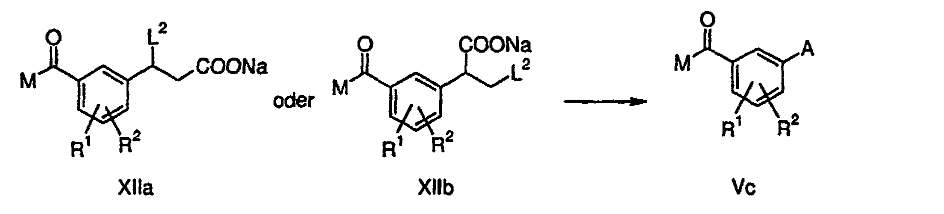

Die Abspaltung von Halogenwasserstoff aus β-Halogenfettsäuren oder deren Salzen, wie z.B. den Natriumsalzen durch wäßrige Lösungen von Alkalimetallsalzen, z.B. Natriumcarbonatlösung führt zur Bildung von β-Lactonen (A. Einhorn, Chem. Ber. 16, 2208 (1883)). Statt mit Natriumcarbonat gelingt die Abspaltung von Halogenwasserstoff häufig mit Silberoxid bzw. mit Silbersalzen.

Auf Grund der Labilität der β-Lactone gegenüber wäßrigen Alkalihalogeniden, ist es notwendig bei der Darstellung wasserlöslicher β-Lactone in Gegenwart von Lösungsmitteln zu arbeiten, damit das entstandene β-Lacton möglichst rasch aus der wäßrigen Phase entfernt wird (Org. Reactions 8, 309 (1954)). Geeignete Lösungsmittel sind z.B. Diethylether oder Chloroform.

### Verfahren D:

Cycloaddition von Aldehyden der Formel IX oder Ketonen der Formel X mit Ketenen XIII zu den erfindungsgemäßen Benzoesäureestern der Formel Vc, in der die Variablen R¹, R², R³, R⁴, R⁵ und M die unter Formel V genannte Bedeutung haben und A für eine Gruppe der Formel IIIa oder IIIb steht.

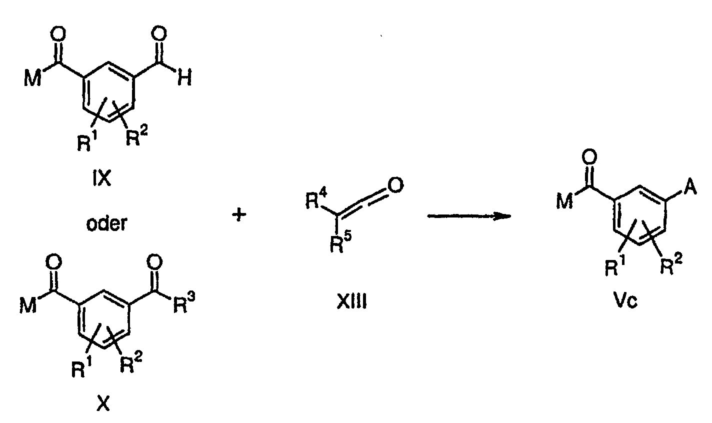

Ketene reagieren mit Carbonylverbindungen je nach Art des Katalysators und der Reaktionsbedingungen bei Temperaturen von 20 - 100°C, bevorzugt bei Temperaturen von 40 - 80°C unter Bildung von Enolacetaten oder von β-Lactonen (z.B. H. Kröper in Houben-Weyl, Methoden der Organischen Chemie, Band VI/2, Sauerstoffverbindungen I, Teil 2, 4. Aufl., S. 511 ff., Georg Thieme Verlag, 1965). Werden basische Katalysatoren, wie z.B. tertiäre Amine oder Alkali- oder Erdalkalimetalle eingesetzt, erfolgt die Bildung von β-Lactonen nach C-Acetylierung (J.A. Spence, E.F. Degering, Chem. ABst. 43, 6654) (1949)). In Gegenwart spezieller Katalysatoren kondensieren sich Aldehyde oder Ketone schon bei niedrigeren Temperaturen von 0 - 10°C mit dem einfachsten Keten unter Bildung von β-Lactonen. Die Wahl des Katalysators ist von den einzelnen Carbonylverbindungen abhängig. Als Katalysatoren für aromatische Aldehyde können z.B. Borsäure, Triacetylborat, Zinkrhodanid und Zinkchlorid, Aluminiumchlorid, Quecksilber-(II)-chlorid, sowie aktivierte Tonerde und Siliciumdioxid verwendet werden.

Die Reaktion muß zur Erzielung hoher Ausbeuten in wasserfreiem Medium durchgeführt werden.

Als Lösungsmittel eignen sich Ether, z.B. Diethylether, Halogenalkane, z.B. Methylenchlorid oder Chloroform, und wegen der Polymerisationstendenz der β-Lactone bevorzugt Ketone.

### Verfahren E:

Addition von Thiolesterenolaten XIV an Aldehyde der Formel IX oder Ketone der Formel X in Analogie zu literaturbekannten Verfahren (R. L. Danheiser, J. S. Nowick, Journal of Organic Chemistry 56, 1176 (1991)) zu den erfindungsgemäßen Benzoesäureestern der Formel Vc, in der die Variablen R¹, R², R³, R⁴, R⁵ und M die unter Formel V genannte Bedeutung haben und A für eine Gruppe der Formel IIIa oder IIIb steht.

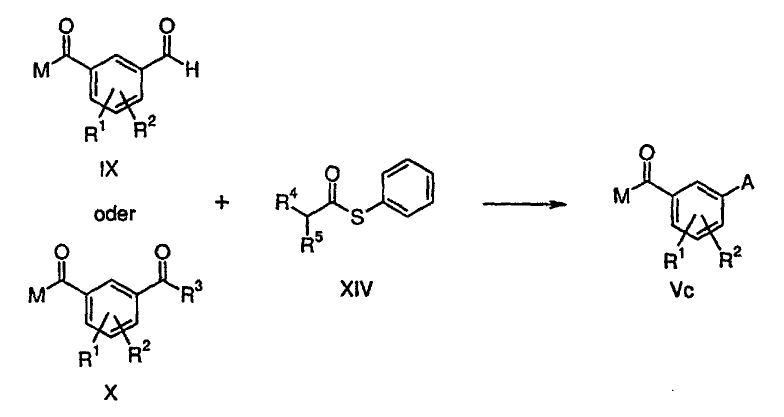

Die als Edukte eingesetzten Thioesterenolate XIV können in einer einfachen Einstufenreaktion aus Carbonsäurederivaten bereitgestellt werden (z.B. T. Mukaiyama, T. Takeda, K. Atsumi, Chemistry Letters 1974, 187).

In Gegenwart von einem Equivalent einer Base, bevorzugt einer Lithiumbase, wie z.B. Lithiumdiisopropylamid wird das entsprechende Enolat gebildet, das mit Aldehyden oder Ketonen die gewünschten Verbindungen der Formel Vc, insbesondere β-Lactone bildet.

### Verfahren F:

Cyclisierung von 1,2-Halogenhydrinen der Formel XVa oder XVb unter alkalischen Reaktionsbedingungen zu den erfindungsgemäßen Benzoesäureestern der Formel Vc, in der die Variablen R¹, R² und M die unter Formel V genannte Bedeutung haben, A für eine Gruppe der Formel IV und L² für eine nucleophil verdrängbare Abgangsgruppe, vorzugsweise Iod, Brom oder Chlor steht.

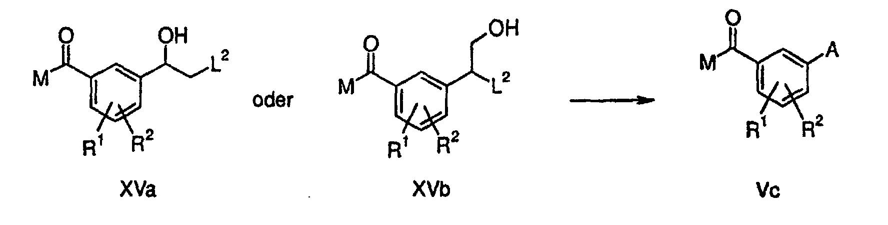

Anstelle der 1,2-Halogenhydrine können in Analogie auch deren Acetate als Ausgangsmaterial eingesetzt werden.

Den beschriebenen Methoden verwandt ist die Darstellung der erfindungsgemäßen Benzoesäureestern der Formel Vc durch Abspaltung von *p*-Toluolsulfonsäure aus 2-Hydroxy-toluolsulfonaten (z.B. H. Ohle, L. v. Vargha, Chemische Berichte 62, 2440 (1929).

Als Abspaltungsmittel werden vor allem Alkali- und Erdalkalihydroxide verwendet, seltener organische Basen, z.B. Alkoholate, sekundäre Amine oder Pyridin bzw. seine Homologen, wie z.B. Kollidin. Bevorzugte Alkali- und Erdalkalihydroxide sind z.B. Kaliumhydroxid, Natriumhydroxid oder Calciumhydroxid.

Die Abspaltung von Halogenwasserstoff erfolgt bisweilen schon durch alkalisch reagierende Salze, z.B. Kaliumcarbonat, Bariumcarbonat oder Kaliumfluorid. Ferner ist die Verwendung von Aluminaten, Silikaten und Zinkaten (z.B. J. D. Zech, Chemical Abstracts, 46, 8672 (1952)), Blei-(II)-oxid, Aluminiumoxid, Silbernitrat oder alkalischer Ionenaustauscher beschrieben.

Die Abspaltungsmittel können sowohl in Lösung als auch in Substanz, teilweise in gepulverter Form, z.B. gepulvertes Kaliumhydroxid verwendet werden.

Die Durchführung der Cyclisierung erfolgt in indifferenten Lösungsmitteln. Geeignet sind offenkettige oder cyclische Ether, z.B. Diethylether oder Dioxan oder aromatische Kohlenwasserstoffe, z.B. Benzol oder Toluol.

### Verfahren G:

Epoxidierung von Olefinen der Formel XVI zu den erfindungsgemäßen Benzoesäureestern der Formel Vc, in der die Variablen R¹, R² und M die unter Formel V genannte Bedeutung haben, A für eine Gruppe der Formel IV steht.

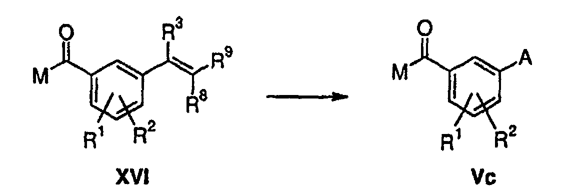

Die Epoxidierung wird in Analogie zu literaturbekannten Verfahren häufig mit Persäuren, z.B. Perbenzoesäure, Monoperphthalsäure, Perameisensäure, Peressigsäure, Trifluorperessigsäure oder Propionpersäure (z.B. R. Criegee, Houben-Weyl, "Methoden der Organischen Chemie", Band VIII, Sauerstoff-Verbindungen III, 4. Aufl., S. 40 ff., Georg Thieme Verlag, 1965), Wasserstoffperoxid oder *tert*.-Butylhydroperoxid in alkalischer Lösung, vorzugsweise in wäßriger Natriumhydroxid-Lösung oder mit Dioxiranen, z.B. Dimethyldioxiran bzw. Derivaten, wie z.B. (Trifluormethyl)-methyldioxiran (W. Adam, A. K. Smerz, Bulletin des Societés Chimiques Belges 105, 581 (1996)) durchgeführt.

Die Durchführung der Epoxidierung mit Persäuren erfolgt in indifferenten Lösungsmitteln, z.B. Diethylether, Chloroform, Tetrachlormethan, Ethylchlorid oder gelegentlich auch in Eisessig, während Dioxirane vorzugsweise in Aceton bzw. Derivaten, wie z.B. (Trifluormethyl)-methylketon eingesetzt wird.

Die Persäure, Wasserstoffperoxid, tert.-Butylhydroperoxid oder das Dioxiran wird oft in geringem Überschuß zur Reaktion gebracht, doch kann auch, wenn das Oxidationsmittel restlos ausgenutzt werden soll, ein Überschuß des Olefins zweckmäßig sein.

### Verfahren H:

Kondensation von Aldehyden der Formel IX mit α-Halogen-fettsäureestern XVII oder verwandten α-Halogenfettsäure-Derivaten, z.B. α-Halogen-fettsäureamide, -nitrile oder -ketone in Gegenwart alkalischer Kondensationsmittel entsprechend literaturbekannter Verfahren (z.B. M. Ballester, Chemical Reviews 55, 283 (1955)) zu den erfindungsgemäßen Benzoesäureestern der Formel Vc, in der die Variablen R¹, R², R¹⁰ und M die unter Formel V genannte Bedeutung haben und A für eine Gruppe der Formel IV steht.

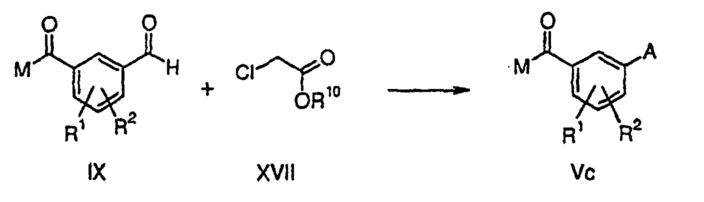

Als Kondensationsmittel eignen sich anorganische oder organische Katalysatoren, z.B. Natriumhydrid in Mineralöl, Lithiumhydrid, Tetraethylammoniumethylat, Natriumethanolat, Kalium-*tert*.-butylat oder Diisopropylmagnesiumbromid.

Die Durchführung erfolgt in indifferenten Lösungsmitteln, z.B. in Xylol, Diethylether, Methanol, Ethanol oder tert.-Butanol.

Hervorzuheben sind die erfindungsgemäßen Verbindungen der Formel I, wobei A für eine Gruppe der Formel IIa oder IIb steht und
- R⁴- R⁷: Wasserstoff, Hydroxy, Mercapto, Amino, Halogen, C₁-C₆-Alkyl, C₃-C₆-Cycloalkyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰, wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/ oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -COKR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
bedeuten.

Darüber hinaus sind die erfindungsgemäßen Verbindungen der Formel I hervorzuheben, wobei A für eine Gruppe der Formel IIa oder IIb steht und
- R⁴, R⁵: zusammen eine gegebenenfalls durch ein Stickstoff- oder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ steht; und/oder
- R⁶, R⁷: zusammen eine gegebenenfalls durch ein Stickstoff- oder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ steht.

Weiterhin sind die erfindungsgemäßen Verbindungen der Formel r hervorzuheben, wobei A für eine Gruppe der Formel IIa oder IIb steht und
- R⁵, R⁶: wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen.

Hervorzuheben sind desweiteren die erfindungsgemäßen Verbindungen der Formel I, wobei A für eine Gruppe der Formel III steht und
- R⁸, R⁹: Wasserstoff, Nitro, Halogen, Cyano, C₁-C₆-Alkyl, C₃-C₆-Cycloalkyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünfoder sechsgliedriges Hetaryl,
wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/oder eine bis drei der folgenden Gruppen tragen können: Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy. Phenyl, Benzyl, Hetaryl, Phenoxy, Benzyloxy und Hetaryloxy, wobei die acht letztgenannten Reste ihrerseits substituiert sein können;
bedeuten.

Darüber hinaus sind die erfindungsgemäßen Verbindungen der Formel I hervorzuheben, wobei A für eine Gruppe der Formel III steht und
- R⁸, R⁹: zusammen eine gegebenenfalls durch ein Stickstoff- oder ein Sauerstoffatom ein- oder zweifach unterbrochene C₂-C₅-Alkylen- oder C₂-C₅-Alkenylenkette
bilden.

Die für die Substituenten R¹-R¹⁶ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylsulfonyl-, Halogenalkylsulfonyl, Alkylcarbonyl-, Halogenalkylcarbonyl, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl: Methyl, Ethyl, n-Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkoxy-C₁-C₆alkyl und C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2.2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brom-methyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆alkyl, C₁-C₆-Alkoxy-C₂-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-l-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-l-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₂-C₆-Alkonyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heteroxyclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy: aromatische mono- oder polycyclische Reste, welche neben Kohlenstoffringgliedern zusätzlich ein bis vier Stickstoffatome oder ein bis drei Stickstoffatome und ein Sauerstoffoder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR¹⁰ oder -S(O)ₙR¹⁰; besonders bevorzugt Nitro, Halogen wie z.B. Fluor, Chlor oder Brom, C₁-C₆-Halogenalkyl, -OR¹⁰ oder -SO₂R¹⁰;
- R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR¹⁰ oder -S(O)ₙR¹⁰; besonders bevorzugt Wasserstoff, Nitro, Halogen wie z.B. Fluor, Chlor oder Brom, C₁-C₆-Alkyl, C₁-C₆-Halogenalkyl, -OR¹⁰ oder -SO₂R¹⁰;
- R⁴ - R⁷: Wasserstoff, Hydroxy, Mercapto, Amino, Halogen, C₁-C₄-Alkyl, C₅-C₆-Cycloalkyl, C₅-C₆-Cycloalkenyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰,
wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl; besonders bevorzugt Wasserstoff, Hydroxy, Mercapto, Halogen, wie z. B. Fluor, Chlor oder Brom, C₁-C₄-Alkyl, -OSO₂R¹⁰, -OPO₃R¹⁰, -Si(CH₃)₃;
- R⁴, R⁵: bilden bevorzugt zusammen eine gegebenenfalls durch ein Sauerstoffatom ein- oder zweifach unterbrochene C₂-C₅-Alkylen- oder C₂-C₅-Alkenylenkette oder eine Gruppe =X, wobei X vorzugsweise für ein Sauerstoffatom oder NR¹⁰ steht;
besonders bevorzugt bilden R⁴ und R⁵ eine Gruppe =X, wobei =X vorzugsweise für ein Sauerstoffatom steht;
- R⁶, R⁷: bilden bevorzugt zusammen eine gegebenenfalls durch ein Sauerstoffatom ein- oder zweifach unterbrochene C₂-C₅-Alkylen- oder C₂-C₅-Alkenylenkette oder eine Gruppe =X, wobei X vorzugsweise für ein Sauerstoffatom oder NR¹⁰ steht;
besonders bevorzugt bilden R⁴ und R⁵ eine Gruppe =X, wobei =X vorzugsweise für ein Sauerstoffatom steht;
- n: zwei;
- R⁵, R⁶: bilden bevorzugt zusammen, wenn sie an benachbarte Kohlenstoffatome gebunden sind, eine gegebenenfalls durch ein Stickstoff- oder ein Sauerstoffatom unterbrochene C₃-C₄-Alkylen- oder C₃-C₄-Alkenylenkette, wenn R⁴ und R⁷ für Wasserstoff stehen;
- R⁸, R⁹: Wasserstoff, Nitro, Halogen, Cyano, C₁-C₄-Alkyl, C₅-C₆-Cycloalkyl, C₅-C₆-Cycloalkenyl, C₅-C₆-Heterocyclyl, -OR¹⁰, -SR¹⁰, -COR¹⁰, -COOR¹⁰, -CONR³R¹⁰, Phenyl, Phenyl-C₁-C₄-alkyl und fünf- oder sechsgliedriges Hetaryl, wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
- R¹⁰: Wasserstoff, C₁-C₄-Alkyl, C₁-C₄-Halogenalkyl, C₂-C₄-Alkenyl, Phenyl oder Phenyl-C₁-C₄-alkyl, wobei der genannte Phenylrest partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl.
- R¹¹, R¹², R¹⁴, R¹⁶: Wasserstoff oder C₁-C₄-Alkyl; besonders bevorzugt Wasserstoff, Methyl oder Ethyl;
- R¹³: Wasserstoff, C₁-C₄-Alkyl, C₃-C₄-Cycloalkyl, wobei die beiden letztgenannten Gruppen gegebenenfalls einen bis drei der folgenden Substituenten tragen können: Halogen, C₁-C₄-Alkoxy oder C₁-C₄-Alkylthio;
Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithian-2-yl oder 1,3-Dithiolan-2-yl, wobei die sechs letztgenannten Gruppen jeweils einen bis drei C₁-C₄-Alkylreste tragen können;
besonders bevorzugt Wasserstoff, Methyl, Ethyl, Cyclopropyl, Di(methoxy)methyl, Di(ethoxy)methyl, 2-Ethylthiopropyl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 5,5-Dimethyl-1-3-dioxan-2-yl, 1,3-Oxathiolan-2-yl, 1,3-Oxathian-2-yl, 1,3-Dithiolan-2-yl, 5,5-Dimethyl-1,3-dithian-2-yl oder 1-Methylthiocyclopropyl;
- R¹⁵: Wasserstoff, C₁-C₄-Alkyl oder C₁-C₄-Alkoxycarbonyl; besonders bevorzugt Wasserstoff, Methyl oder Methoxycarbonyl.

Ebenso kann vorteilhaft in Betracht kommen, daß R¹³ und R¹⁶ eine π-Bindung ausbilden, so daß ein Doppelbindungssystem entsteht.

Die CR¹³R¹⁴-Einheit kann auch vorteilhaft durch C=O ersetzt werden.

Insbesondere bevorzugt sind die Verbindungen der Formel Ia, wobei R¹ in Position 2 und R² in Position 4 des Phenylringes gebunden sind.

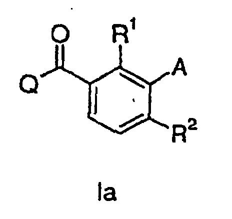

Außerordentlich bevorzugt sind die Verbindungen der Formel Ia, in der die Substituenten R¹, R² und Q die oben genannte Bedeutung haben, A für eine Gruppe der Formel IIa oder IIb steht und
- R⁴ - R⁷: Wasserstoff, Hydroxy, Amino, Halogen, C₁-C₄-Alkyl, C₅-C₆-Cyclpalkyl, Phenyl, -OR¹⁰, -SO₂R¹⁰, -OSO₂R¹⁰, PO₃(R¹⁰)₂, -OPO₃(R¹⁰)₂, -NR³R¹⁰ oder -OCOR¹⁰, wobei die genannten Alkyl und Cycloalkylreste sowie R³ und R¹⁰ der Reste -OR¹⁰, -SO₂R¹⁰, -OSO₂R¹⁰, -PO₃(R¹⁰)₂, -OPO₃(R¹⁰)₂, -NR³R¹⁰, -OCOR¹⁰ partiell oder vollständig halogeniert sein können und/ oder eine bis drei der folgenden Gruppen tragen können: Hydroxy, Amino, Cyano, R¹⁰, -OR¹⁰, -NR³R¹⁰, -OCOR¹⁰, -CO₂R¹⁰, C₁-C₄-Alkylcarbonyl, C₁-C₄-Alkylsulfonyl, Phenyl, Benzyl, Phenoxy und Benzyloxy, wobei die vier letztgenannten Reste ihrerseits substituiert sein können;
bedeuten.

Darüber hinaus sind die erfindungsgemäßen Verbindungen der Formel Ia außerordentlich bevorzugt, in der die Substituenten R¹, R² und Q die oben genannte Bedeutung haben, A für eine Gruppe der Formel IIa oder IIb steht und
- R⁴, R⁵: zusammen eine gegebenenfalls durch ein Sauerstoffatom ein- oder zweifach unterbrochene C₂-C₅-Alkylen- oder C₂-C₅-Alkenylenkette oder eine Gruppe =X bilden, wobei X vorzugsweise für ein Sauerstoffatom oder steht;
und/oder
- R⁶, R⁷: zusammen eine gegebenenfalls durch ein Sauerstoffatom ein- oder zweifach unterbrochene C₂-C₅-Alkylen- oder C₂-C₅-Alkenylenkette oder eine Gruppe =X bilden, wobei X vorzugsweise für ein Sauerstoffatom steht.

Weiterhin sind die erfindungsgemäßen Verbindungen der Ia außerordentlich bevorzugt, in der die Variablen R¹, R² und Q die oben genannte Bedeutung haben, A für eine Gruppe der Formel IIa oder IIb steht und
- R⁵, R⁶: wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen.

Außerordentlich bevorzugt sind die Verbindungen der Formel Ia, in der die Variablen R¹, R² und Q die oben genannte Bedeutung haben, A für eine Gruppe der Formel III steht und
- R⁸, R⁹: Wasserstoff, Nitro, Halogen, Cyano, C₁-C₄-Alkyl, C₅-C₆-Cycloalkyl, C₅-C₆-Cycloalkenyl, C₅-C₆-Heterocyclyl, -OR¹⁰, -COR¹⁰, -COOR¹⁰, -CONR³R¹⁰, Phenyl, Phenyl-C₁-C₄-alkyl und fünf- oder sechsgliedriges Hetaryl, wobei die genannten Alkyl und Cycloalkylreste sowie R³ und R¹⁰ der Reste -OR¹⁰, -COR¹⁰, -COOR¹⁰, -CONR³R¹⁰ partiell oder vollständig halogeniert sein können und/oder eine bis drei der folgenden Gruppen tragen können:
Hydroxy, Amino, Cyano, R¹⁰, -OR¹⁰, -NR³R¹⁰, -OCOR¹⁰, -CO₂R¹⁰, C₁-C₄-Alkylcarbonyl, C₁-C₄-Alkylsulfonyl, Phenyl, Benzyl, Phenoxy und Benzyloxy, wobei die vier letztgenannten Reste ihrerseits substituiert sein können;
bedeuten.

Darüber hinaus sind die erfindungsgemäßen Verbindungen der Formel Ia außerordentlich bevorzugt, in der die Variablen R¹, R² und Q die oben genannte Bedeutung haben, A für eine Gruppe der Formel III steht und
- R⁸, R⁹: zusammen eine gegebenenfalls durch ein Sauerstoffatom ein- oder zweifach unterbrochene C₂-C₅-Alkylen- oder C₂-C₅-Alkenylenkette
bilden.

Insbesondere bevorzugt sind die Verbindungen Ib der Tabellen 1 bis 36.

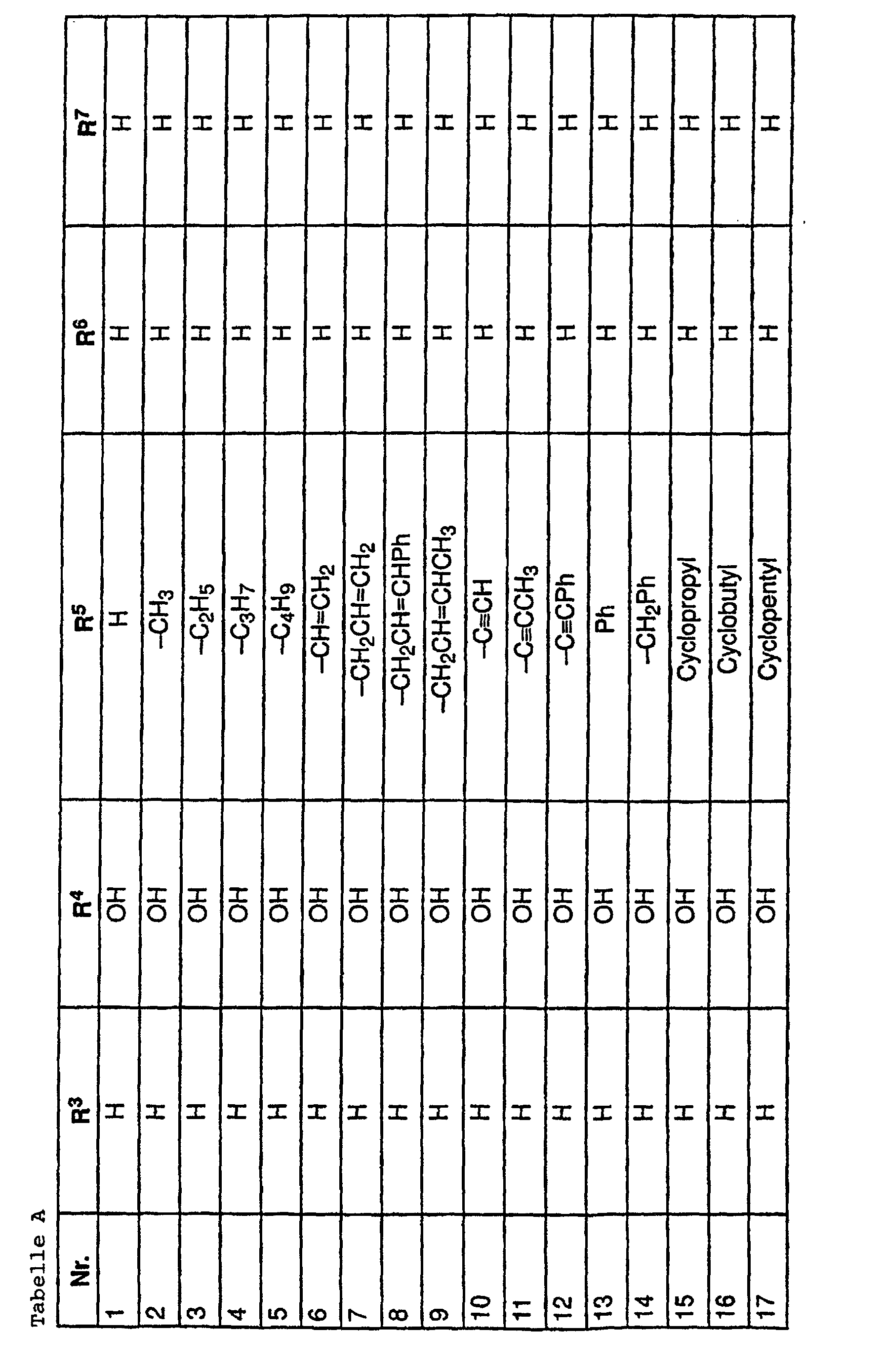

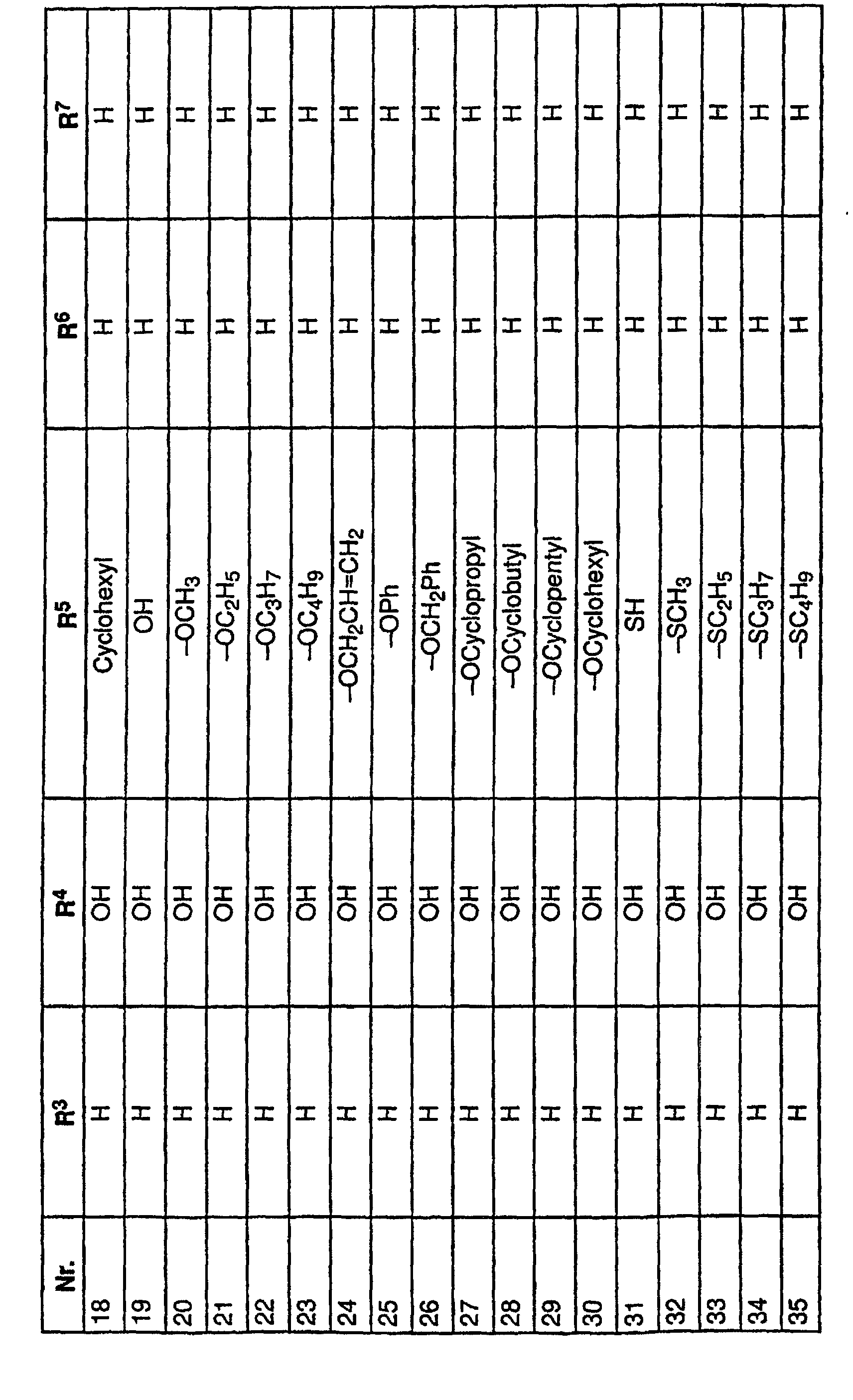

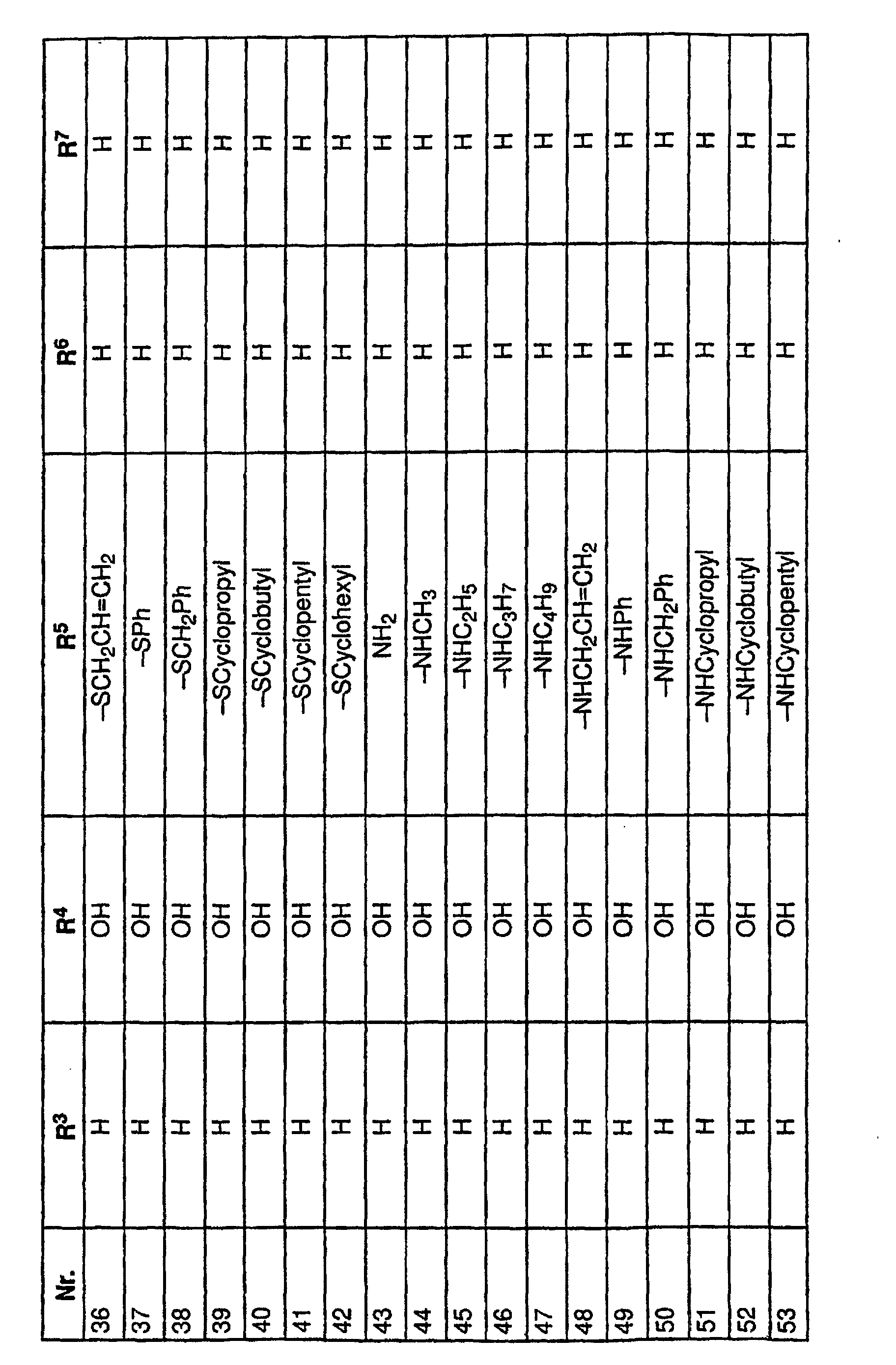

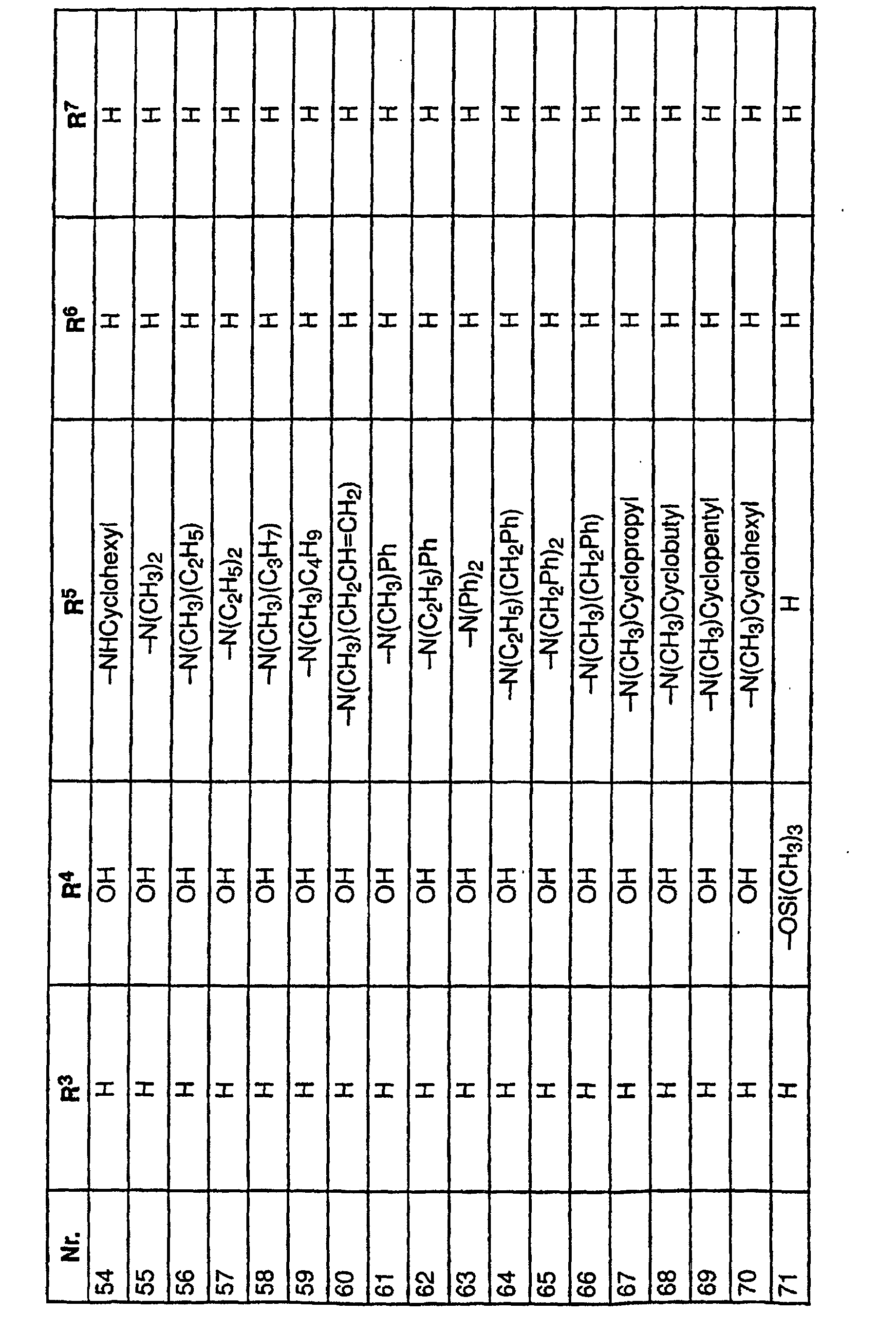

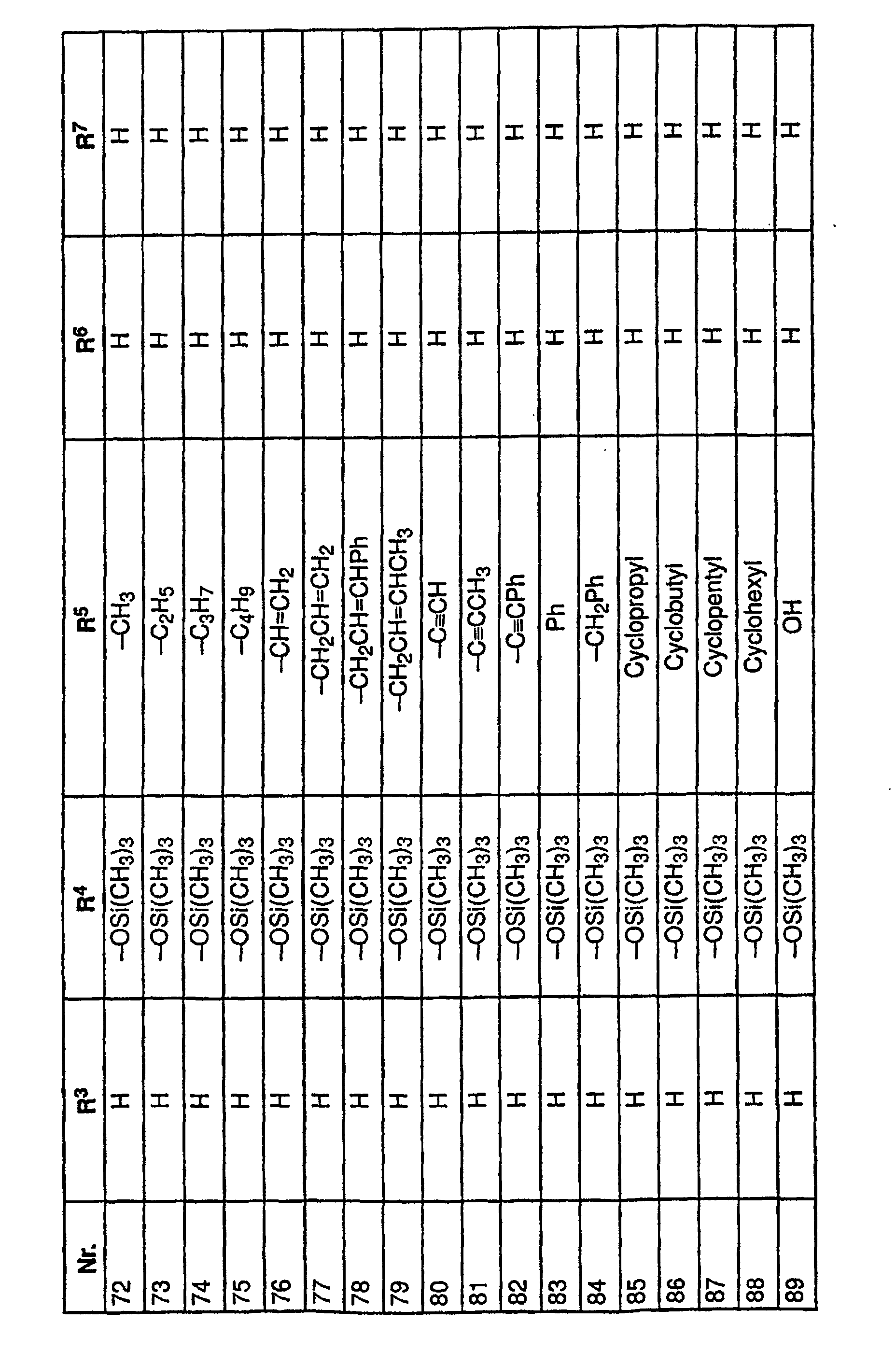

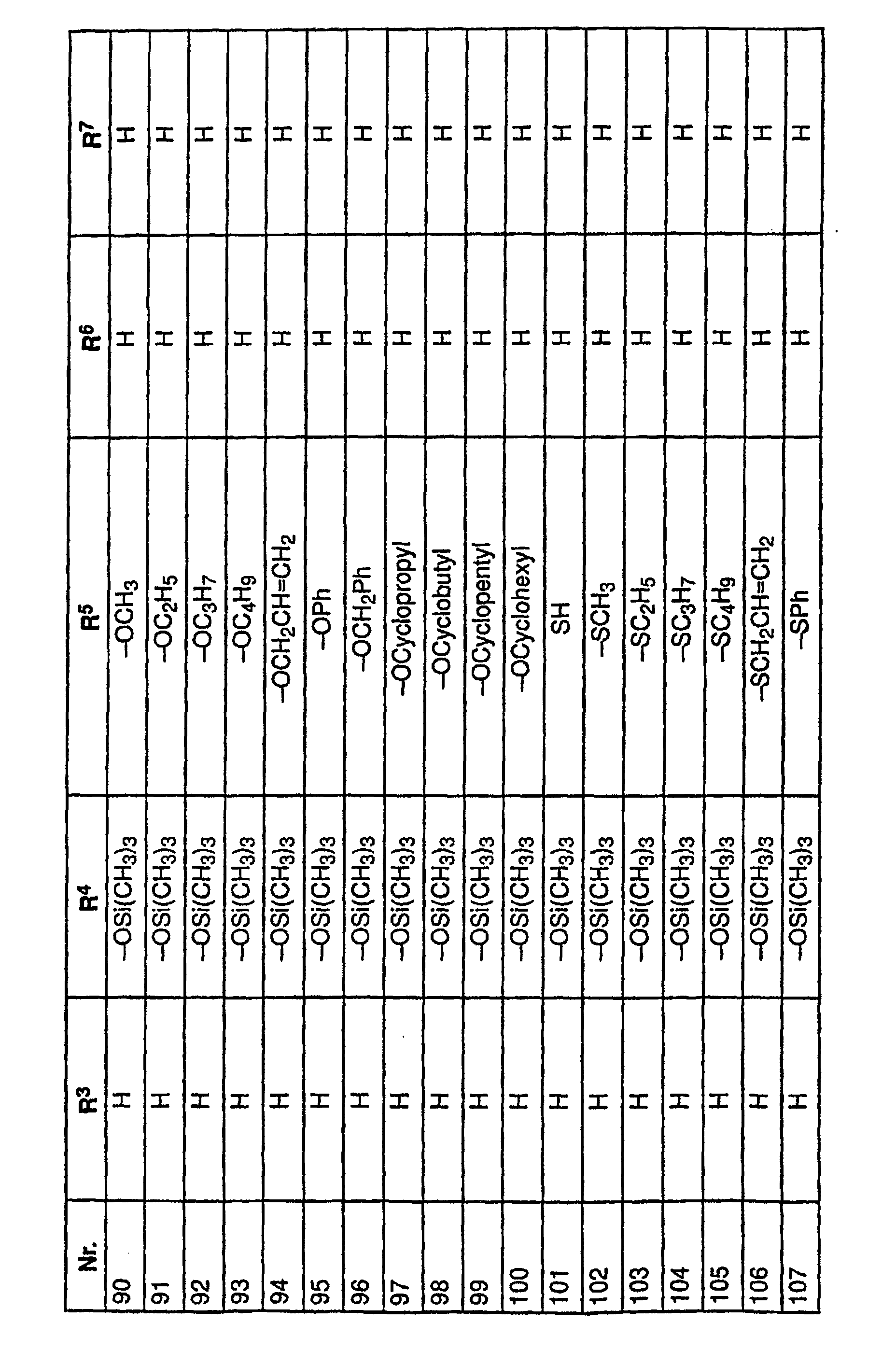

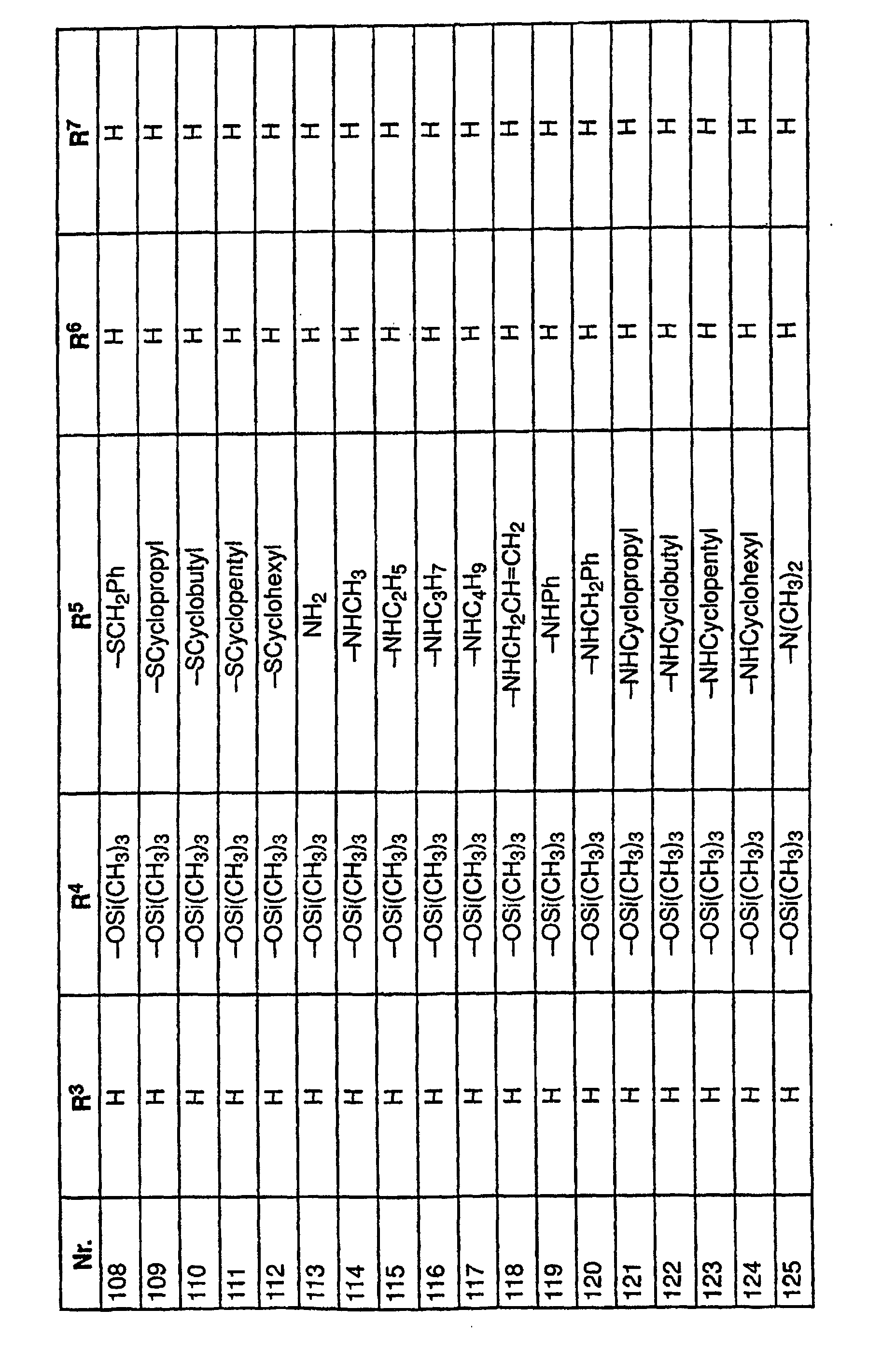

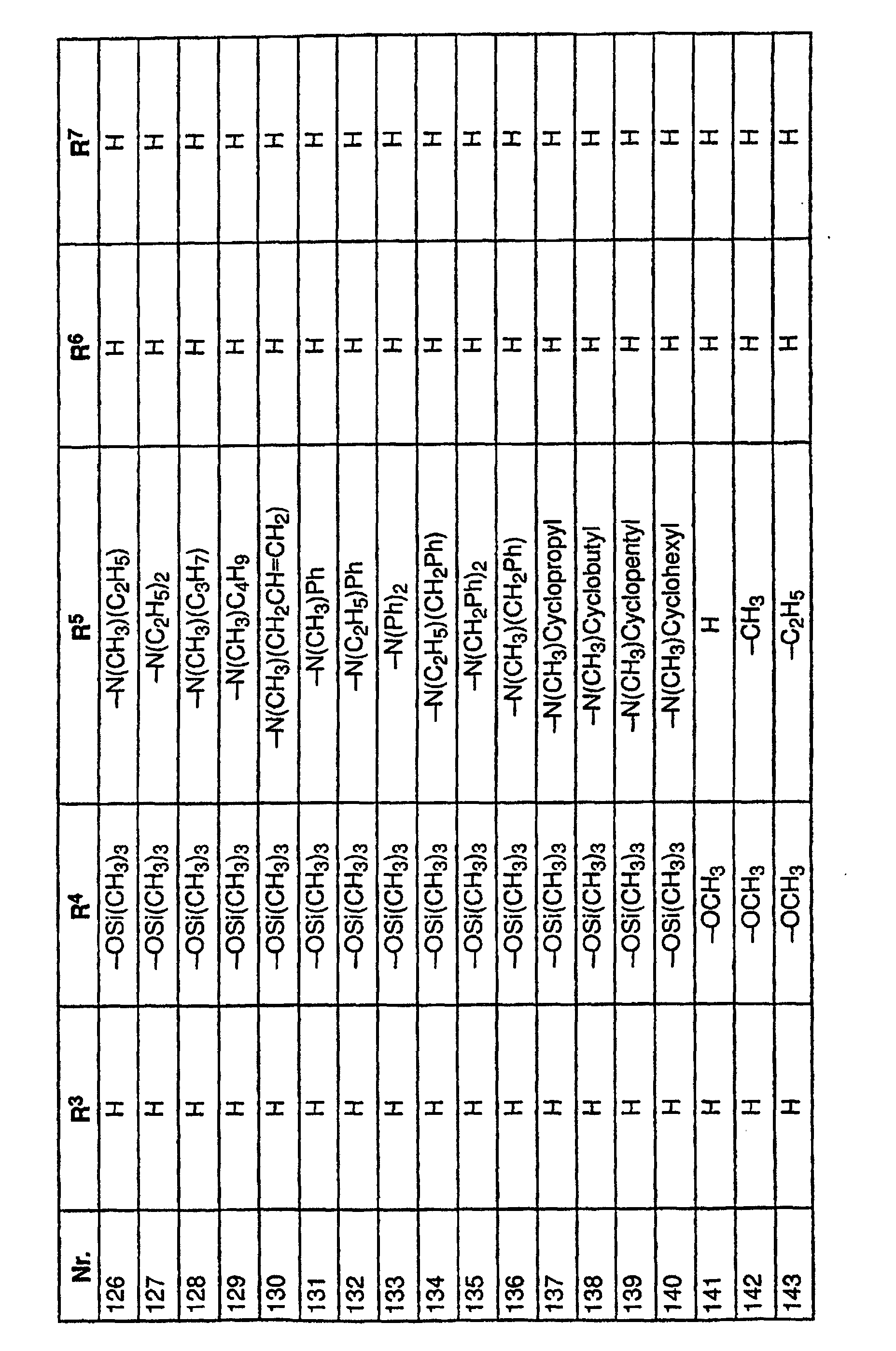

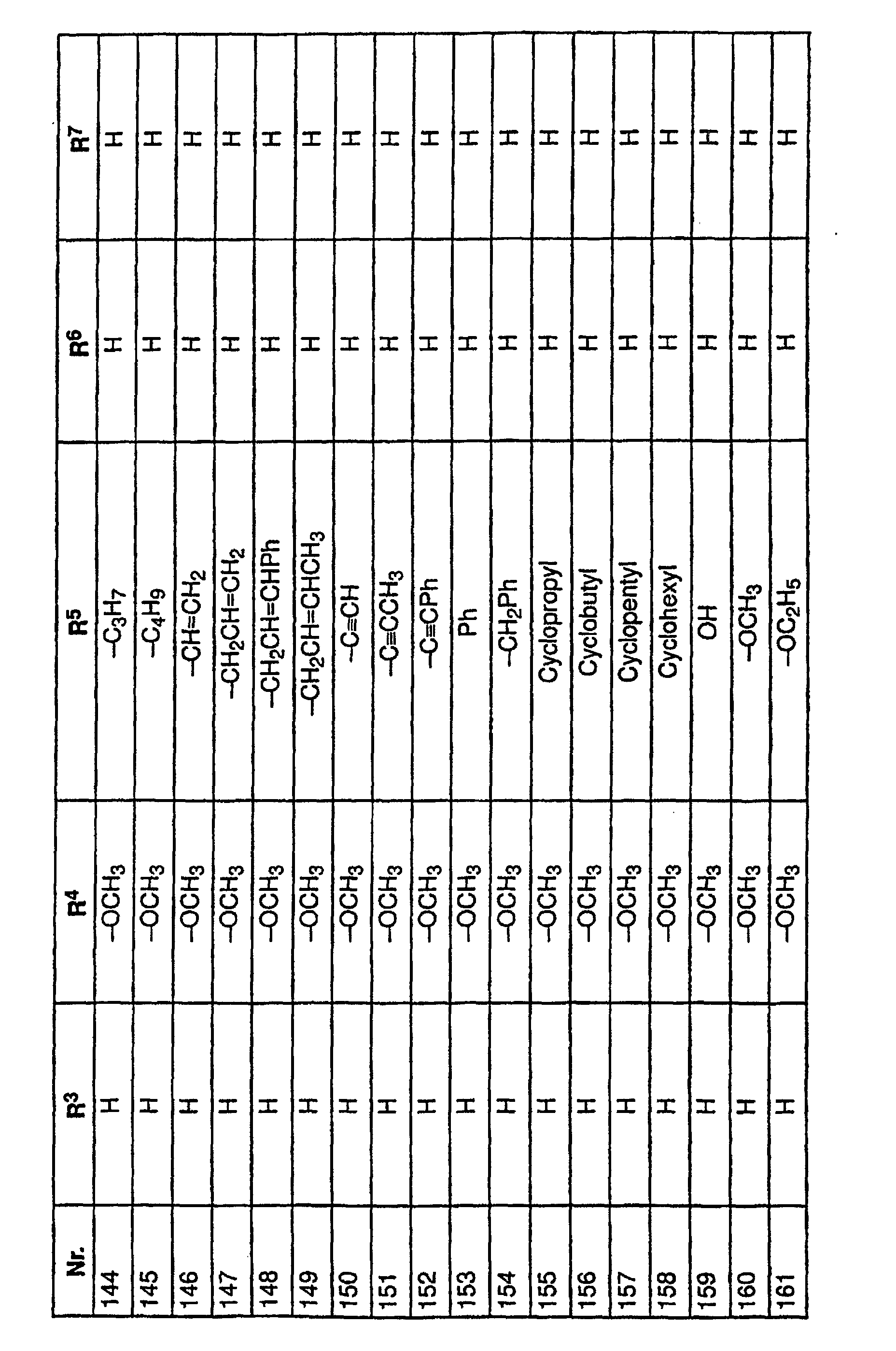

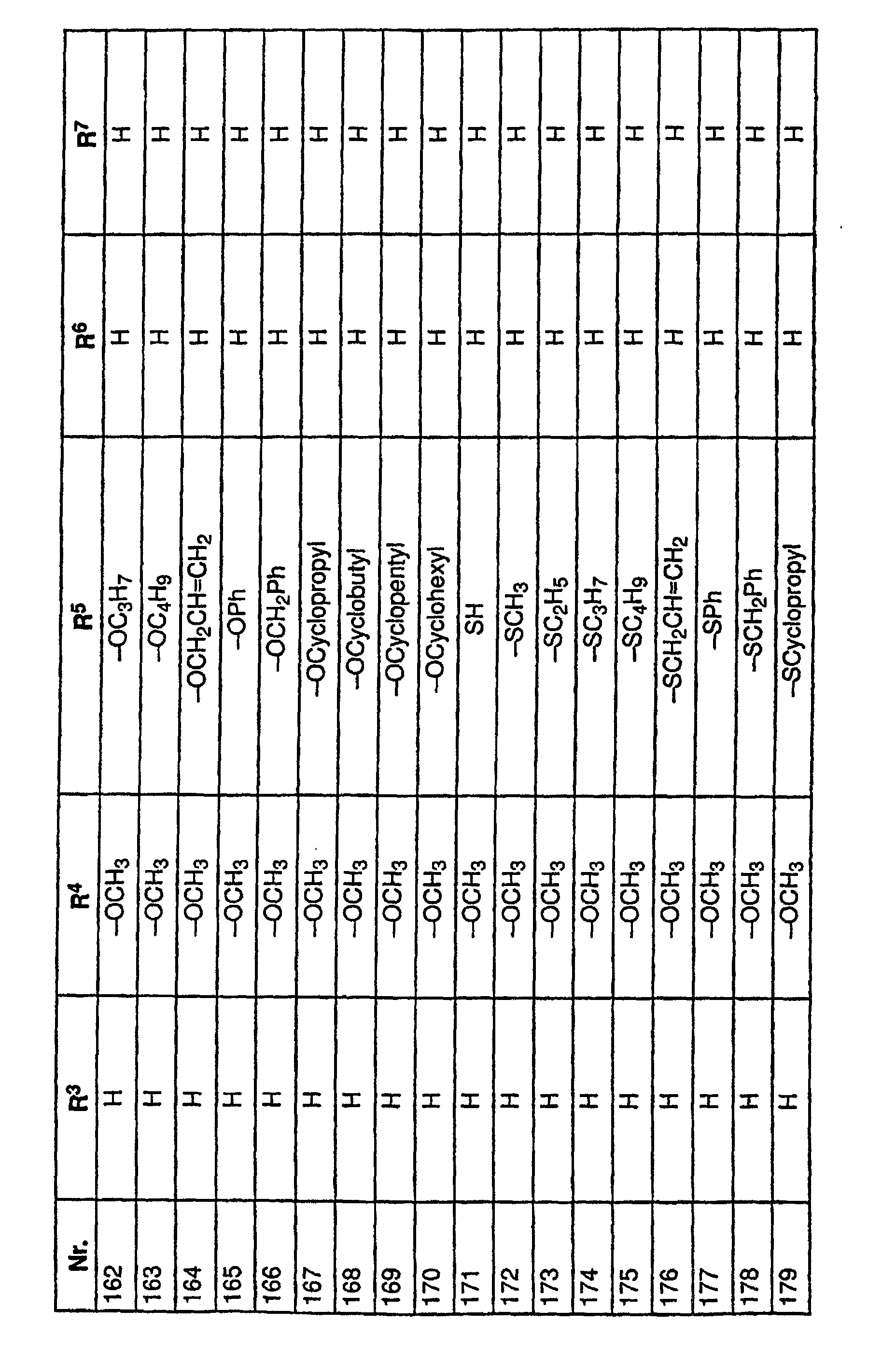

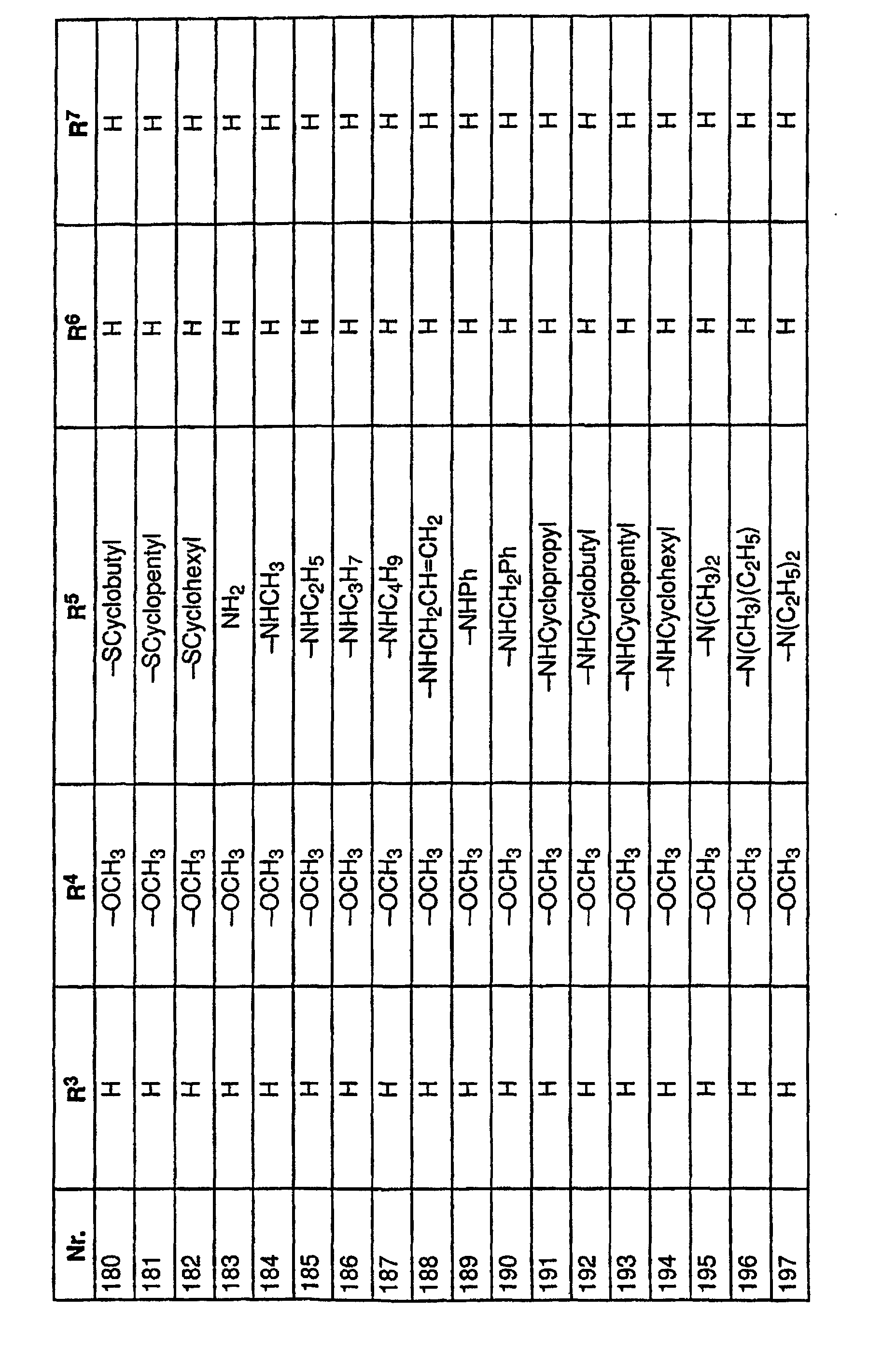

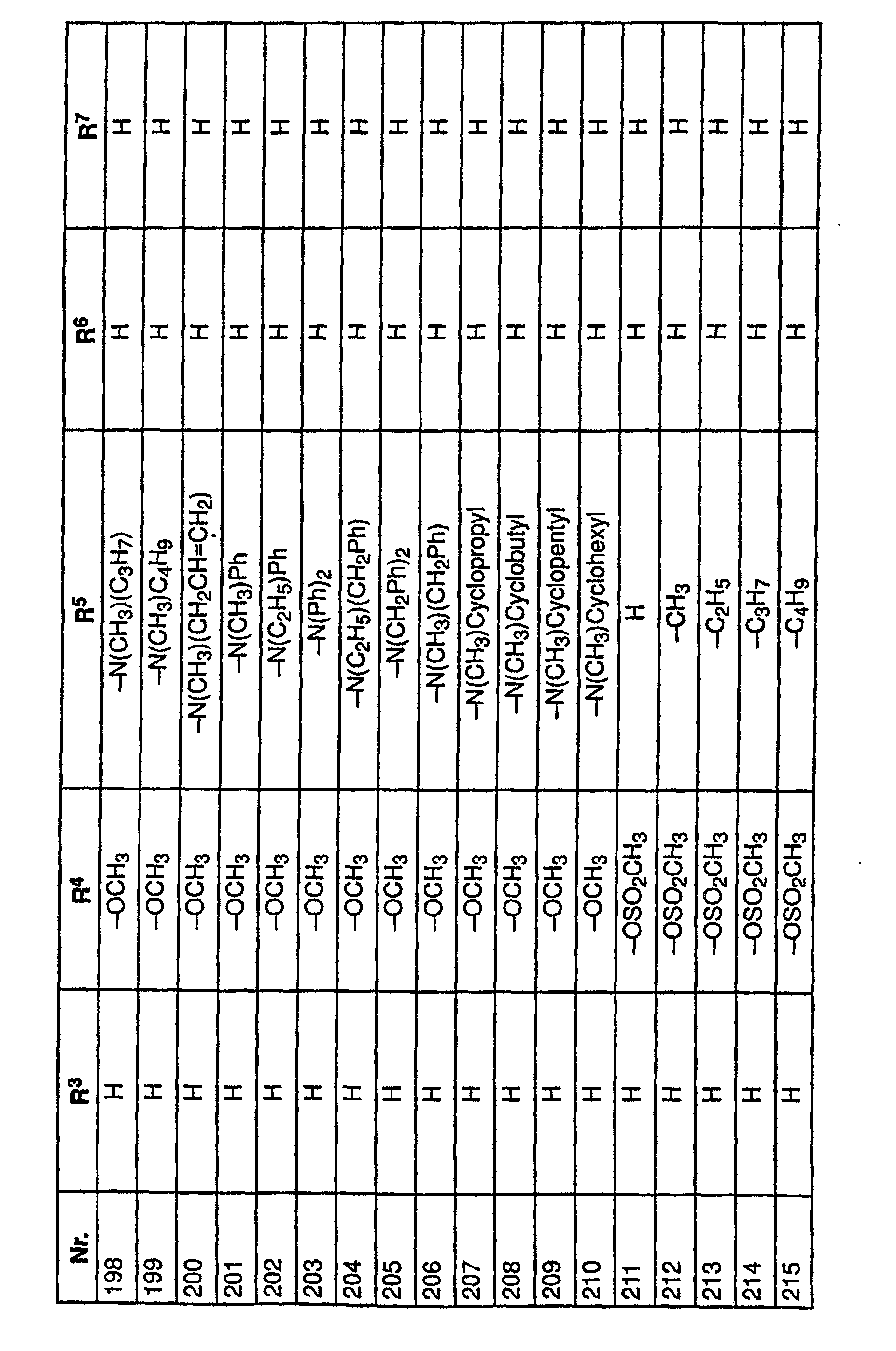

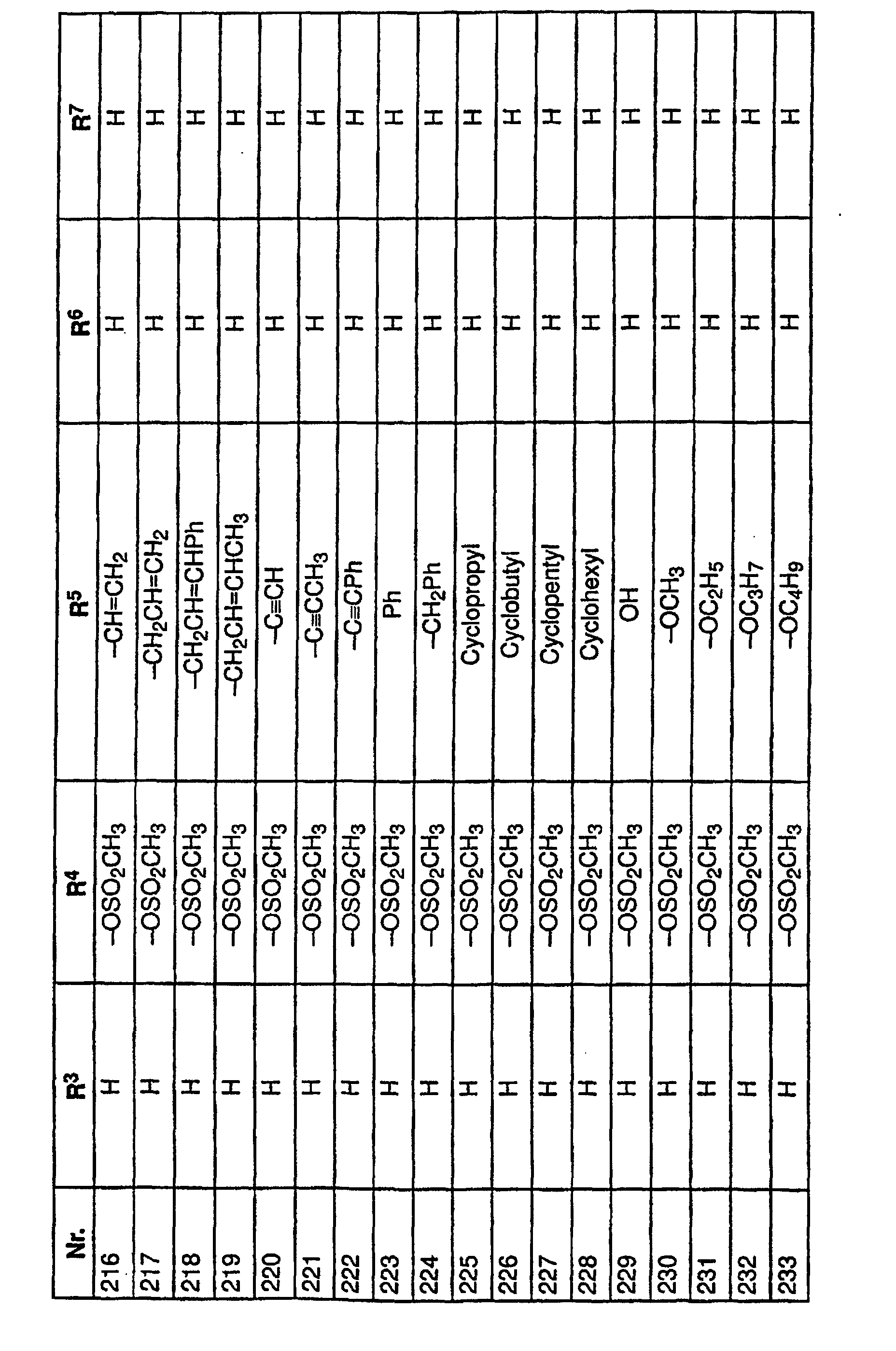

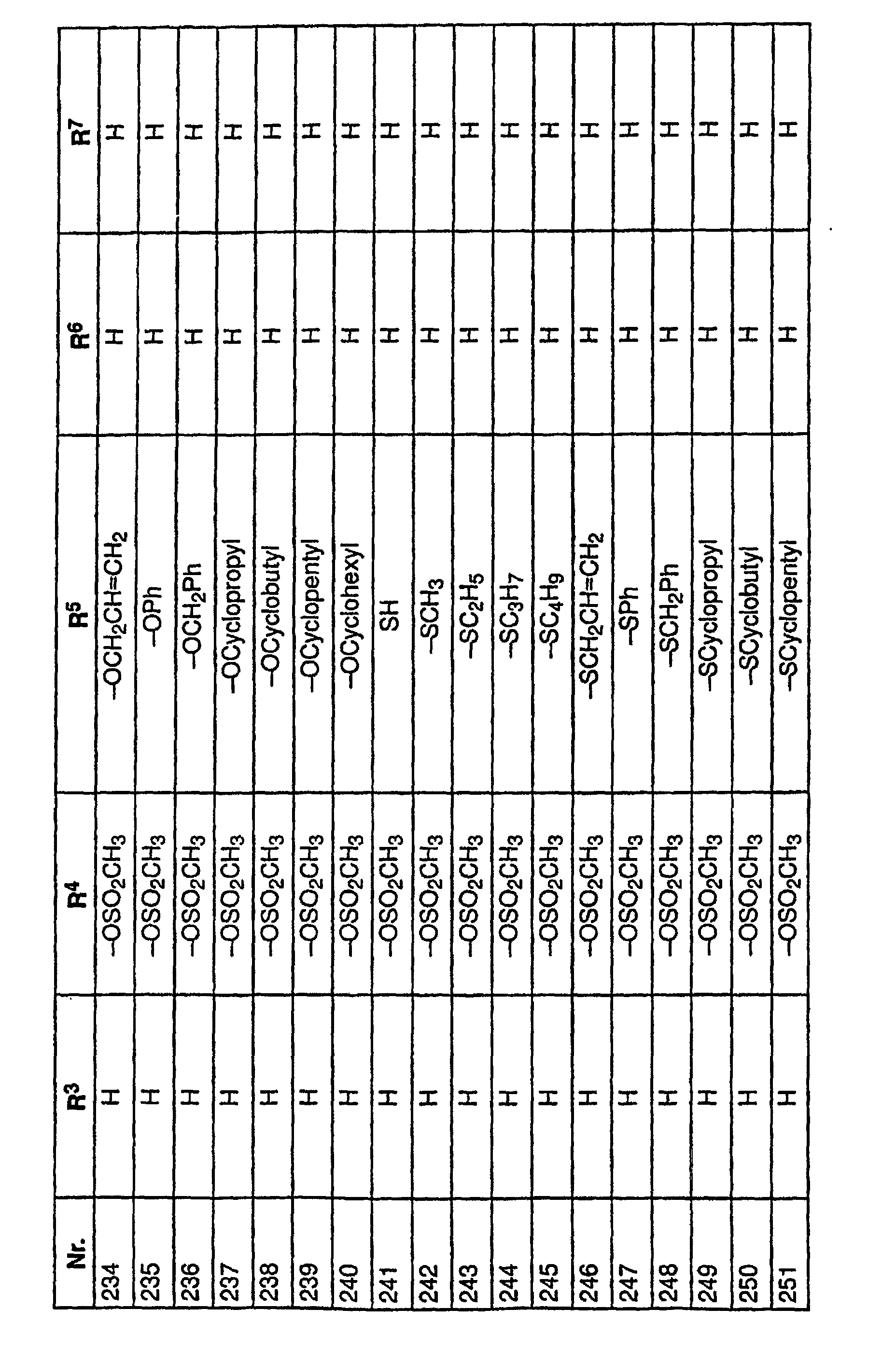

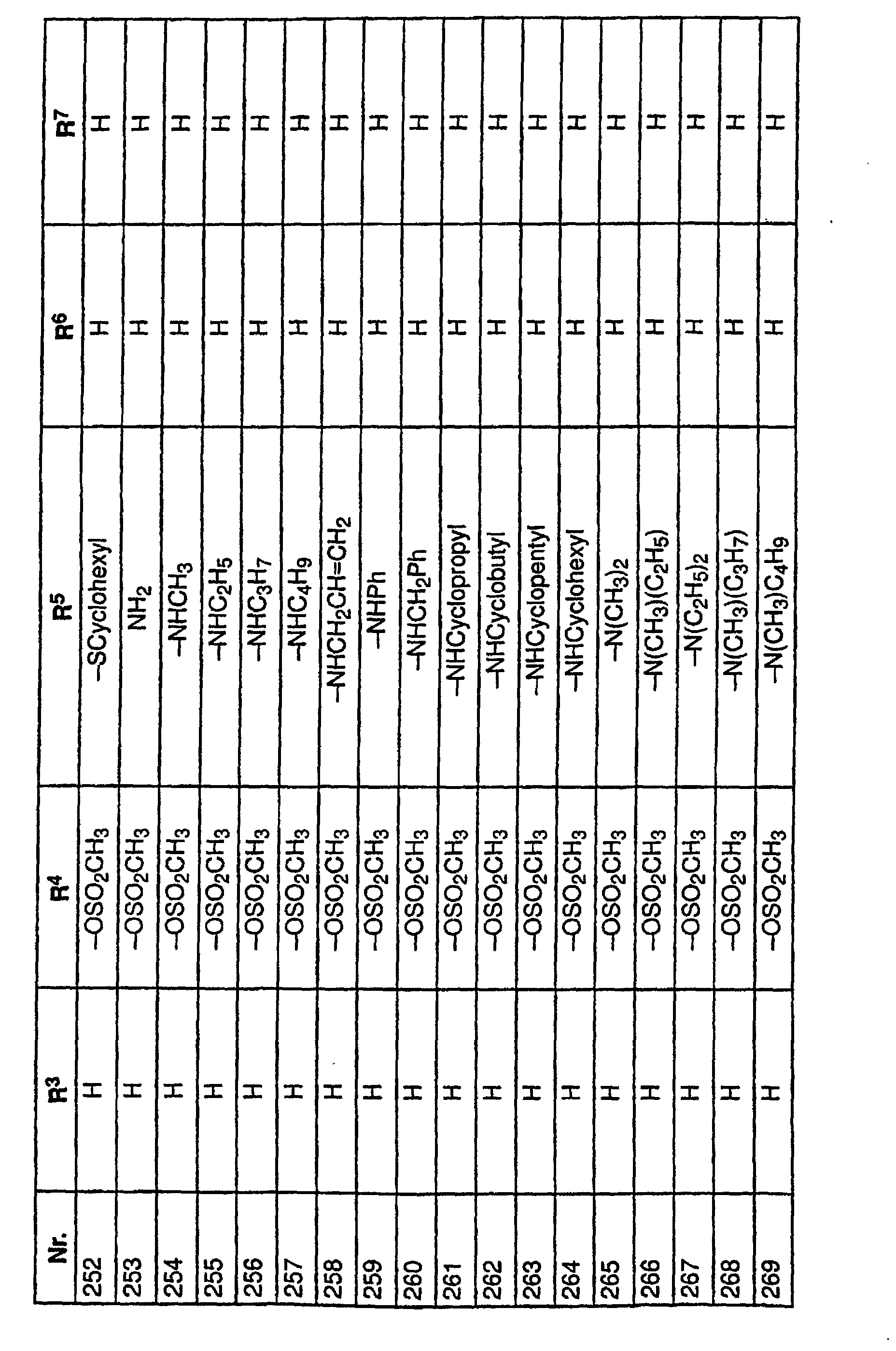

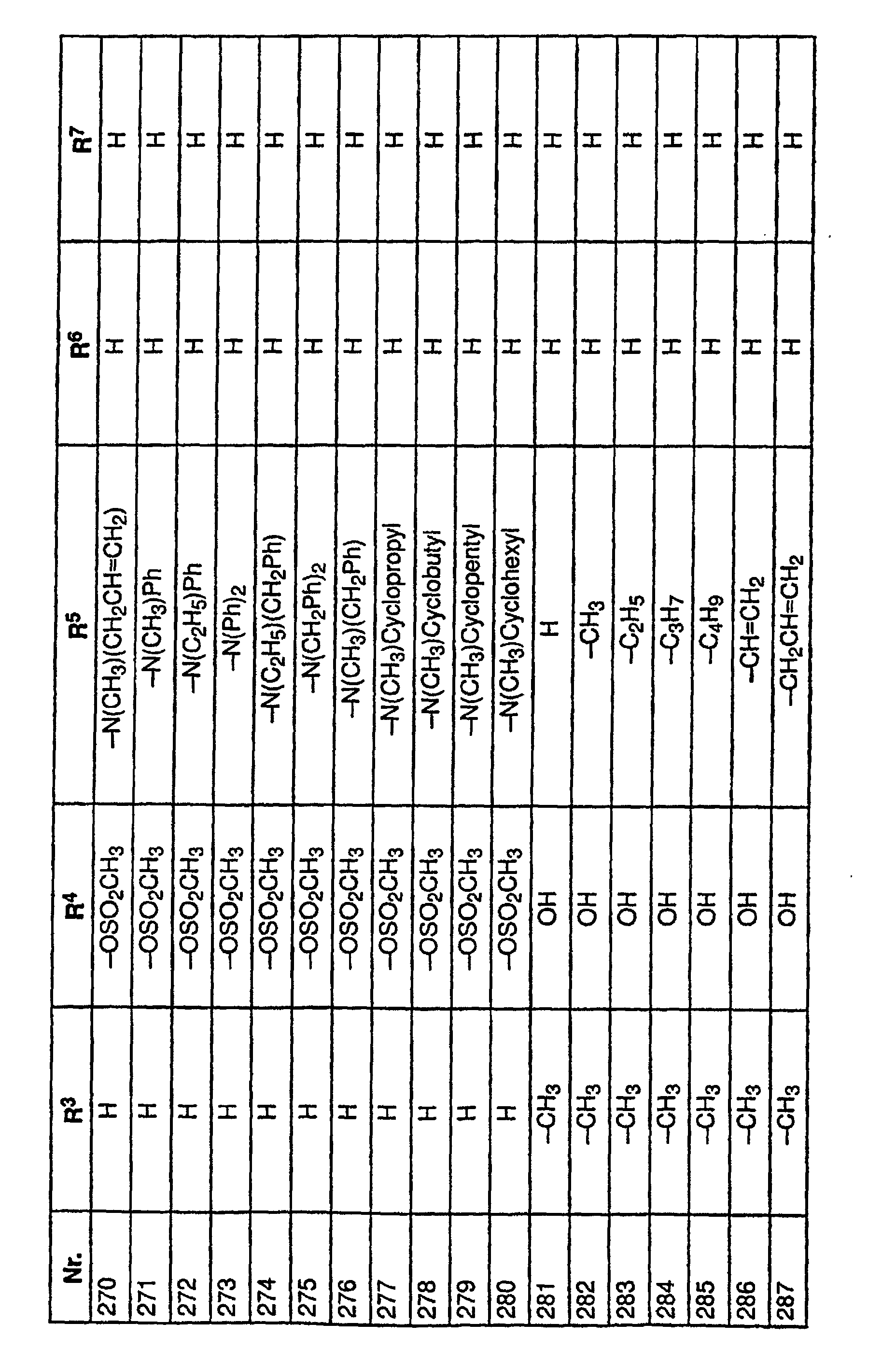

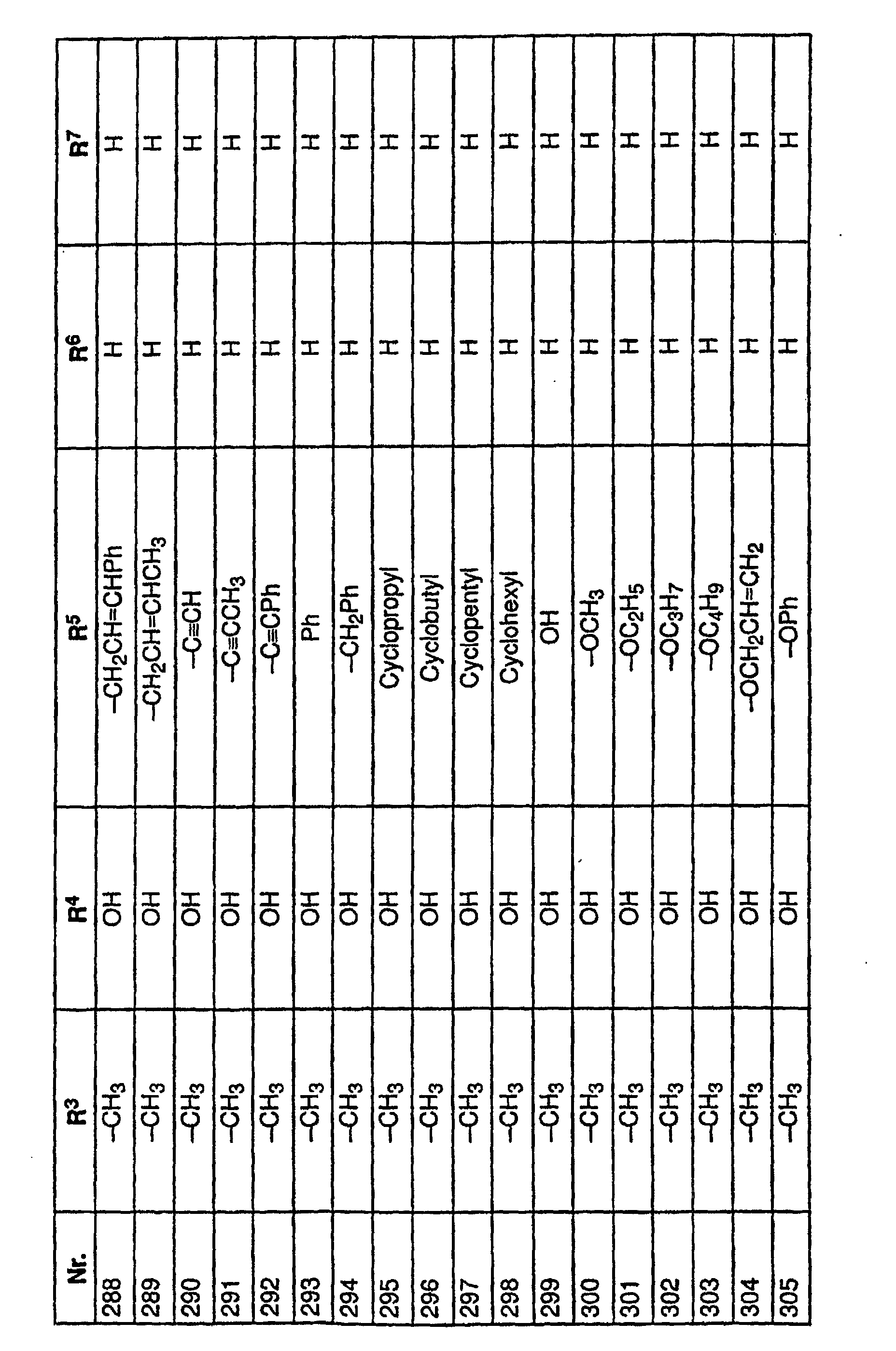

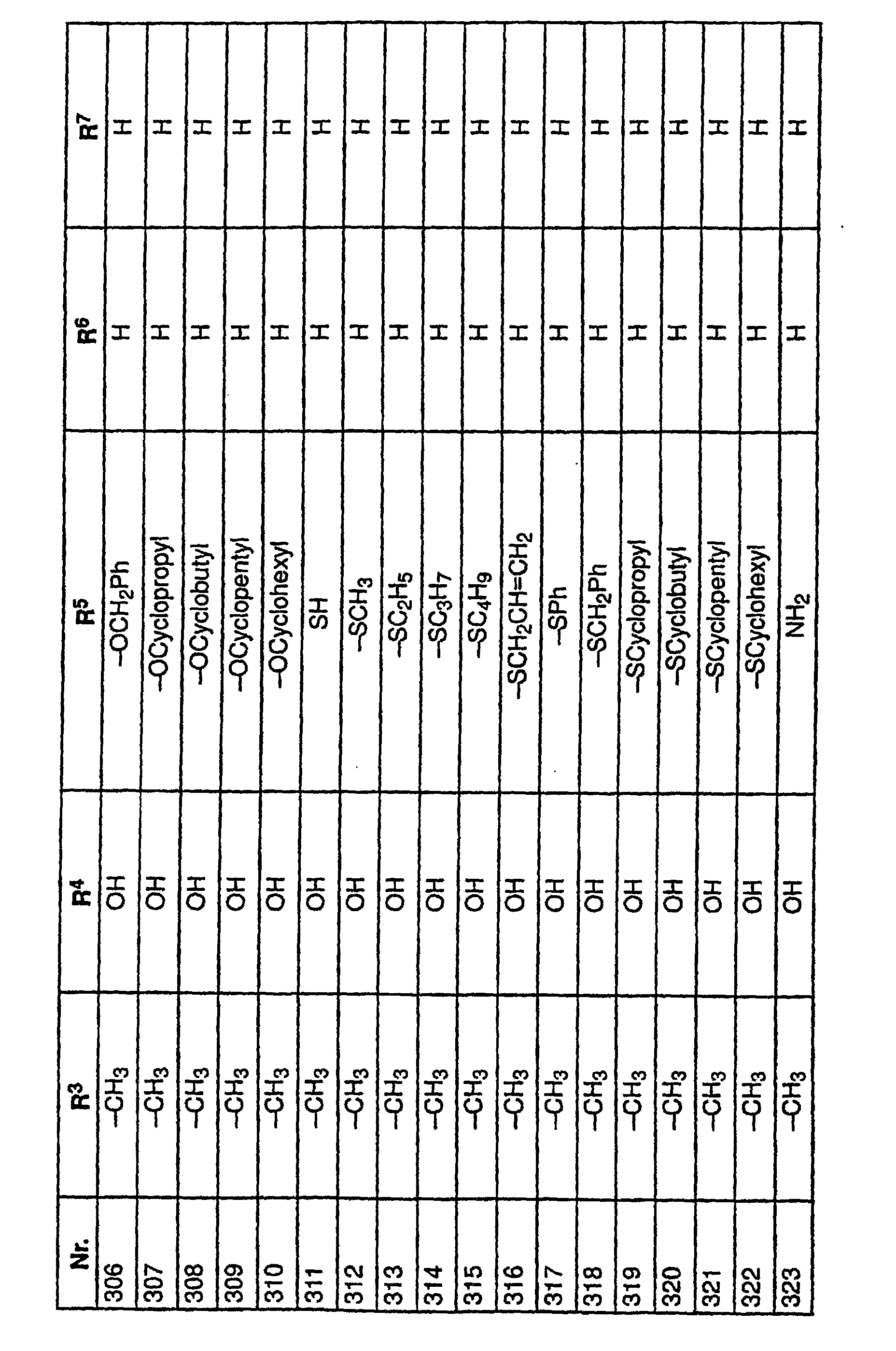

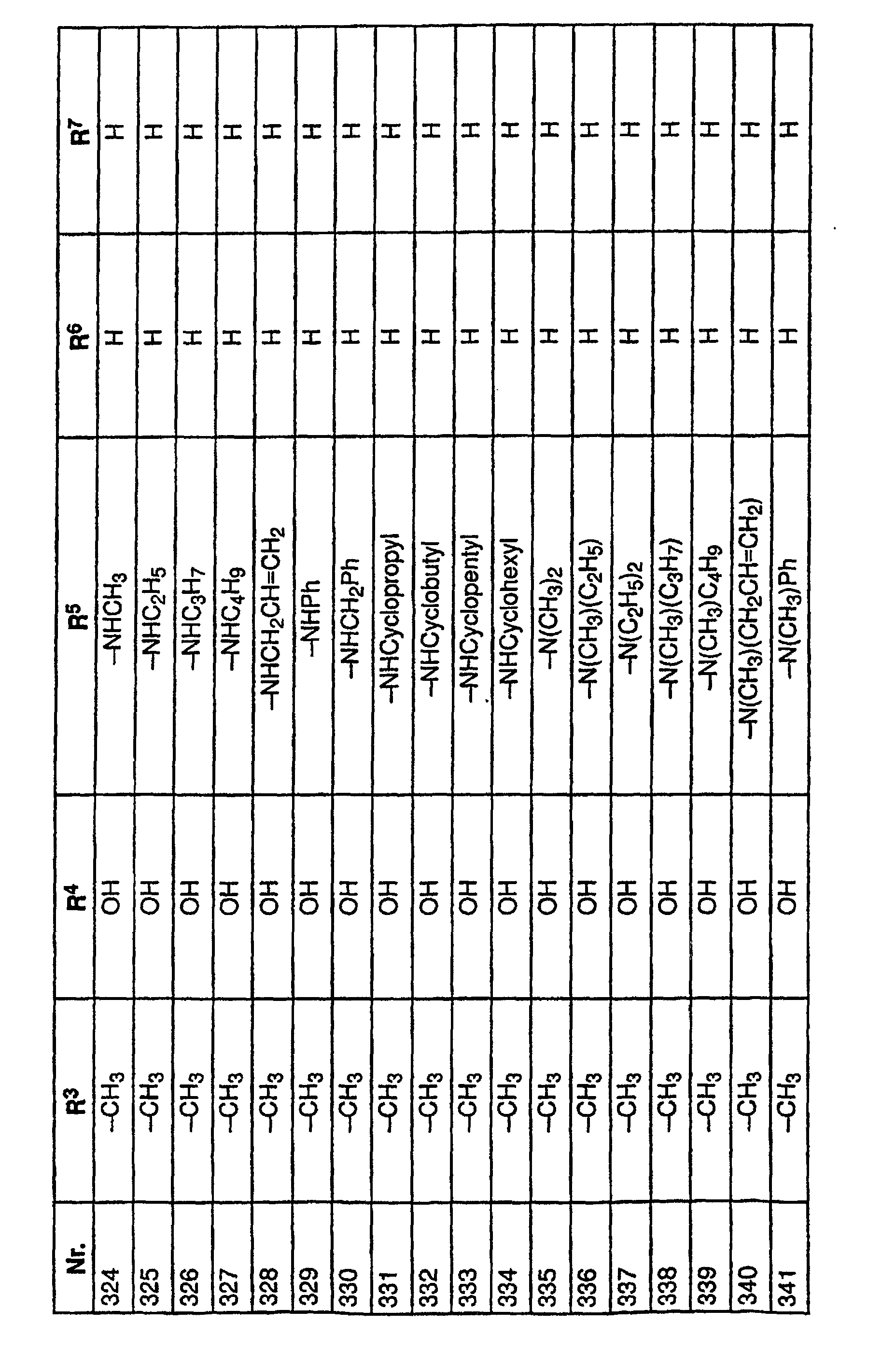

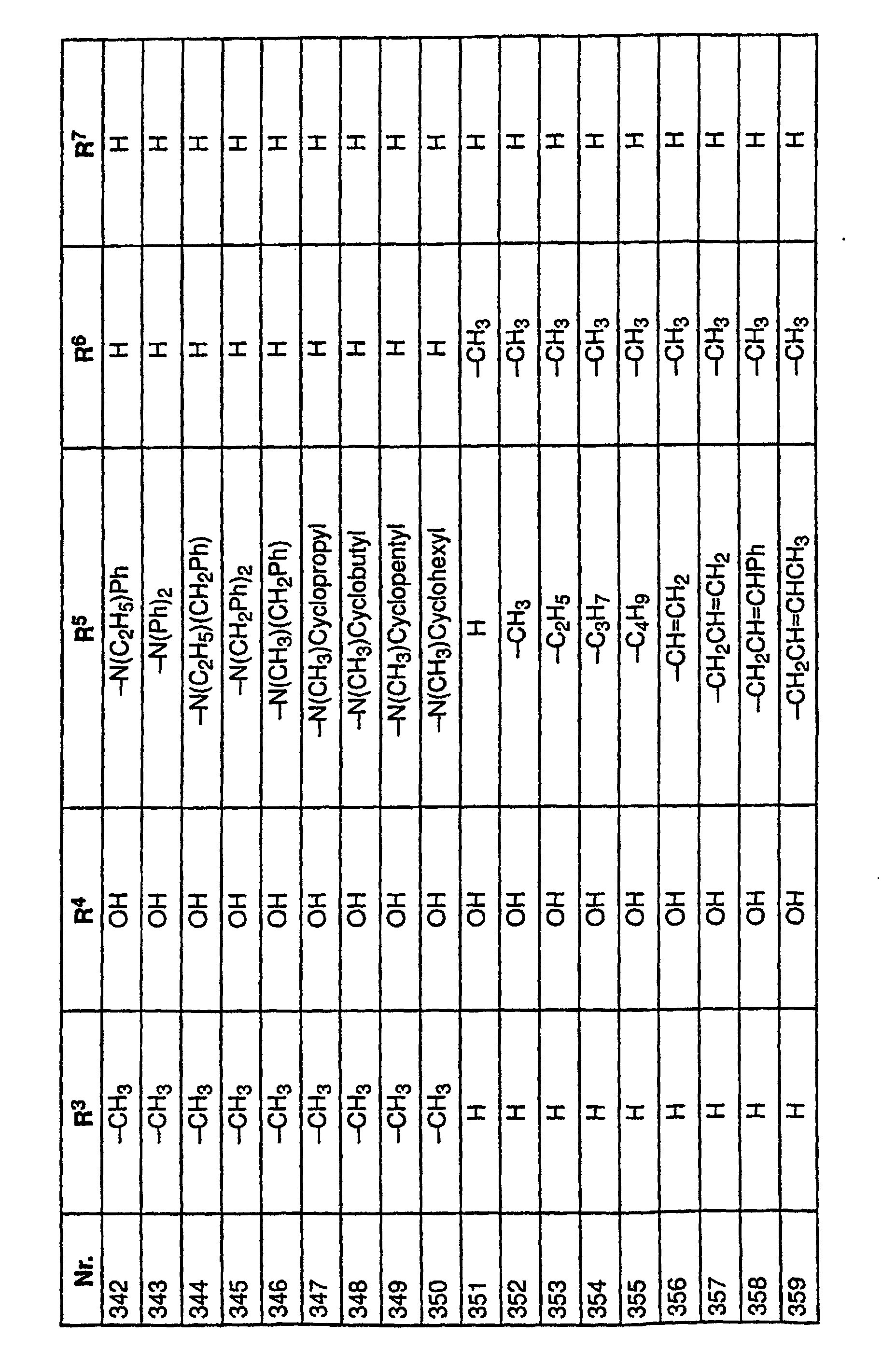

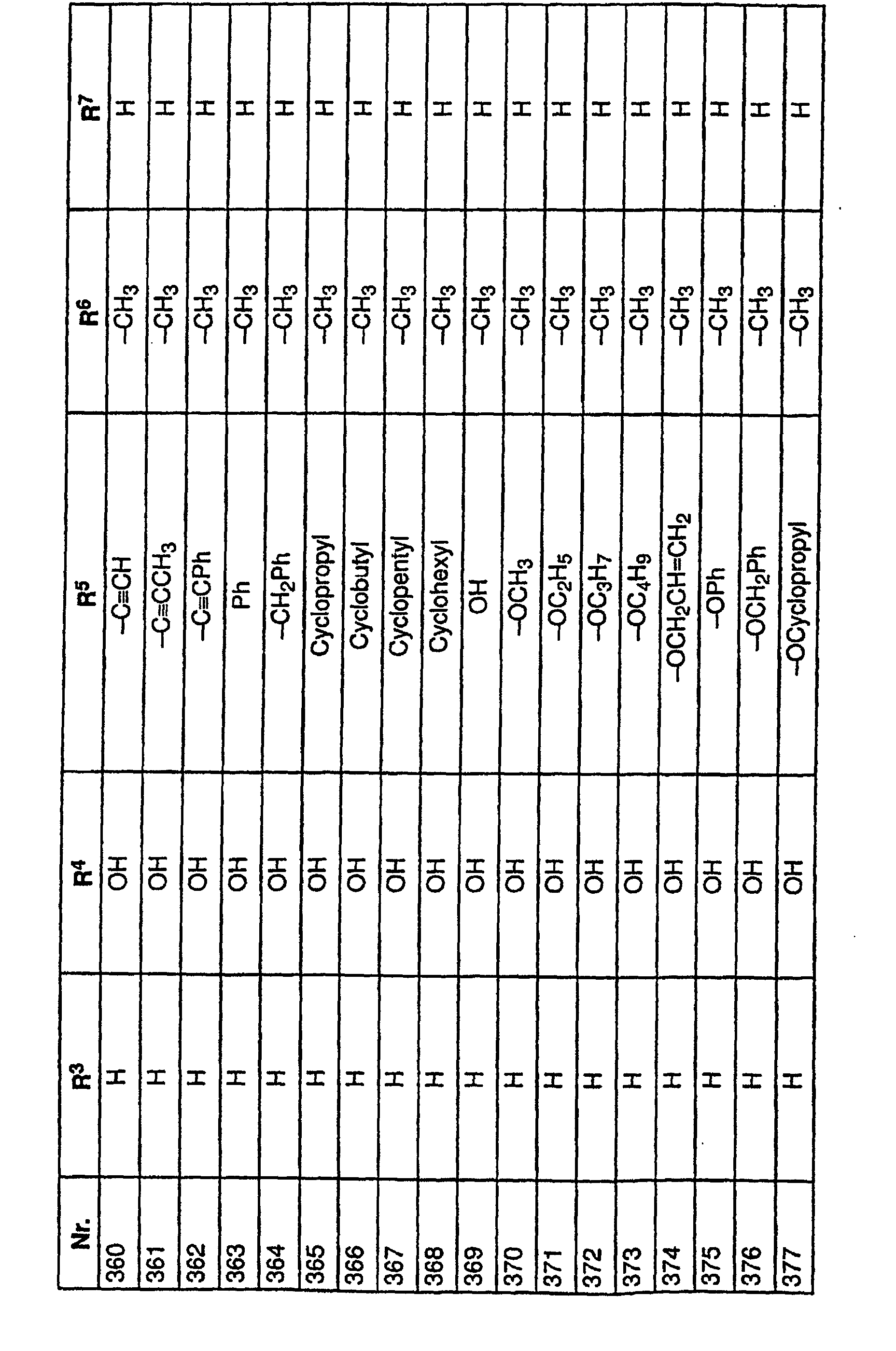

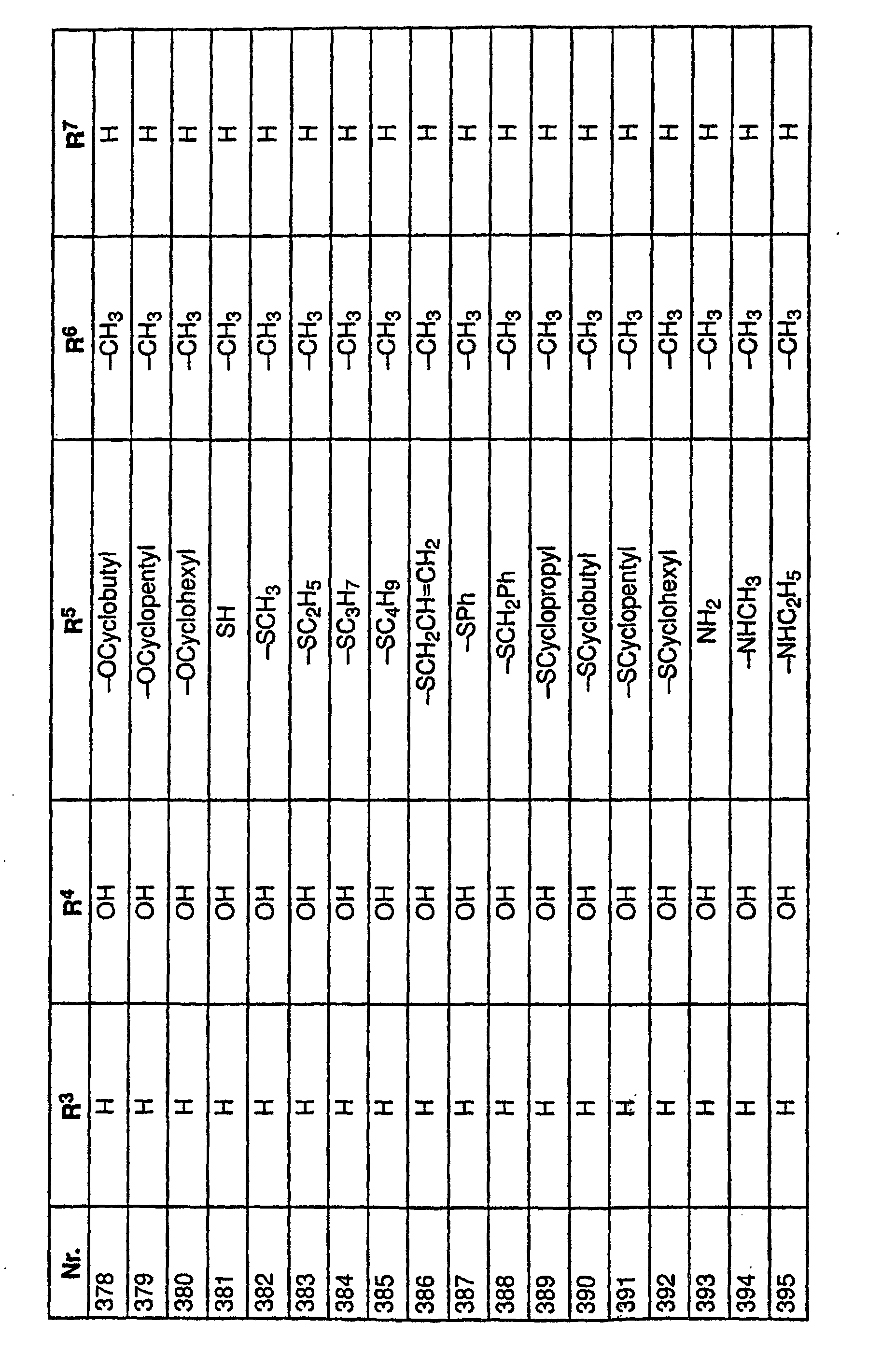

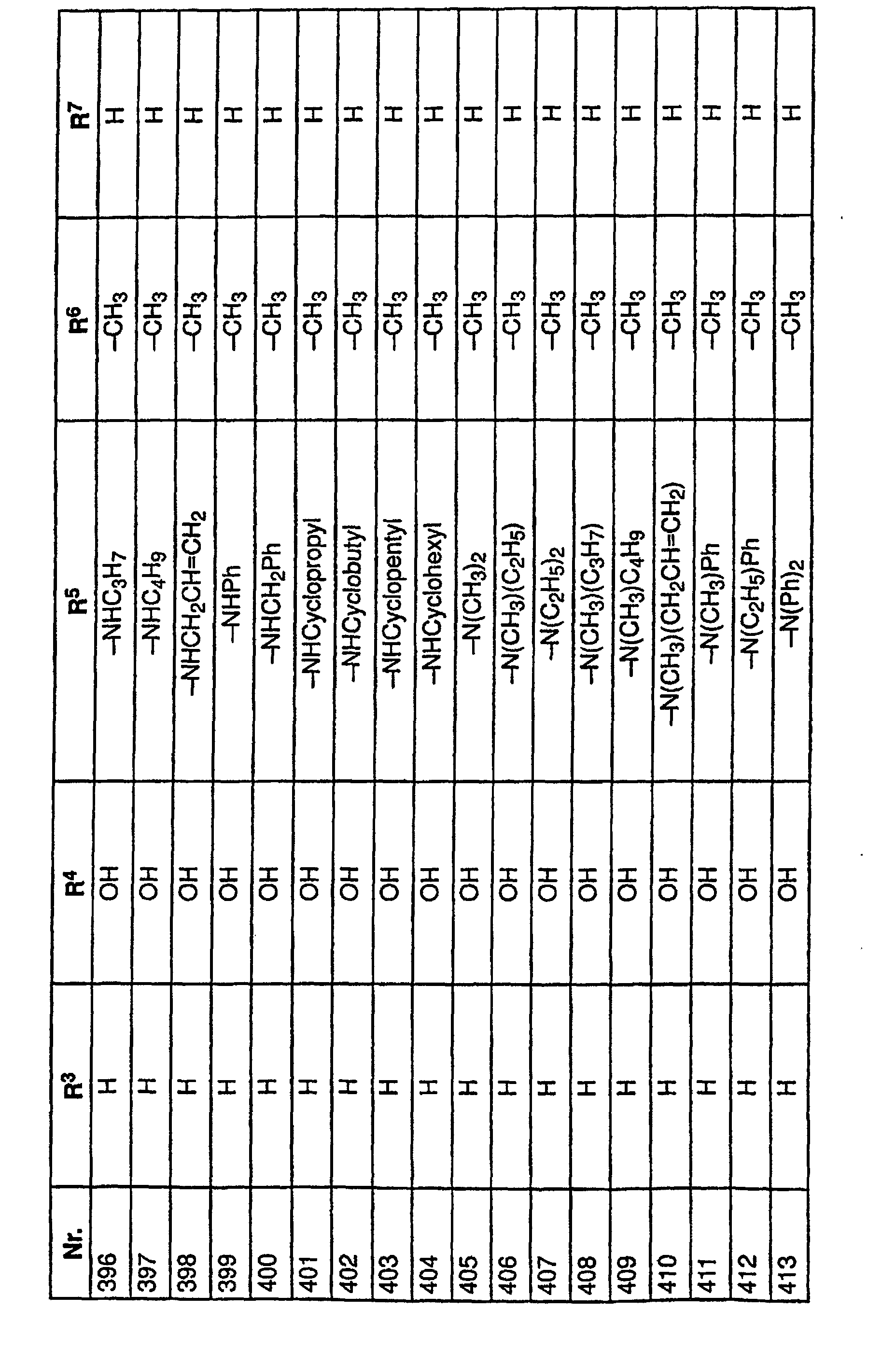

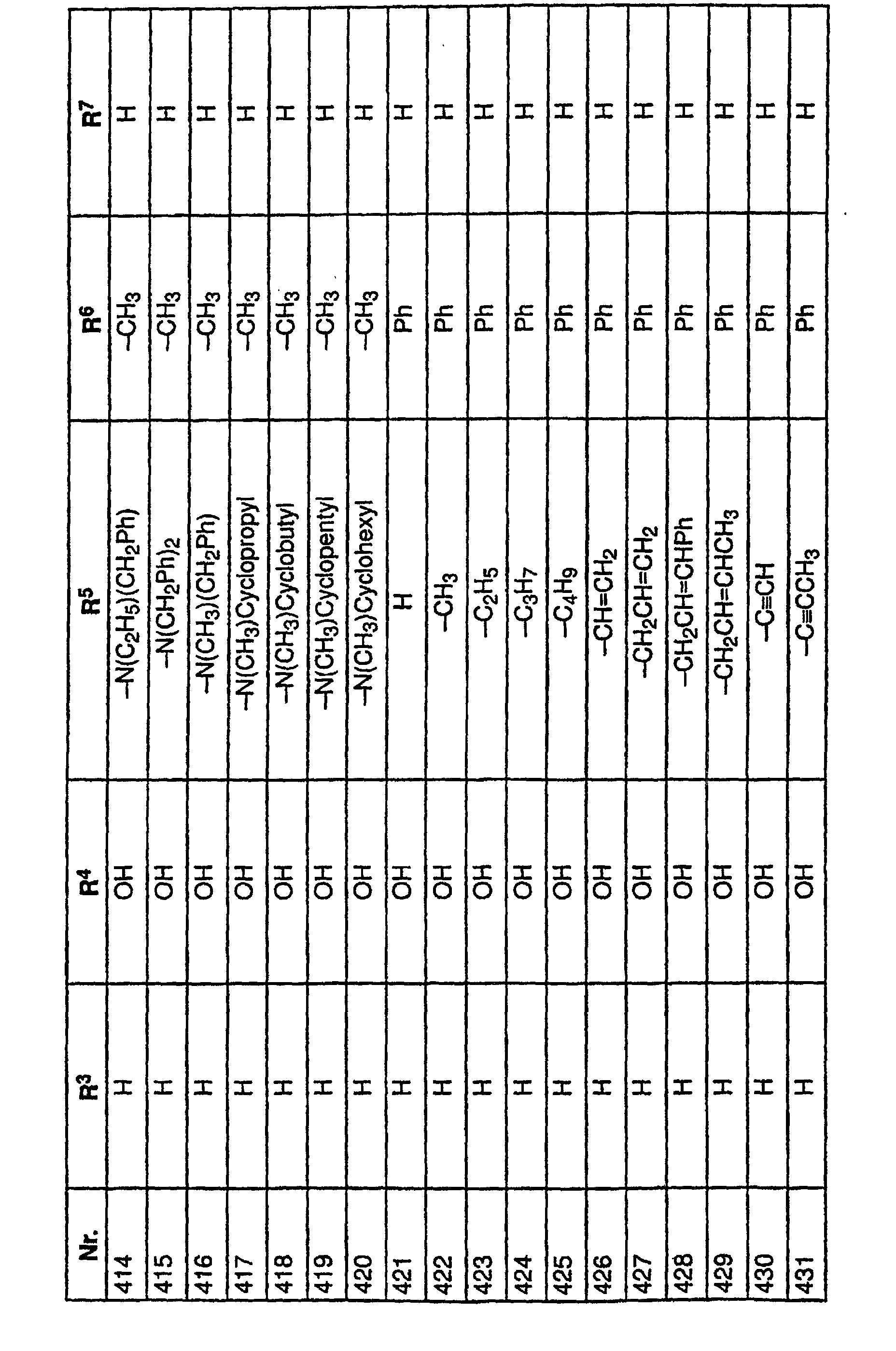

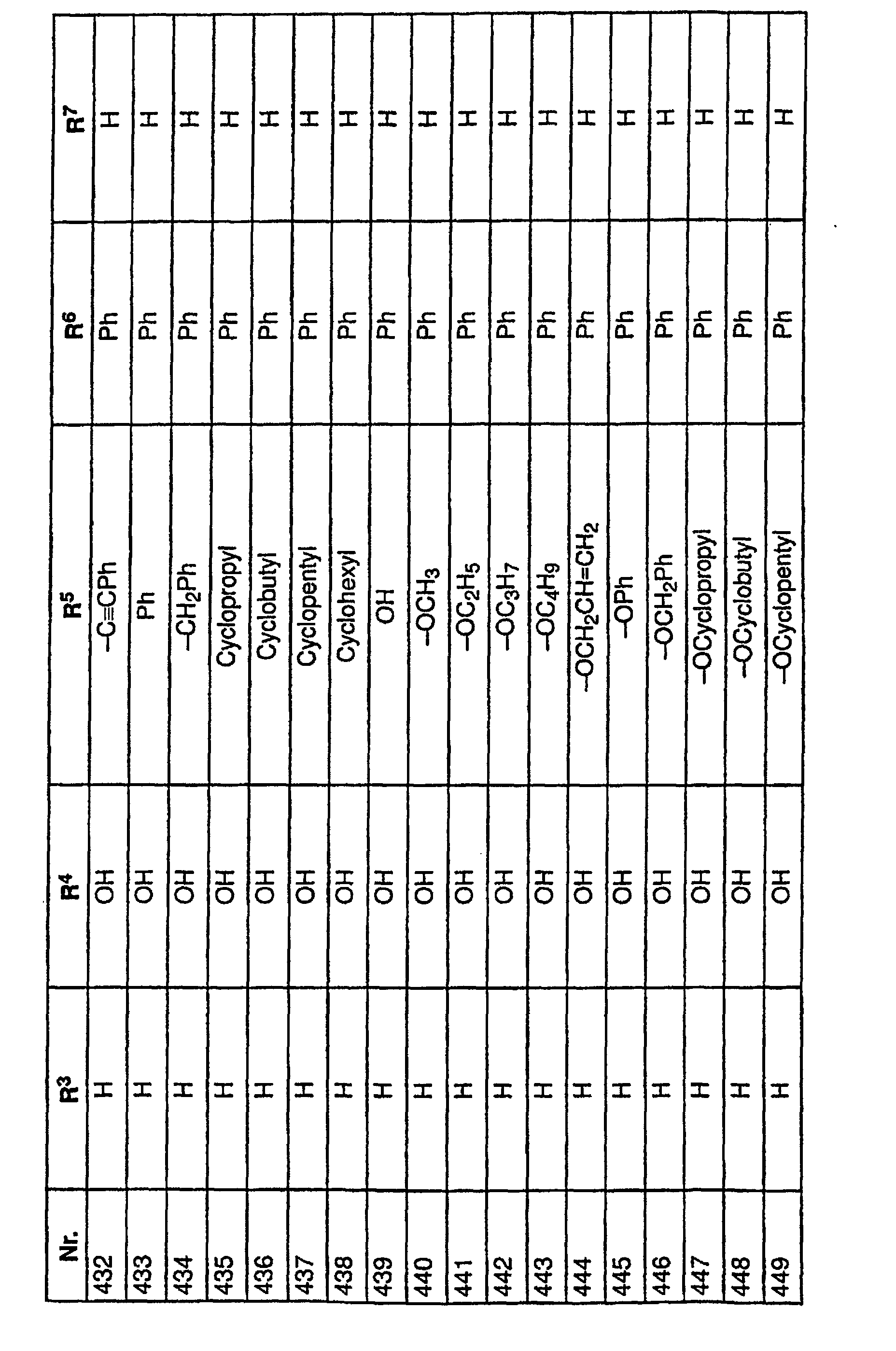

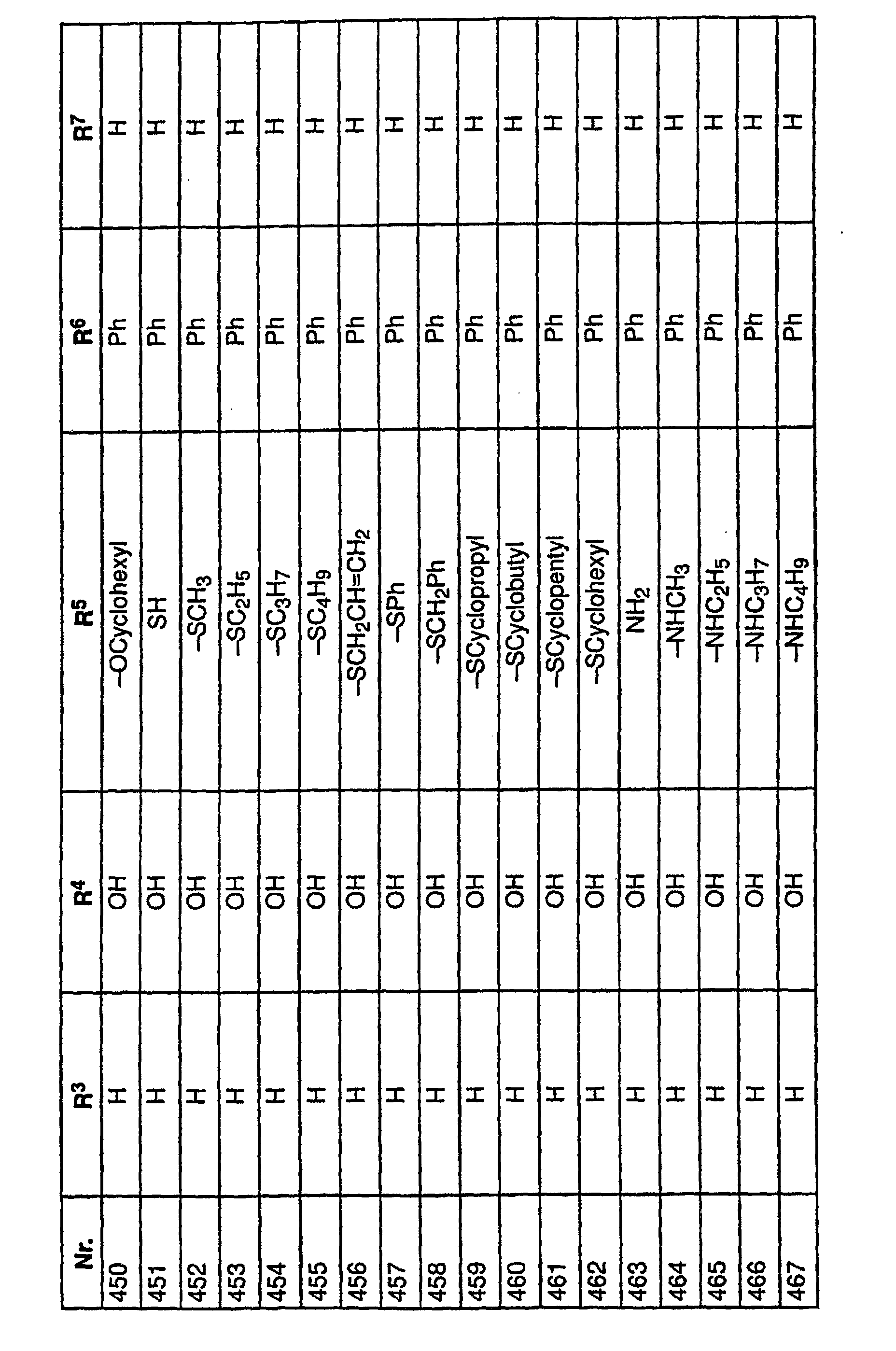

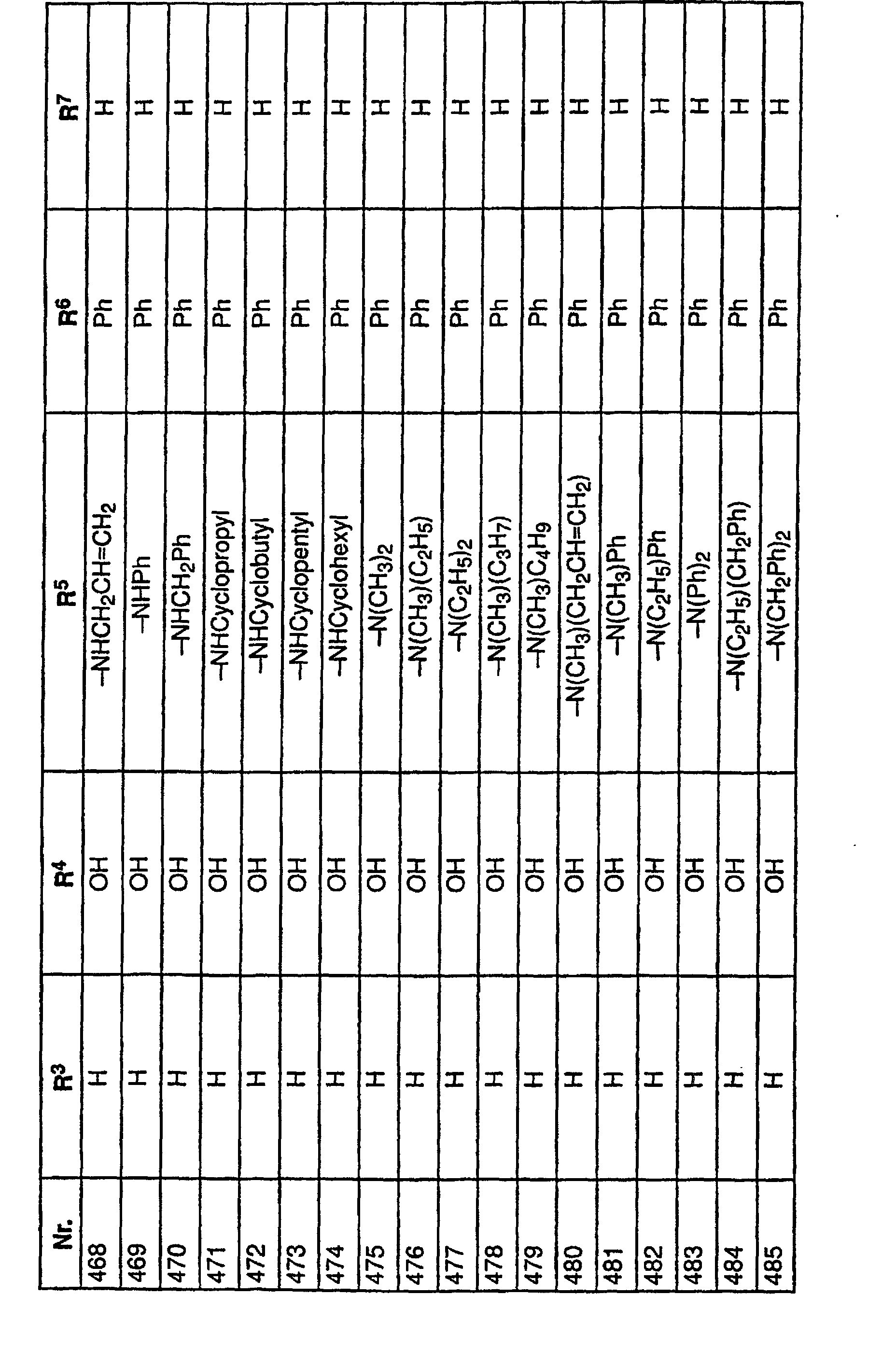

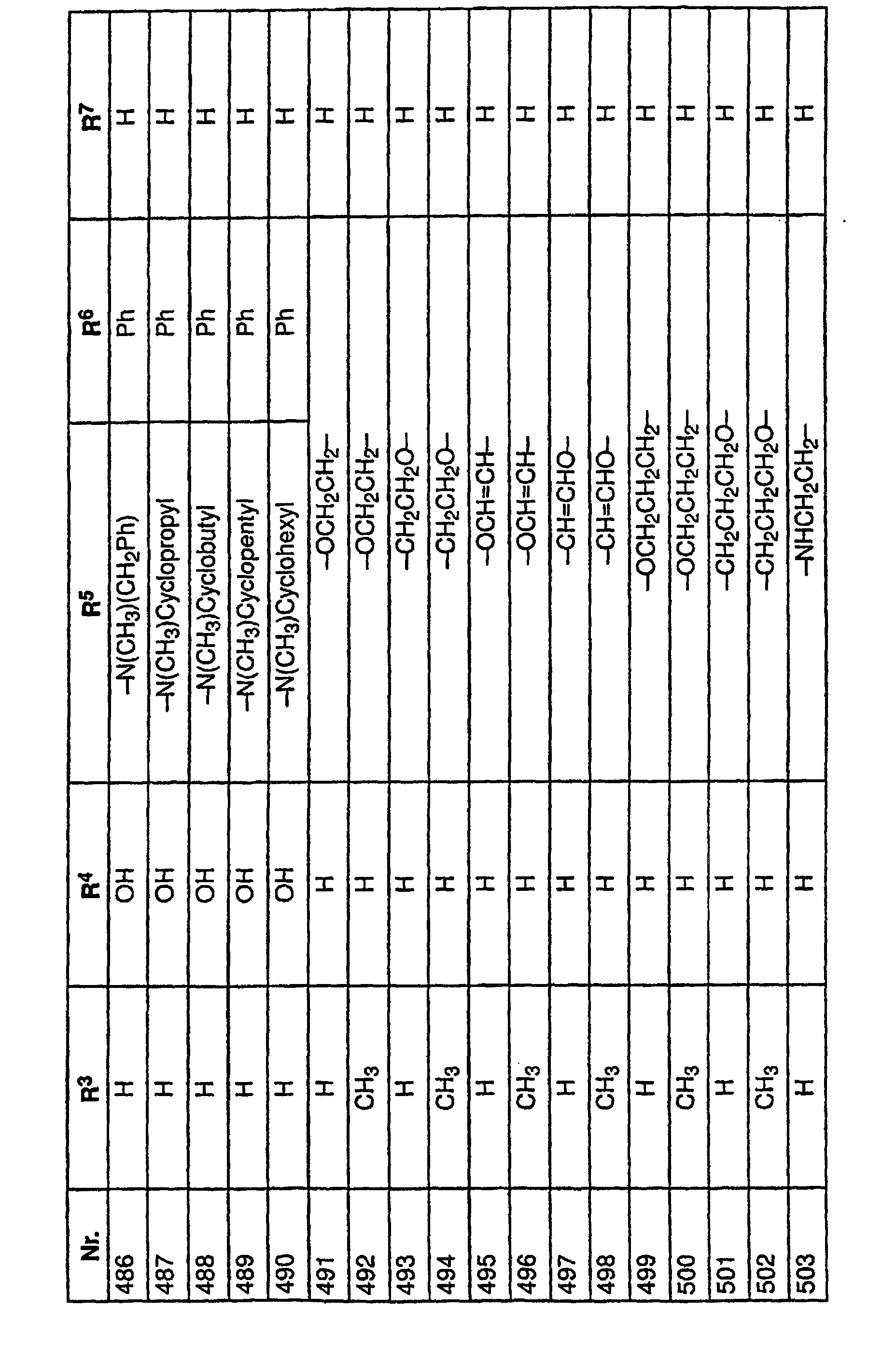

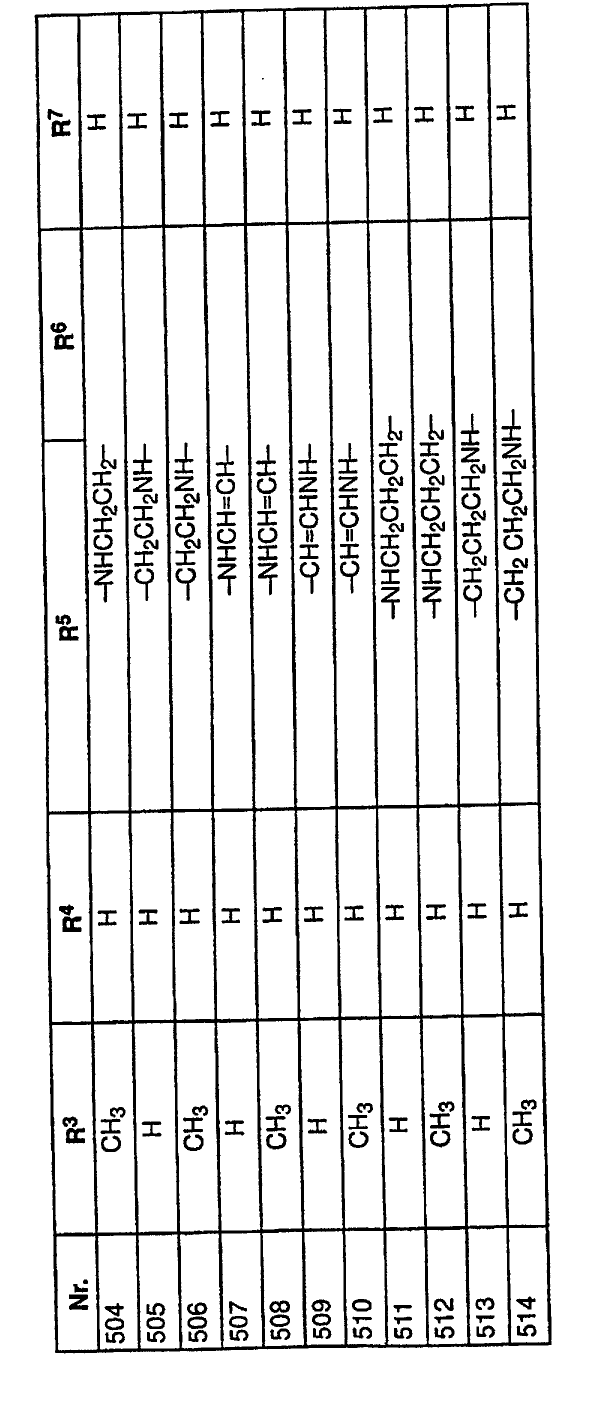

Die folgenden Tabellen 1 - 36 basieren auf den 2-Benzoyl-cyclohexan-1,3-dionen der Formel Ib

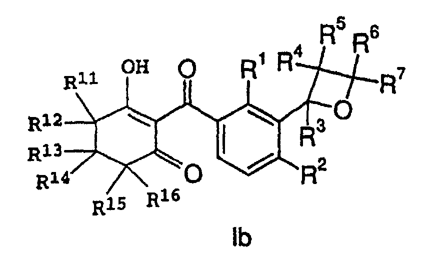

### Tabelle 1: Verbindungen 1.1-1.514

Verbindungen der allgemeinen Formel Ib, in der R¹ Chlor, R² Methylsulfonyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle A entsprechen.

### Tabelle 2: Verbindungen 2.1-2.514

Verbindungen der allgemeinen Formel Ib, in der R¹ und R² Chlor, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle A entsprechen.

### Tabelle 3: Verbindungen 3.1-3.514

Verbindungen der allgemeinen Formel Ib, in der R¹ Chlor, R² Trifluormethyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle A entsprechen.

### Tabelle 4: Verbindungen 4.1-4.514

Verbindungen der allgemeinen Formel Ib, in der R¹ Methyl, R² Chlor, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle A entsprechen.

### Tabelle 5: Verbindungen 5.1-5.514

Verbindungen der allgemeinen Formel Ib, in der R¹ Methyl, R² Methylsulfonyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle A entsprechen.

### Tabelle 6: Verbindungen 6.1-6.514

Verbindungen der allgemeinen Formel Ib, in der R¹ Methyl, R² Trifluormethyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle A entsprechen.

### Tabelle 7: Verbindungen 7.1-7.514

Verbindungen der allgemeinen Formel Ib, in der R¹ Chlor, R² Methylsulfonyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle A entsprechen.

### Tabelle 8: Verbindungen 8.1-8.514

Verbindungen der allgemeinen Formel Ib, in der R¹ und R² Chlor, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle A entsprechen.

### Tabelle 9: Verbindungen 9.1-9.514

Verbindungen der allgemeinen Formel Ib, in der R¹ Chlor, R² Trifluormethyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle A entsprechen.

### Tabelle 10: Verbindungen 10.1-10.514

Verbindungen der allgemeinen Formel Ib, in der R¹ Methyl, R² Chlor, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle A entsprechen.

### Tabelle 11: Verbindungen 11.1-11.514

Verbindungen der allgemeinen Formel Ib, in der R¹ Methyl, R² Methylsulfonyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle A entsprechen.

### Tabelle 12: Verbindungen 12.1-12.514

Verbindungen der allgemeinen Formel Ib, in der R¹ Methyl, R² Trifluormethyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle A entsprechen.

### Tabelle 13: Verbindungen 13.1-13.514

Verbindungen der allgemeinen Formel Ib, in der R¹ Chlor, R² Methylsulfonyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle A entsprechen.

### Tabelle 14: Verbindungen 14.1-14.514

Verbindungen der allgemeinen Formel Ib, in der R¹ und R² Chlor, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle A entsprechen.

### Tabelle 15: Verbindungen 15.1-15.514

Verbindungen der allgemeinen Formel Ib, in der R¹ Chlor, R² Trifluormethyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle A entsprechen.

### Tabelle 16: Verbindungen 16.1-16.514

Verbindungen der allgemeinen Formel Ib, in der R¹ Methyl, R² Chlor, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle A entsprechen.

### Tabelle 17: Verbindungen 17.1-17.514

Verbindungen der allgemeinen Formel Ib, in der R¹ Methyl, R² Methylsulfonyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle A entsprechen.

### Tabelle 18: Verbindungen 18.1-18.514

Verbindungen der allgemeinen Formel Ib, in der R¹ Methyl, R² Trifluormethyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle A entsprechen.

### Tabelle 19: Verbindungen 19.1-19.514

Verbindungen der allgemeinen Formel Ib, in der R¹ Chlor, R² Methylsulfonyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle A entsprechen.

### Tabelle 20: Verbindungen 20.1-20.514

Verbindungen der allgemeinen Formel Ib, in der R¹ und R² Chlor, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle A entsprechen.

### Tabelle 21: Verbindungen 21.1-21.514

Verbindungen der allgemeinen Formel Ib, in der R¹ Chlor, R² Trifluormethyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle A entsprechen.

### Tabelle 22: Verbindungen 22.1-22.514

Verbindungen der allgemeinen Formel Ib, in der R¹ Methyl, R² Chlor, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle A entsprechen.

### Tabelle 23: Verbindungen 23.1-23.514

Verbindungen der allgemeinen Formel Ib, in der R¹ Methyl, R² Methylsulfonyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle A entsprechen.

### Tabelle 24: Verbindungen 24.1-24.514

Verbindungen der allgemeinen Formel Ib, in der R¹ Methyl, R² Trifluormethyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle A entsprechen.

### Tabelle 25: Verbindungen 25.1-25.514

Verbindungen der allgemeinen Formel Ib, in der R¹ Methylsulfonyl, R¹¹, R¹², R¹³, R¹⁵ und R¹⁶ Wasserstoff, R¹⁴ Methyl bedeutet, die Substituenten R³ bis R⁷ für jede einzelne Verbindung jeweils einer Zeile der Tabelle A entsprechen.

### Tabelle 26: Verbindungen 26.1-26.514

Verbindungen der allgemeinen Formel Ib, in der R¹ und R² Chlor, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle A entsprechen.

### Tabelle 27: Verbindungen 27.1-27.514

Verbindungen der allgemeinen Formel Ib, in der R¹ Chlor, R² Trifluormethyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle A entsprechen.

### Tabelle 28: Verbindungen 28.1-28.514

Verbindungen der allgemeinen Formel Ib, in der R¹ Methyl, R² Chlor, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle A entsprechen.

### Tabelle 29: Verbindungen 29.1-29.514

Verbindungen der allgemeinen Formel Ib, in der R¹ Methyl, R² Methylsulfonyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle A entsprechen.

### Tabelle 30: Verbindungen 30.1-30.514

Verbindungen der allgemeinen Formel Ib, in der R¹ Methyl, R² Trifluormethyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle A entsprechen.

### Tabelle 31: Verbindungen 31.1-31.514

Verbindungen der allgemeinen Formel Ib, in der R¹ Chlor, R² Methylsulfonyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle A entsprechen.

### Tabelle 32: Verbindungen 32.1-32.514

Verbindungen der allgemeinen Formel Ib, in der R¹ und R² Chlor, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle A entsprechen.

### Tabelle 33: Verbindungen 33.1-33.514

Verbindungen der allgemeinen Formel Ib, in der R¹ Chlor, R² Trifluormethyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle A entsprechen.

### Tabelle 34: Verbindungen 34.1-34.514

Verbindungen der allgemeinen Formel Ib, in der R¹ Methyl, R² Chlor, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle A entsprechen.

### Tabelle 35: Verbindungen 35.1-35.514

Verbindungen der allgemeinen Formel Ib, in der R¹ Methyl, R² Methylsulfonyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle A entsprechen.

### Tabelle 36: Verbindungen 36.1-36.514

Verbindungen der allgemeinen Formel Ib, in der R¹ Methyl, R² Trifluormethyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle A entsprechen.

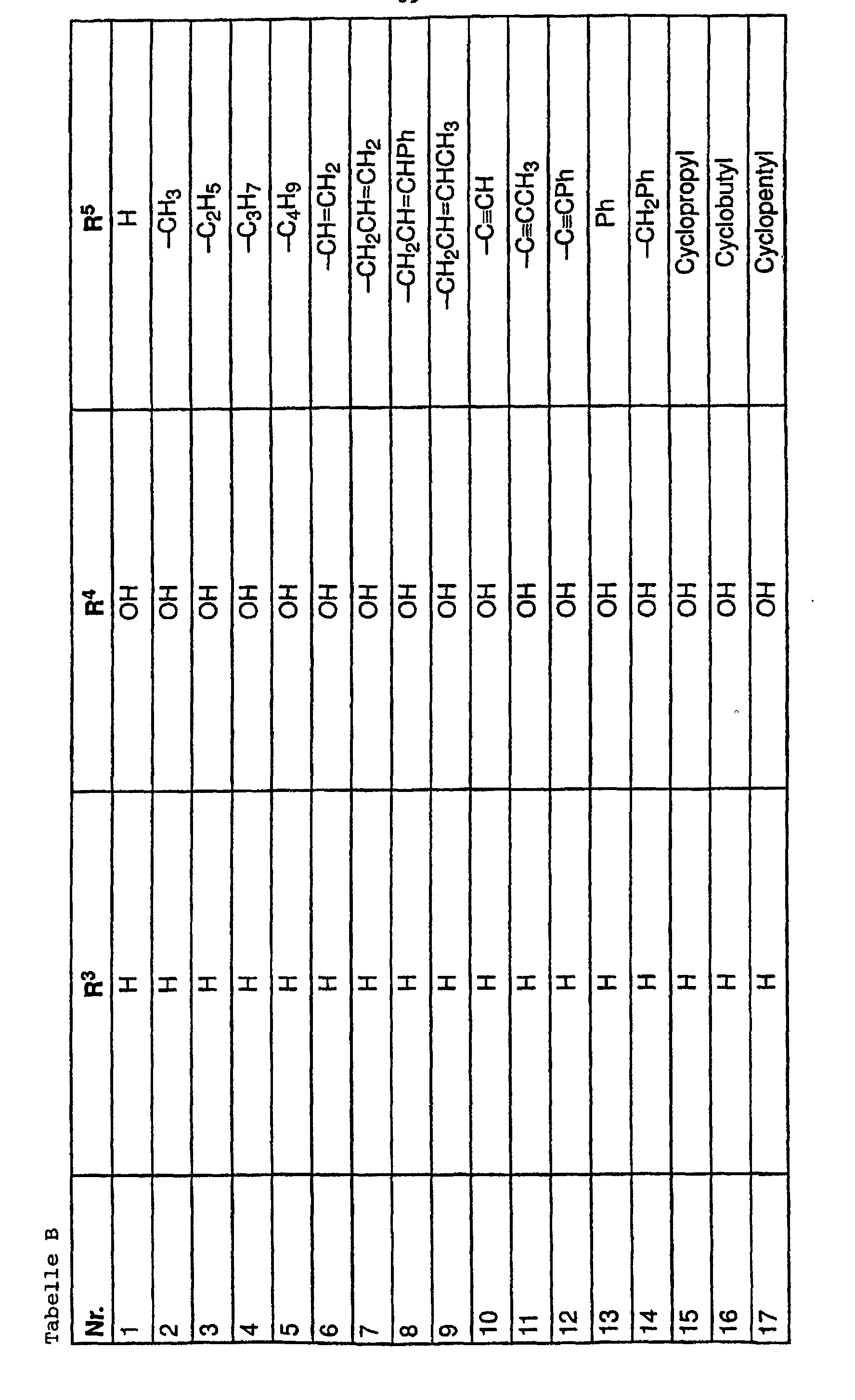

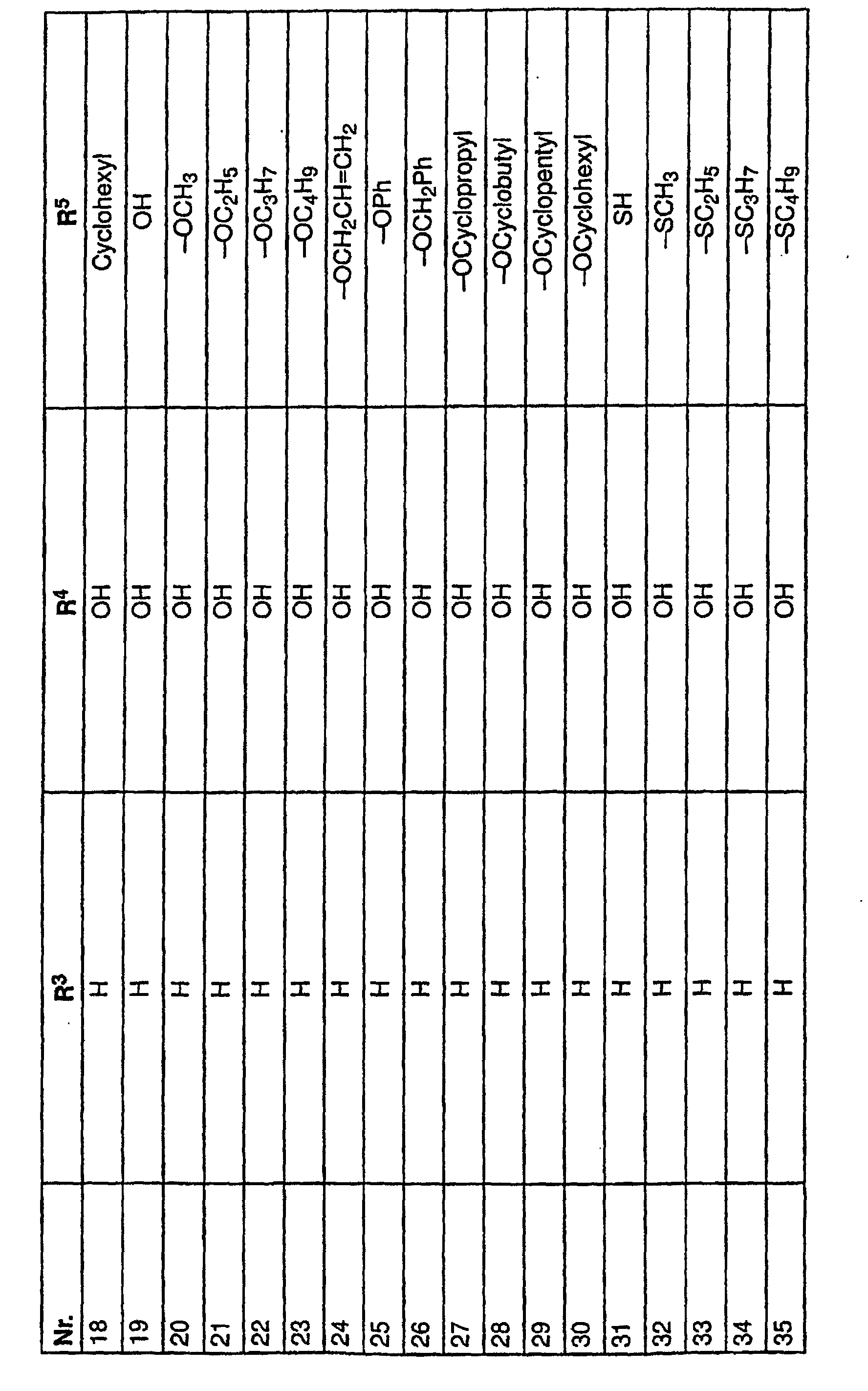

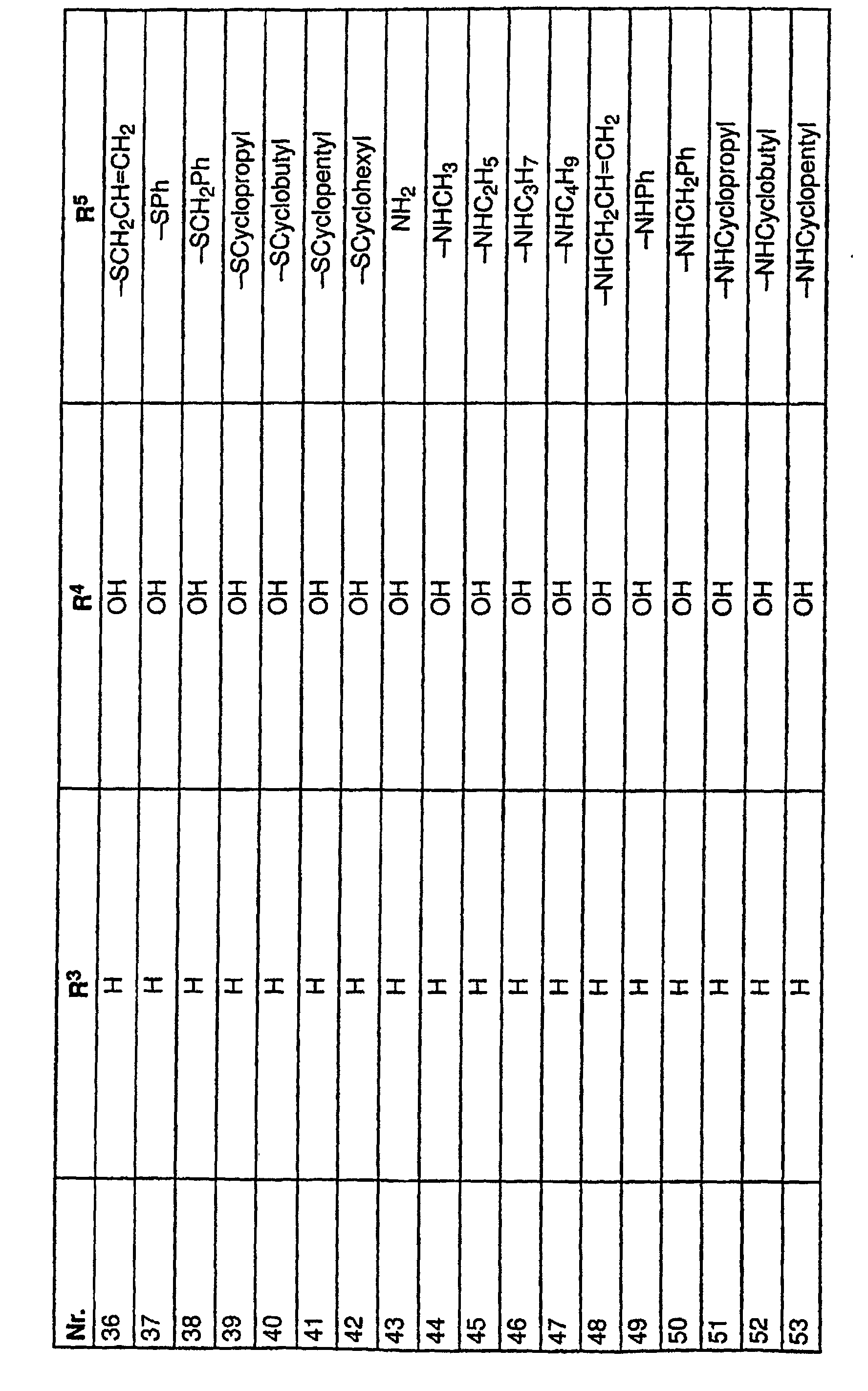

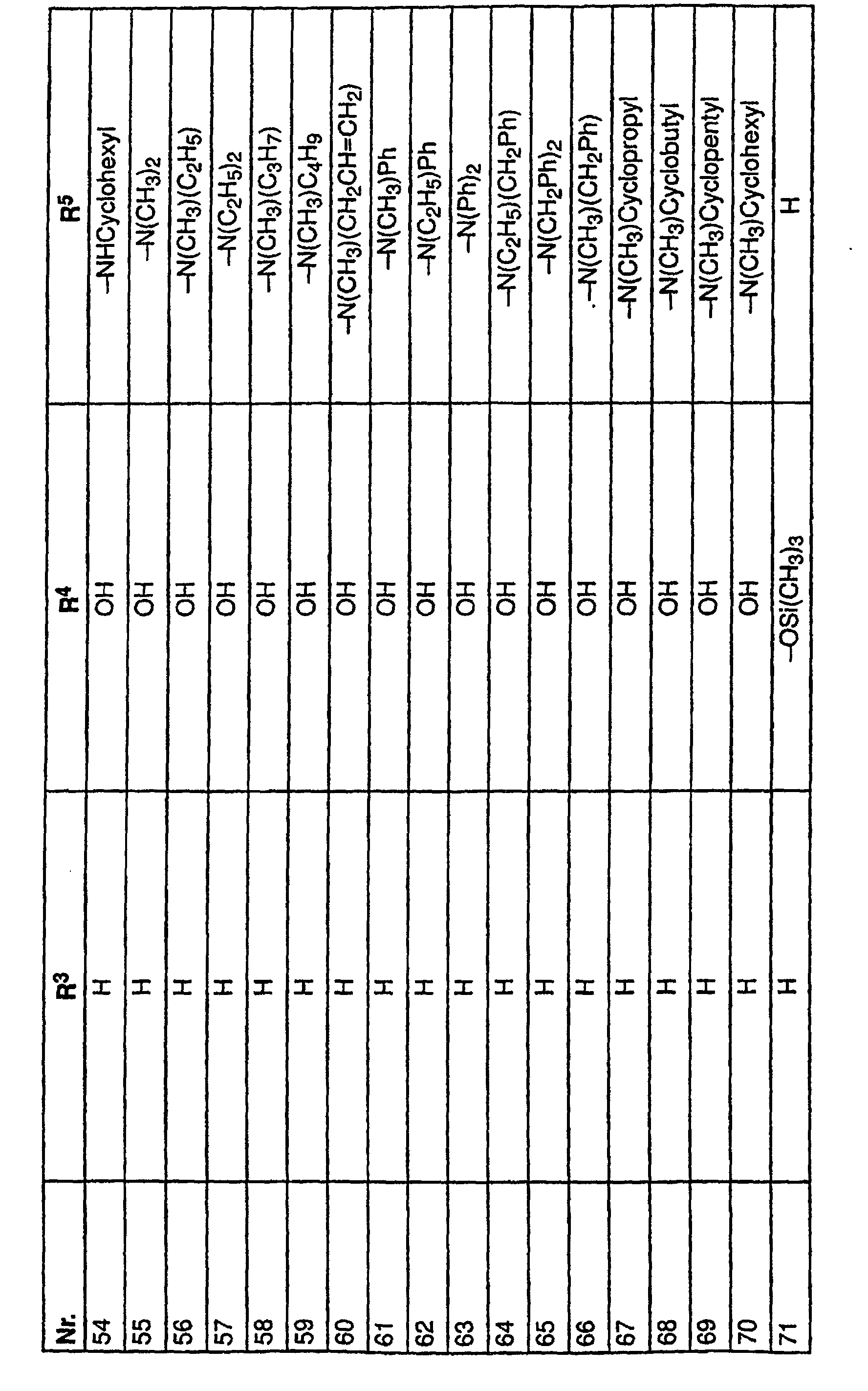

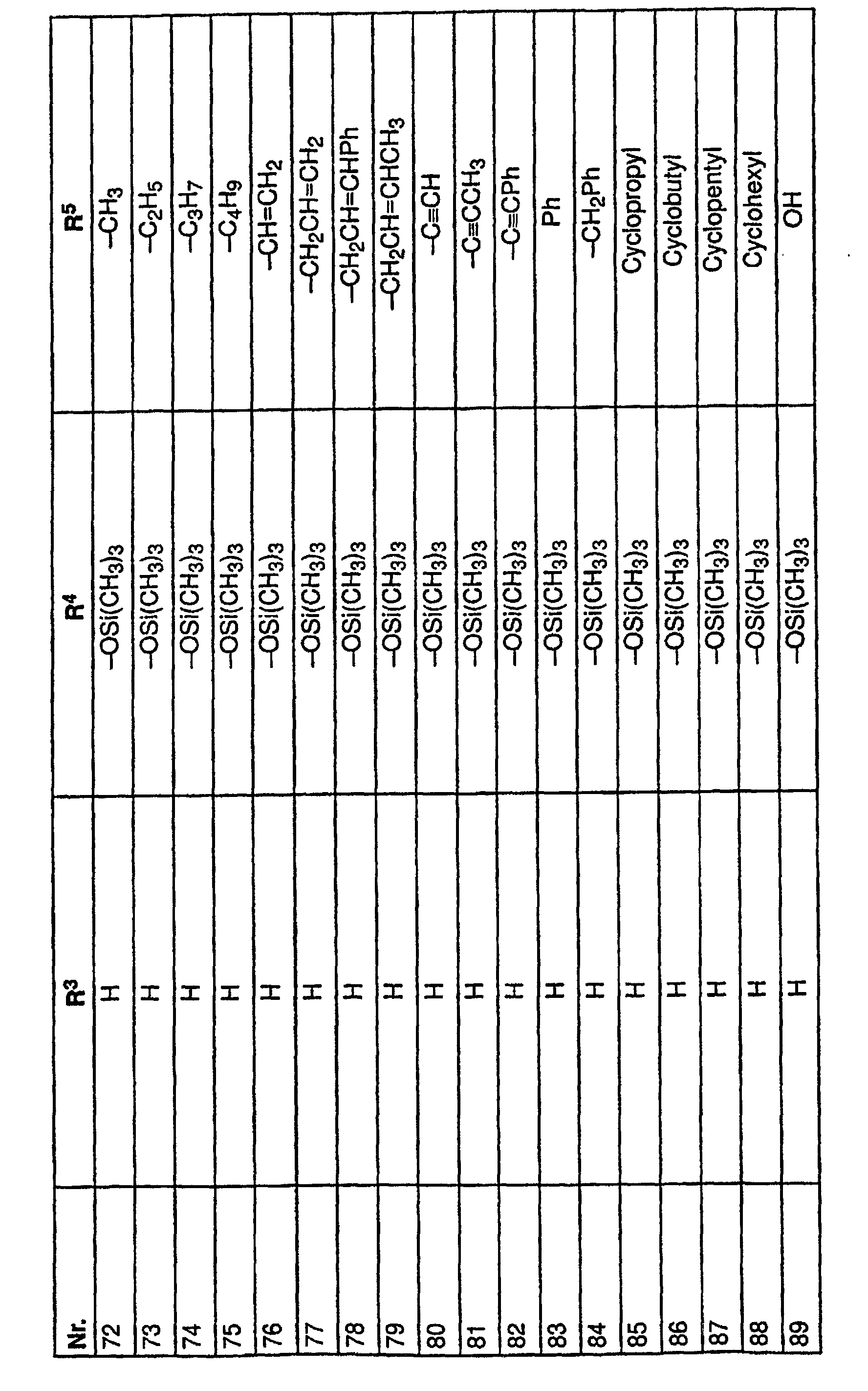

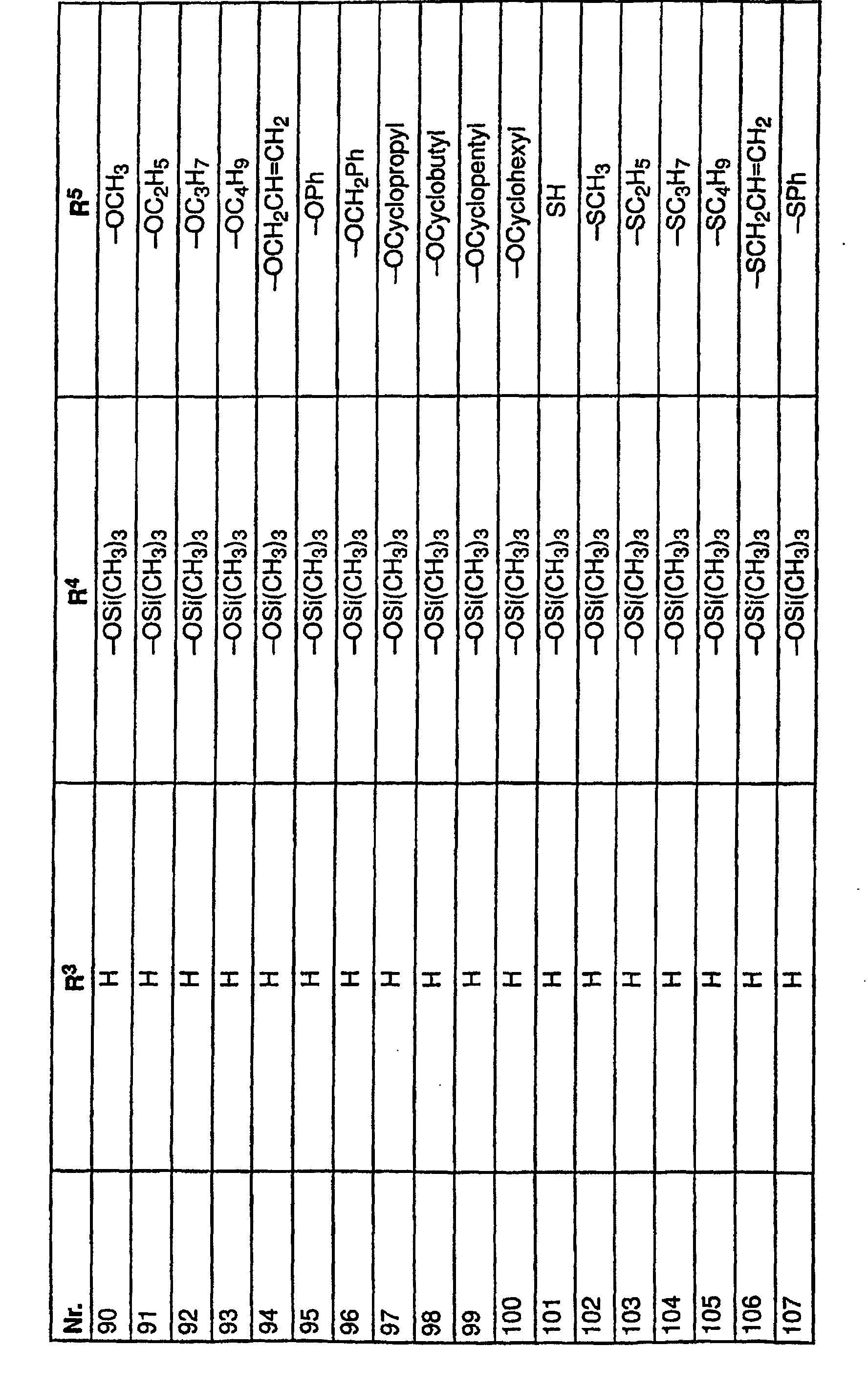

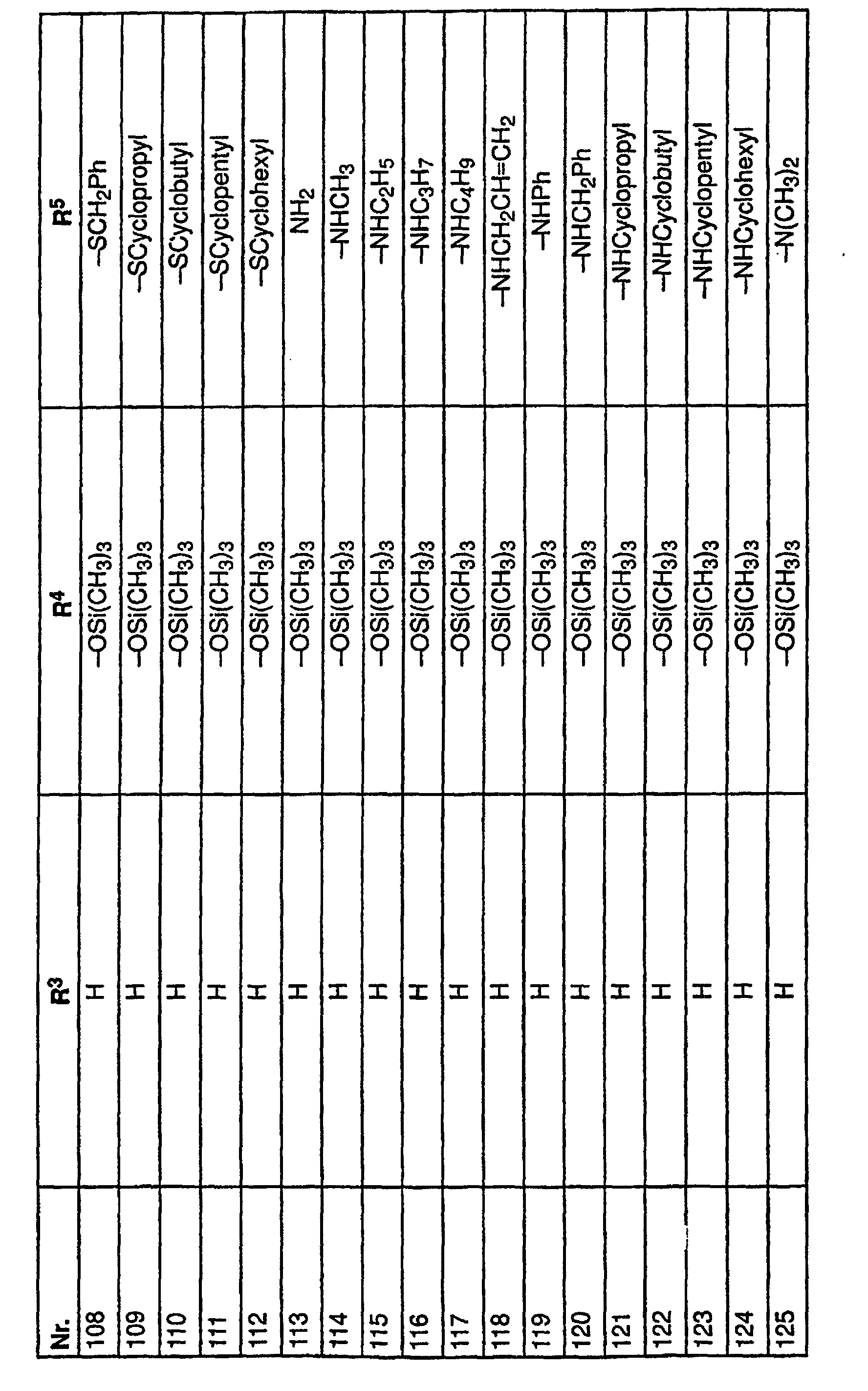

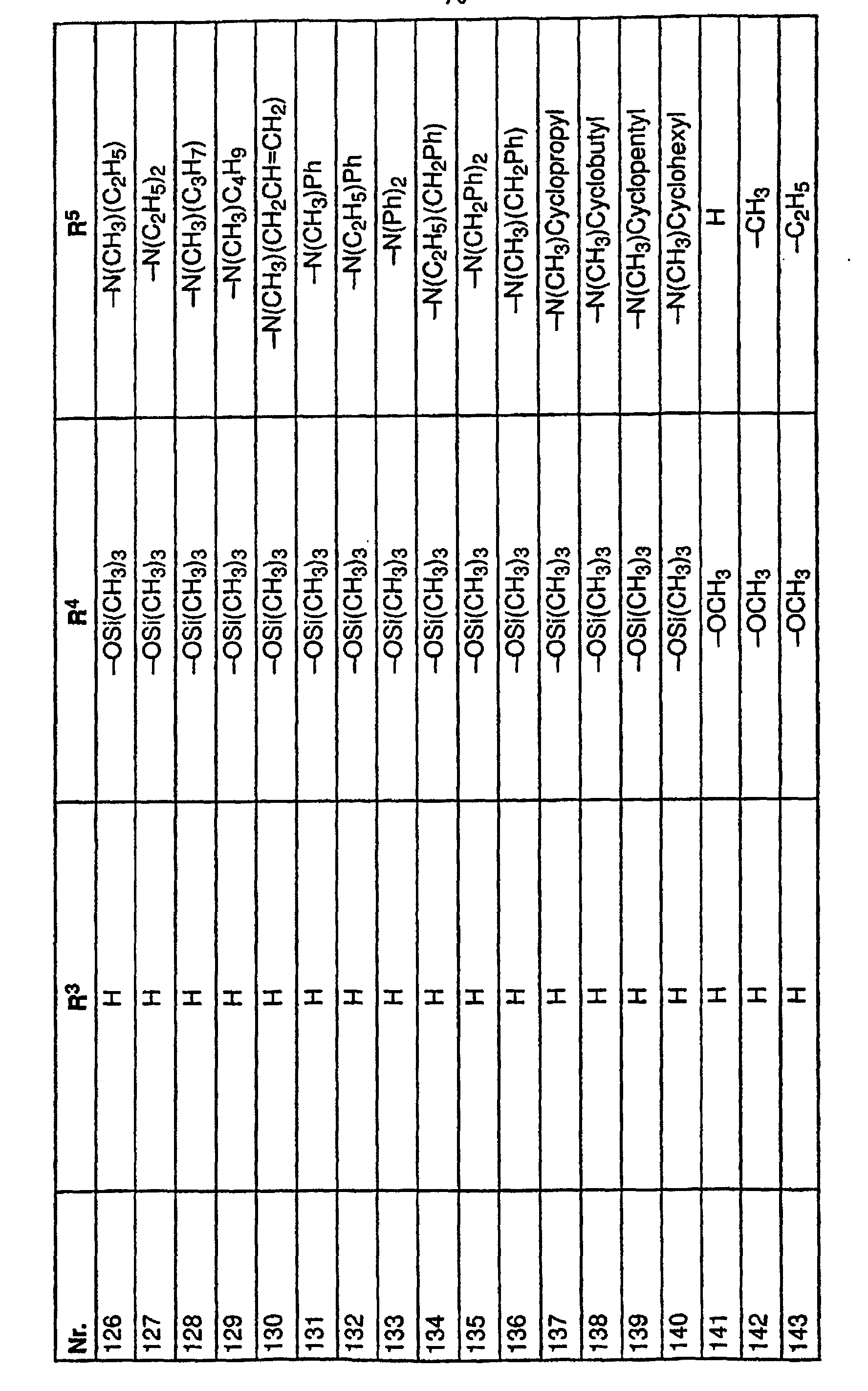

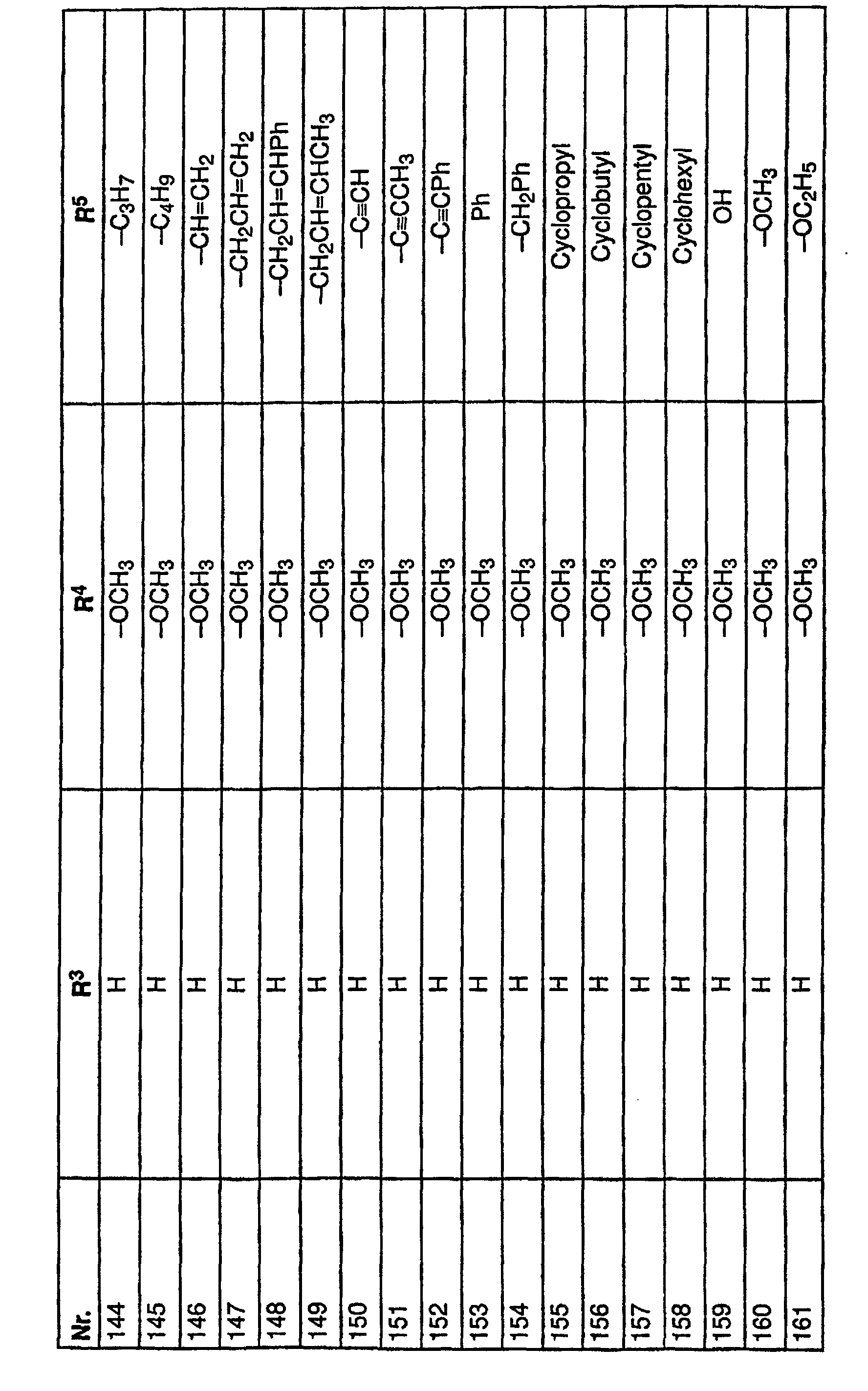

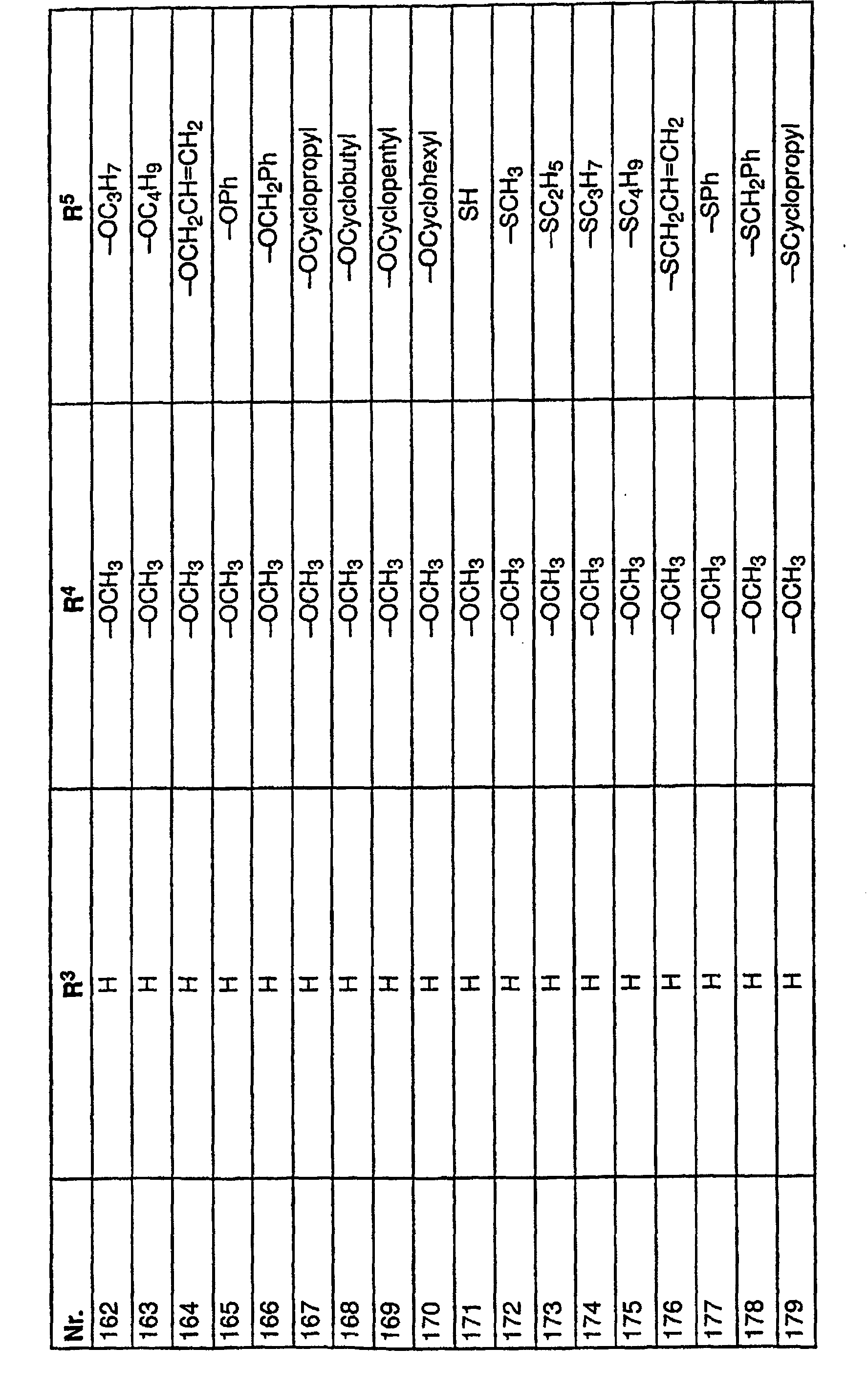

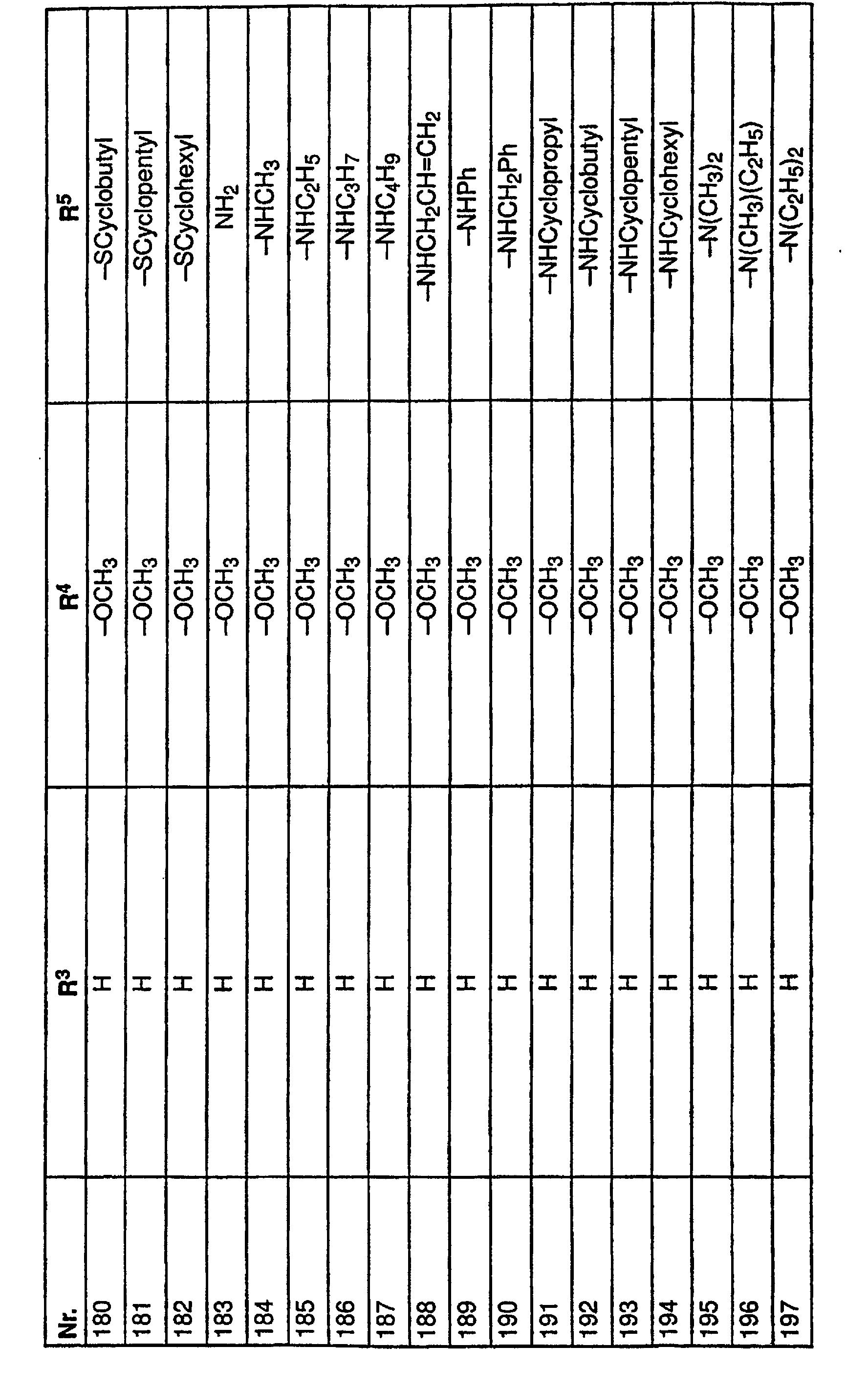

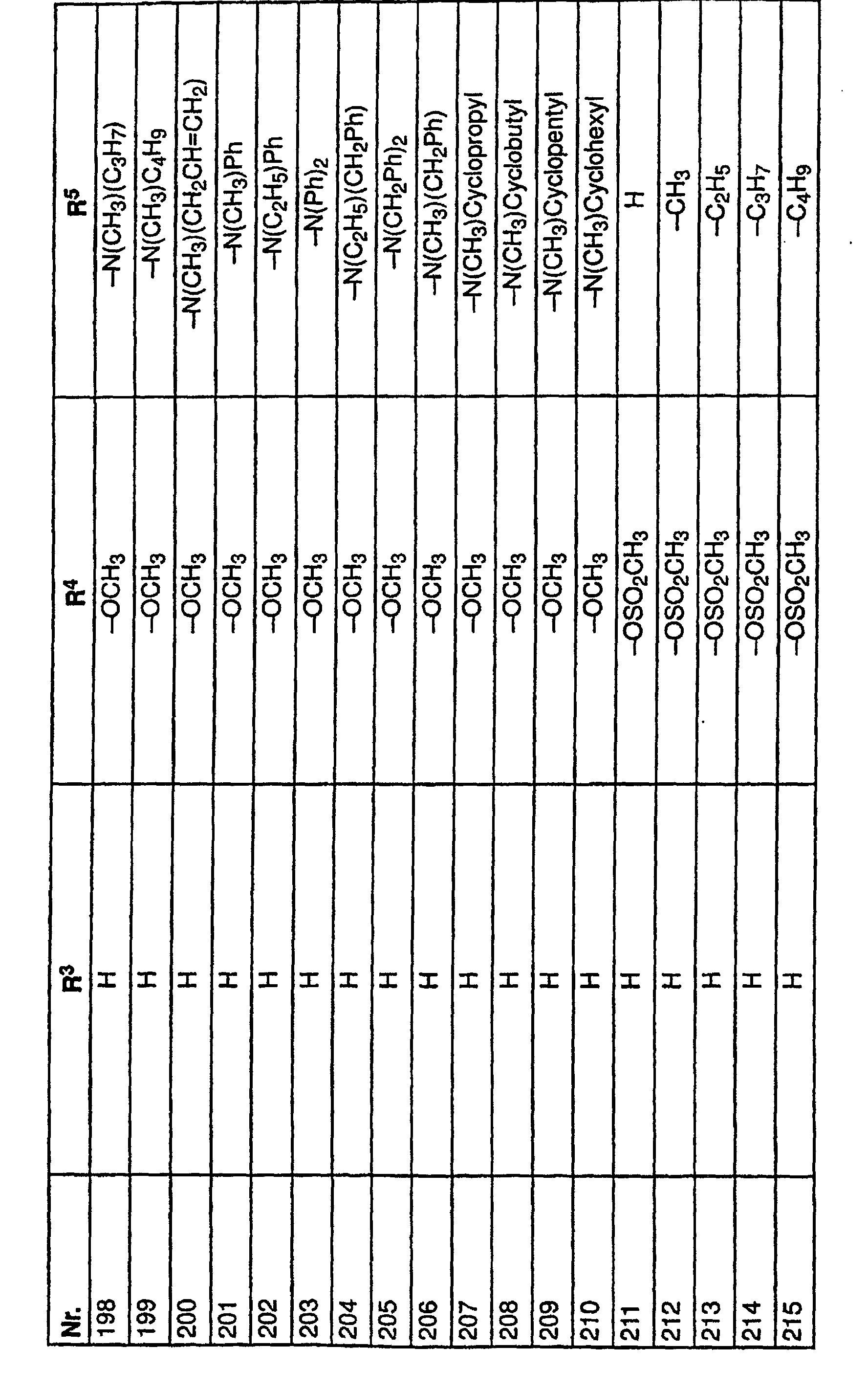

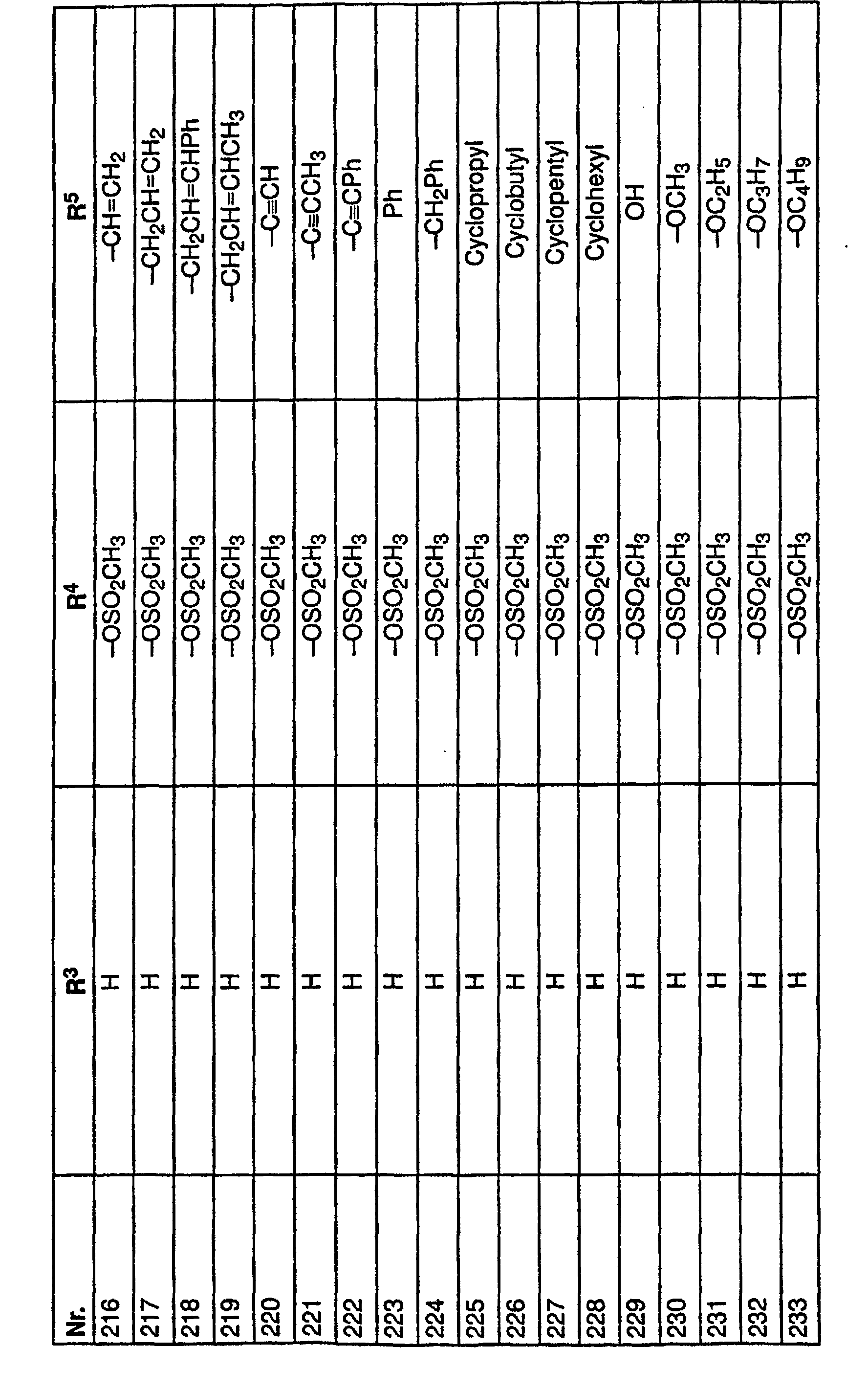

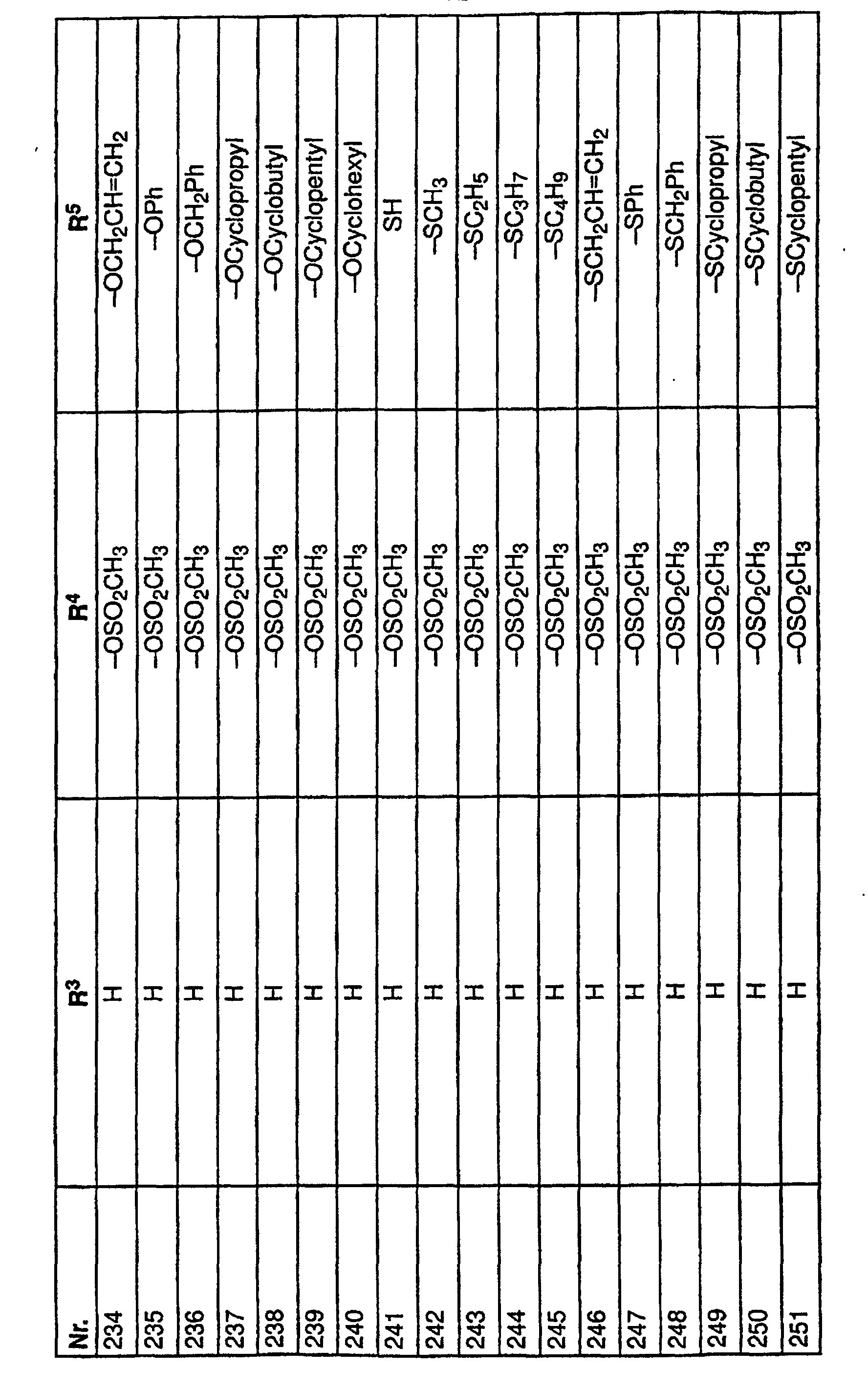

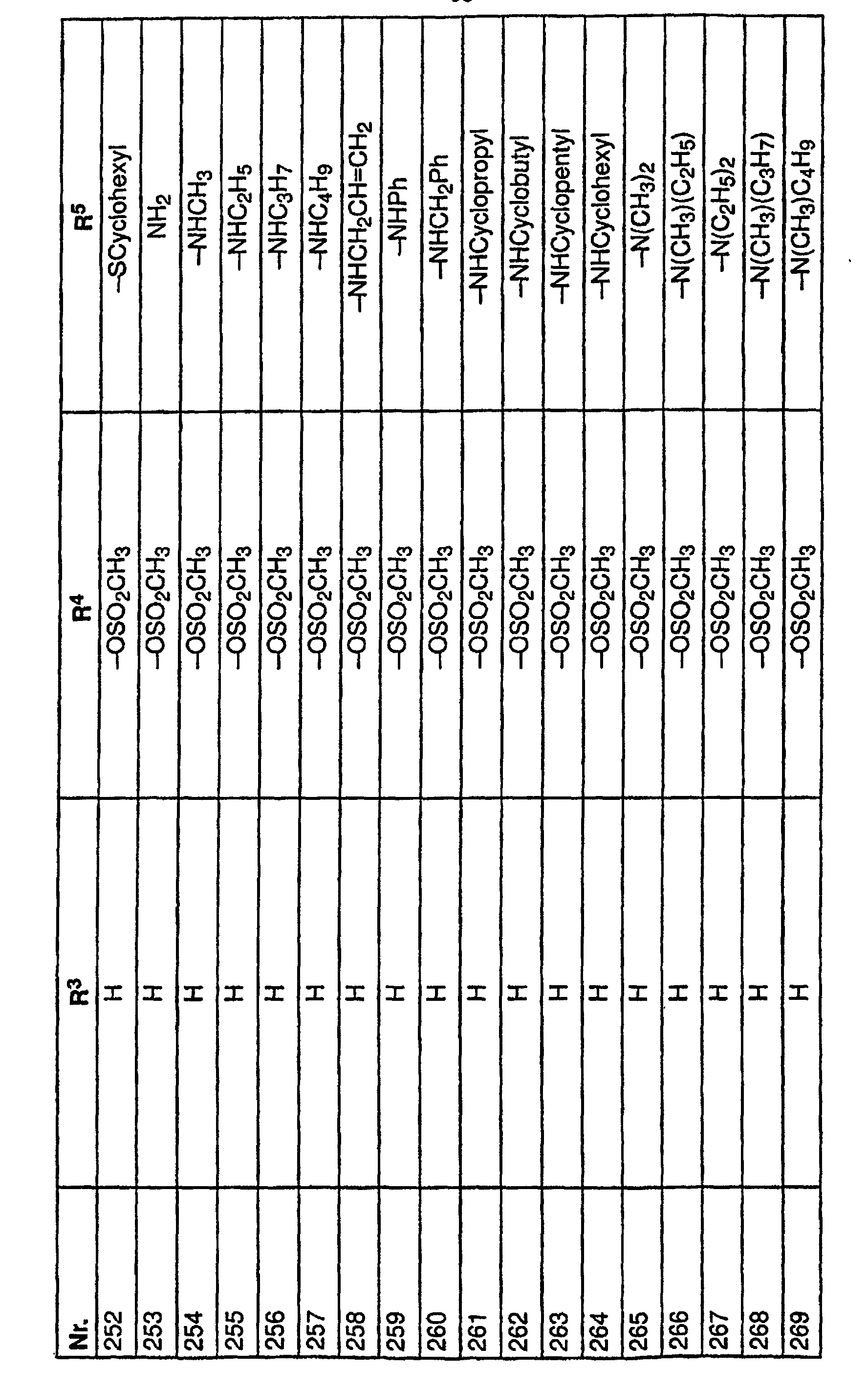

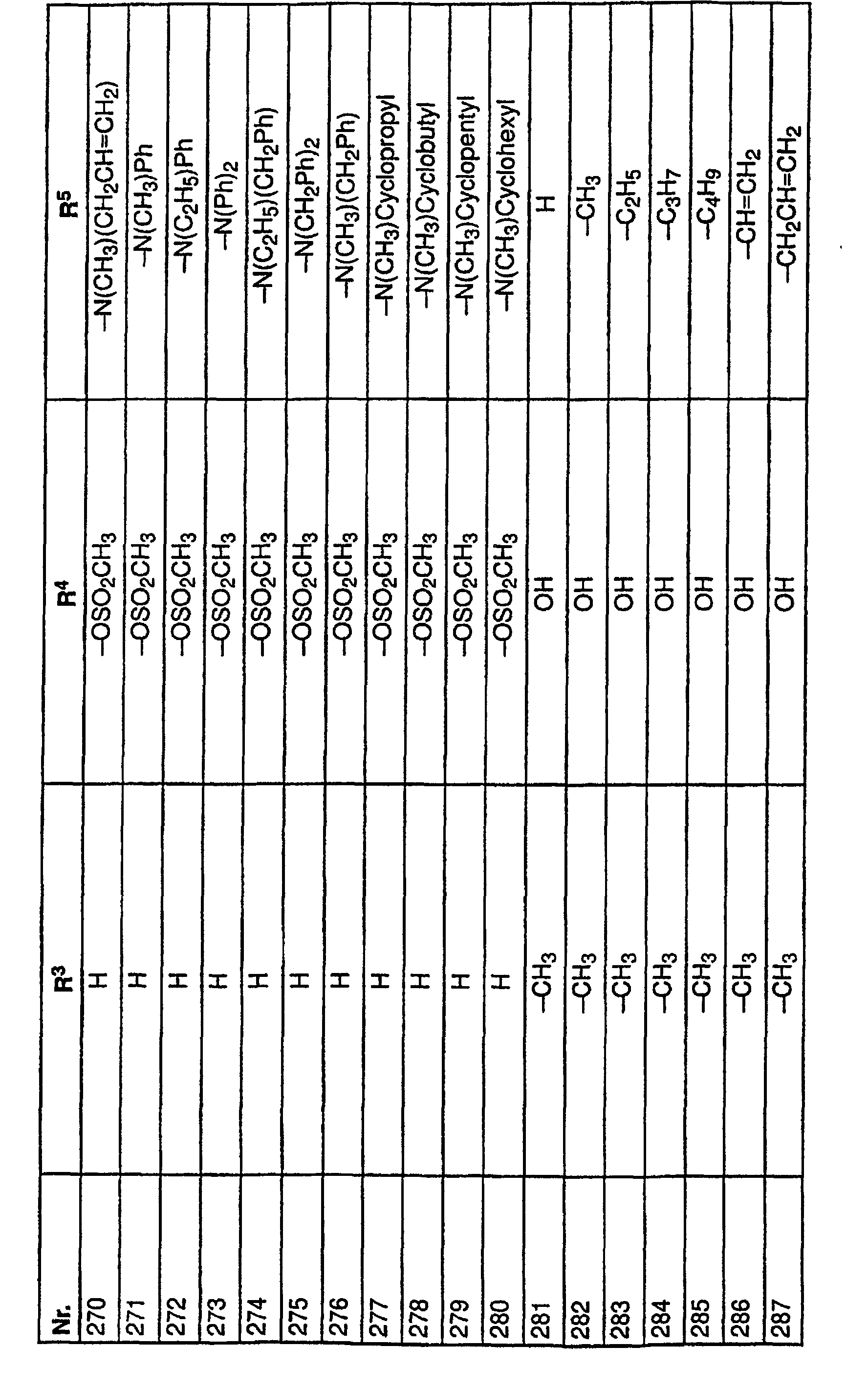

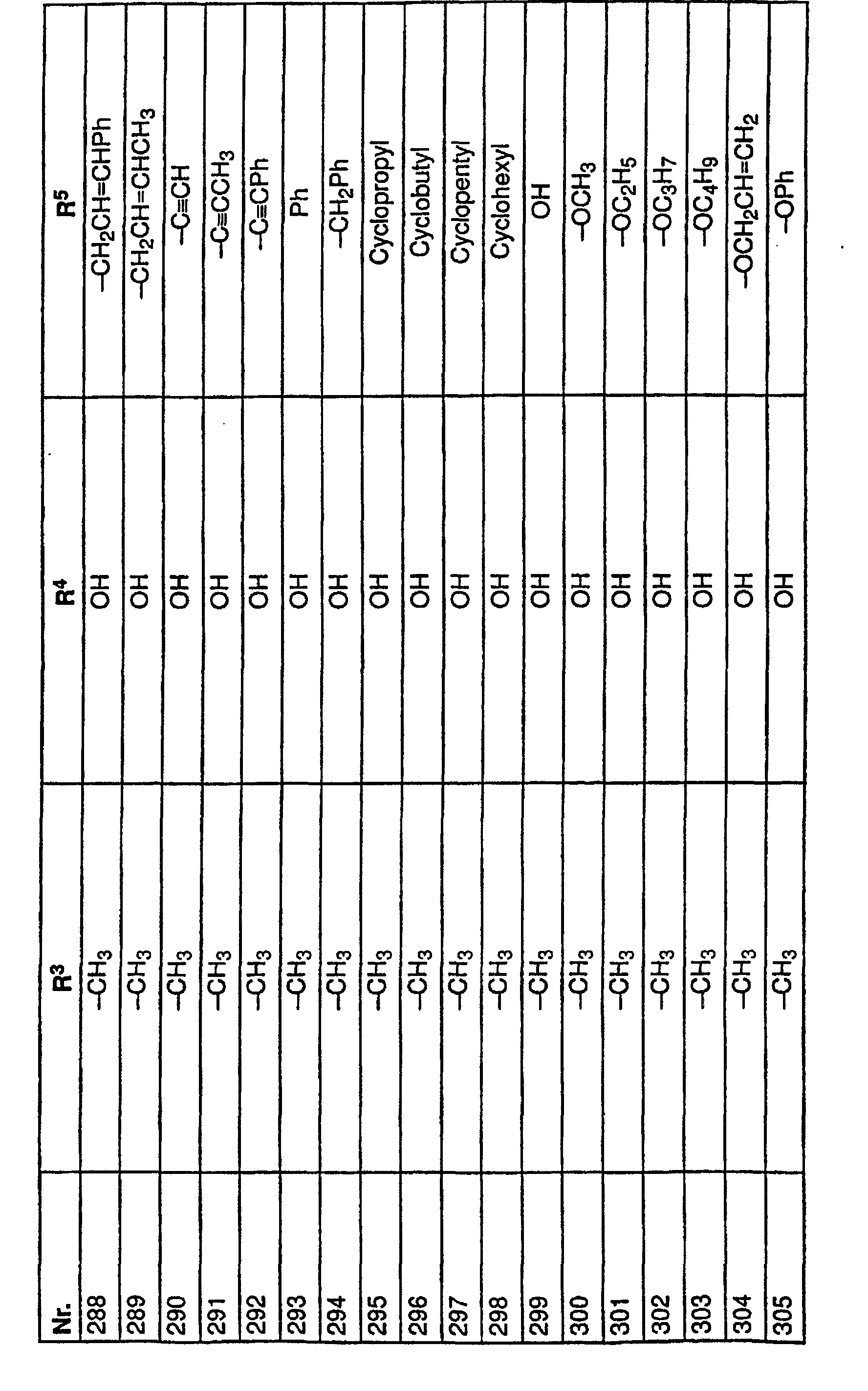

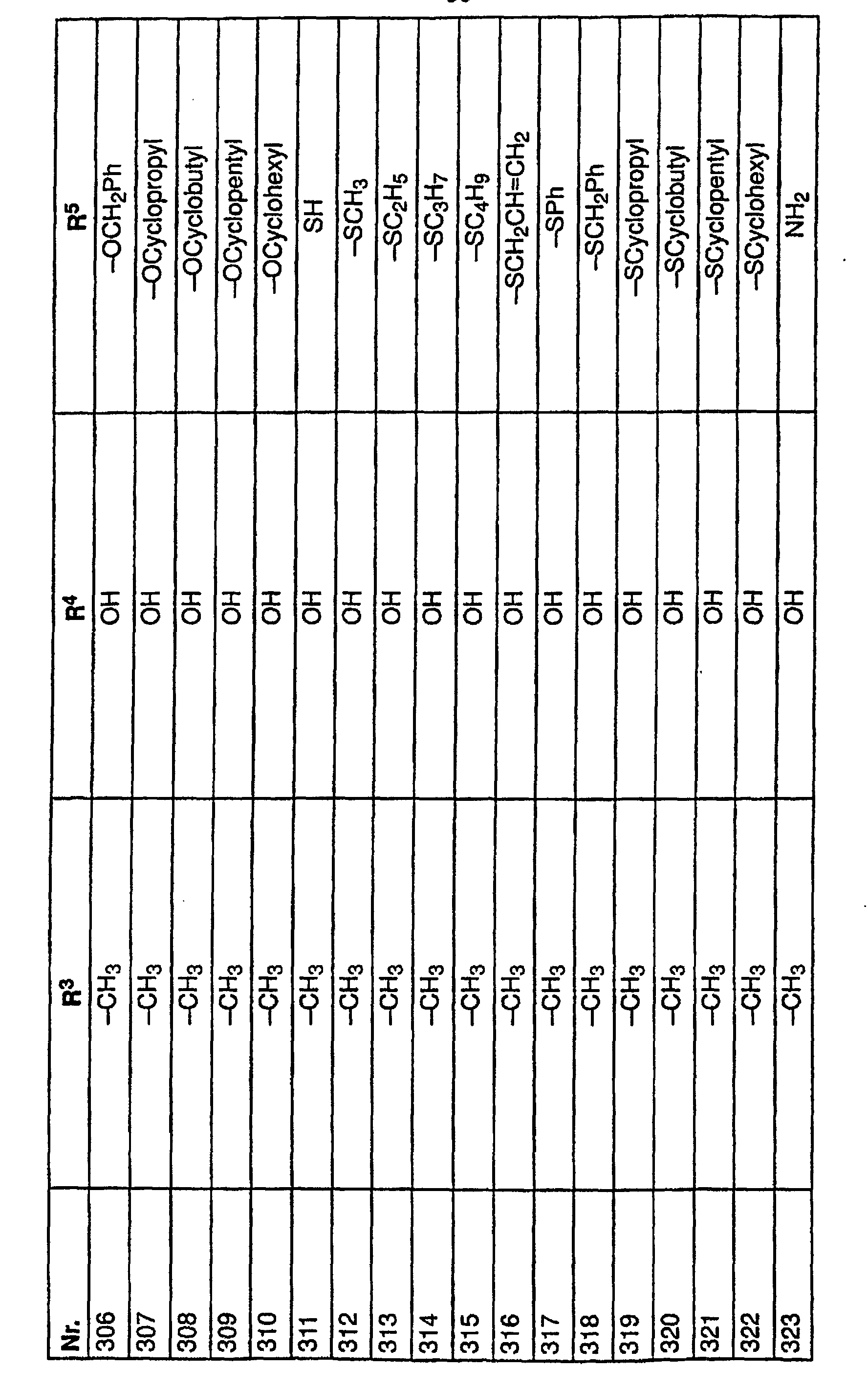

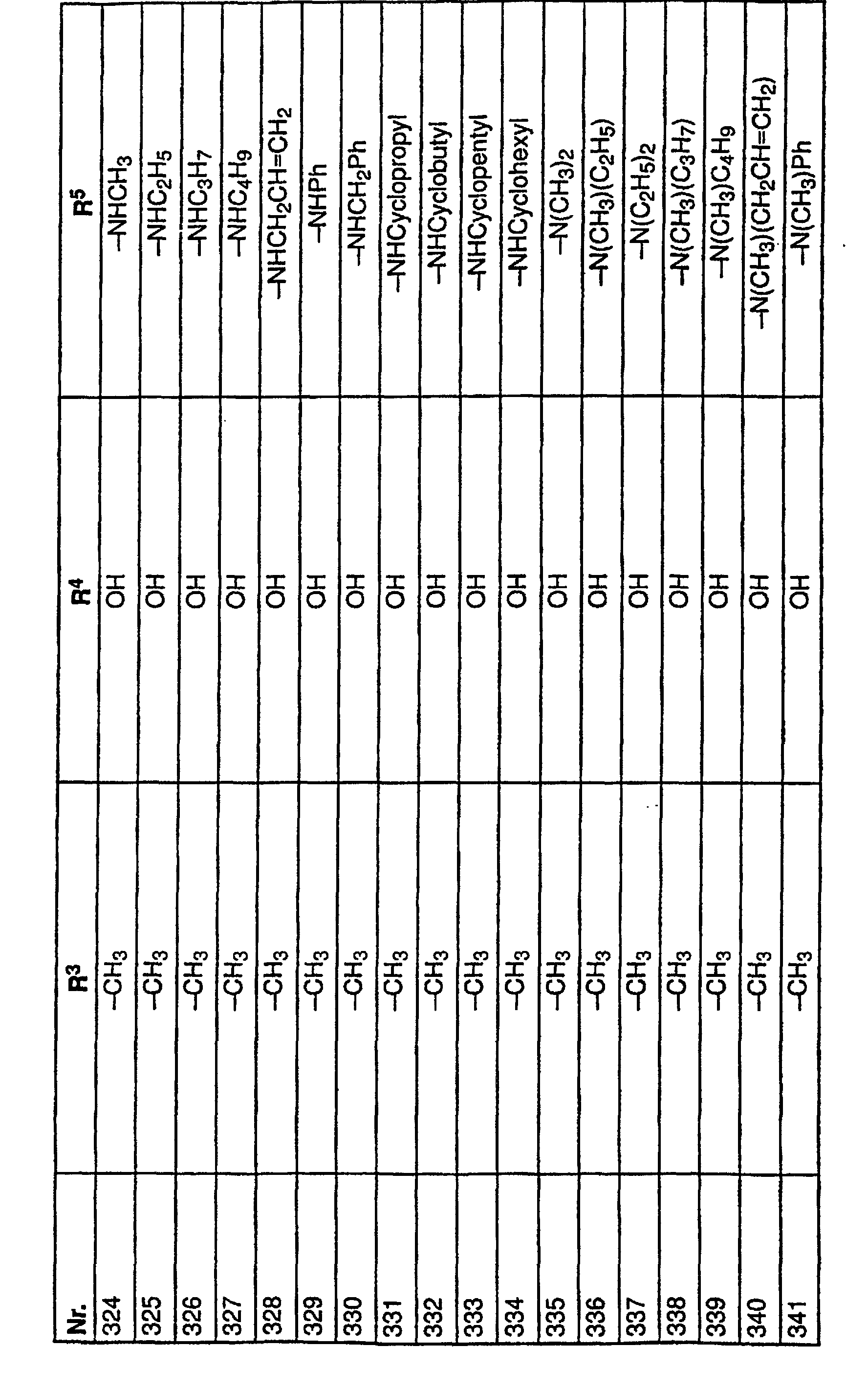

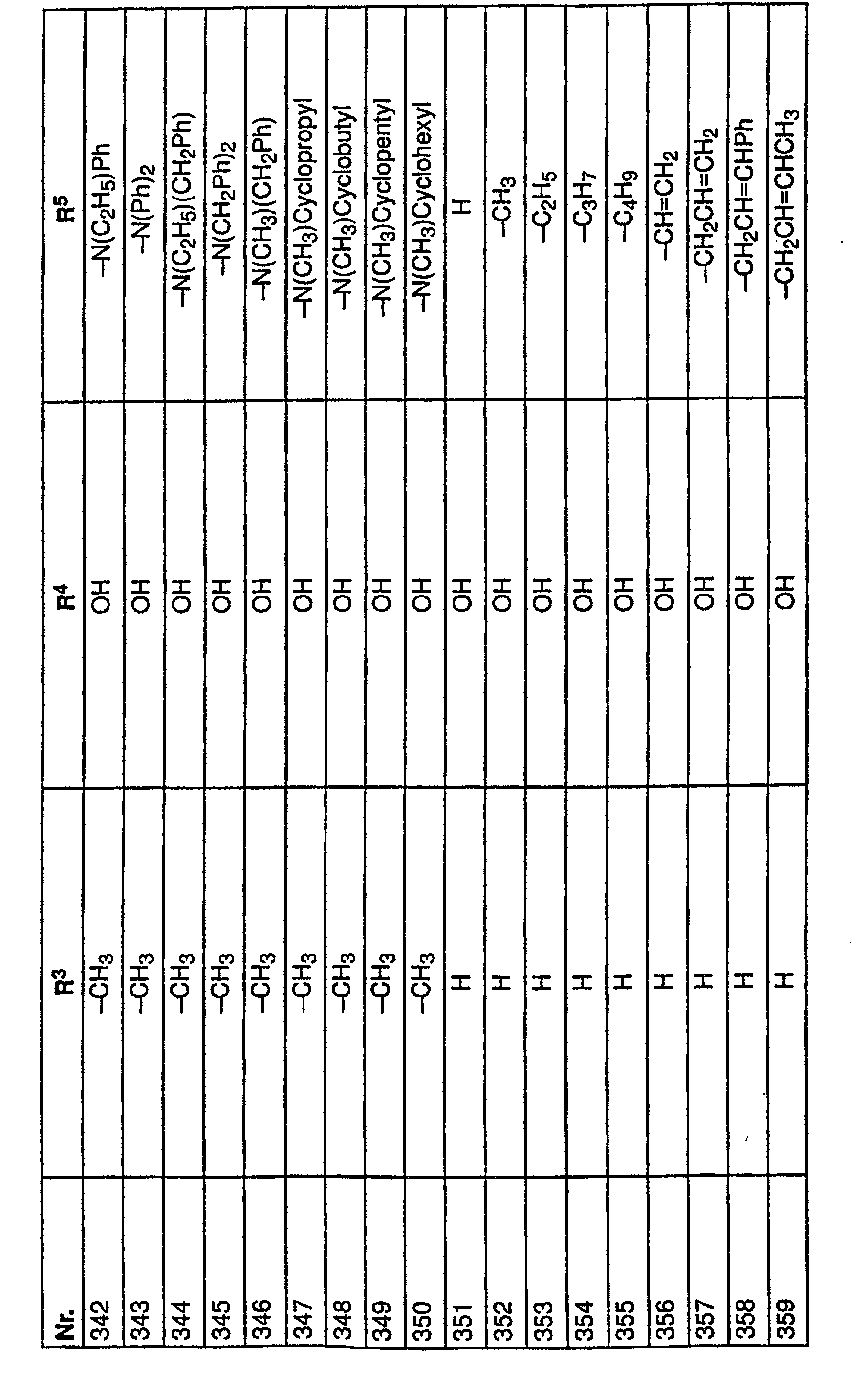

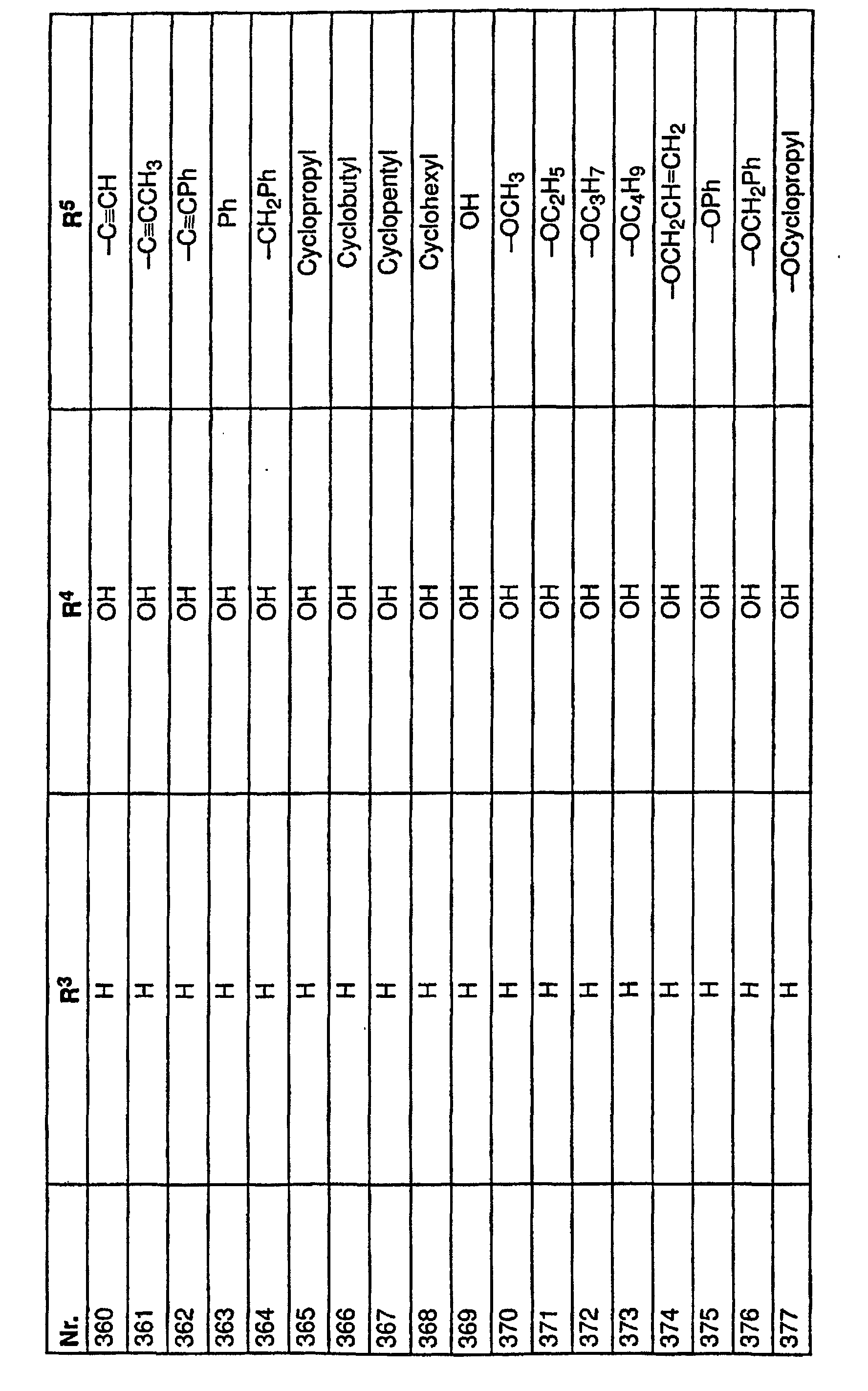

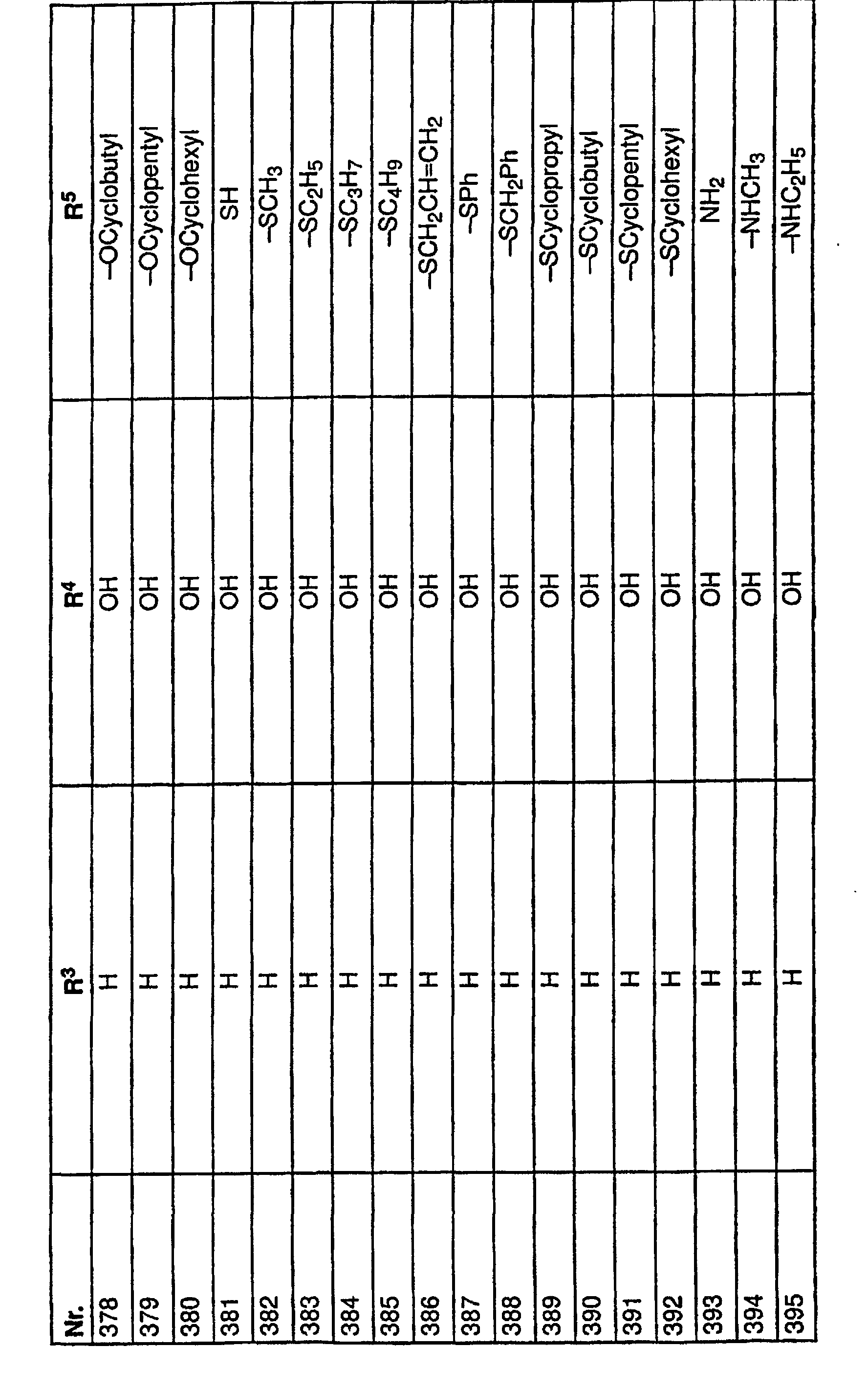

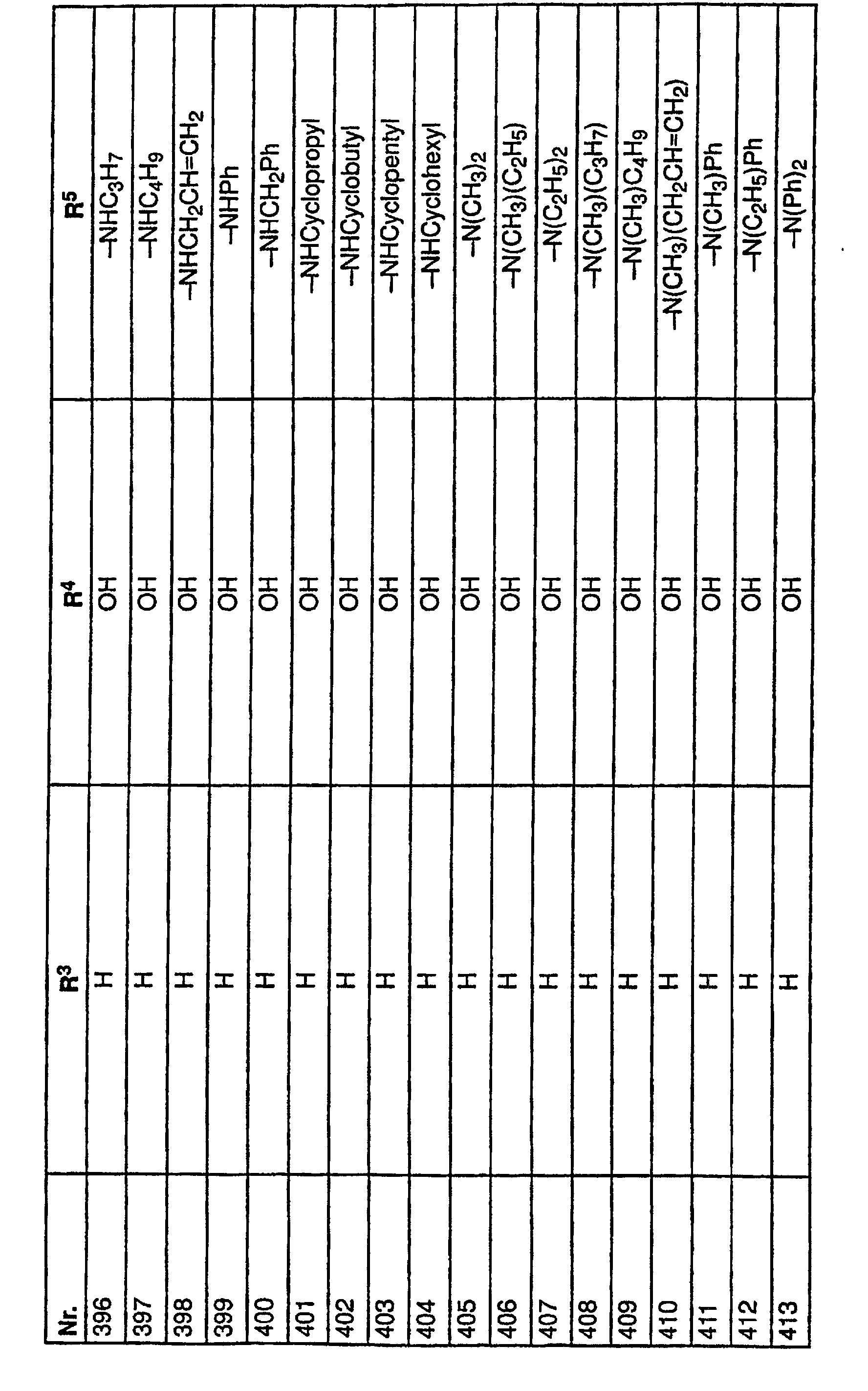

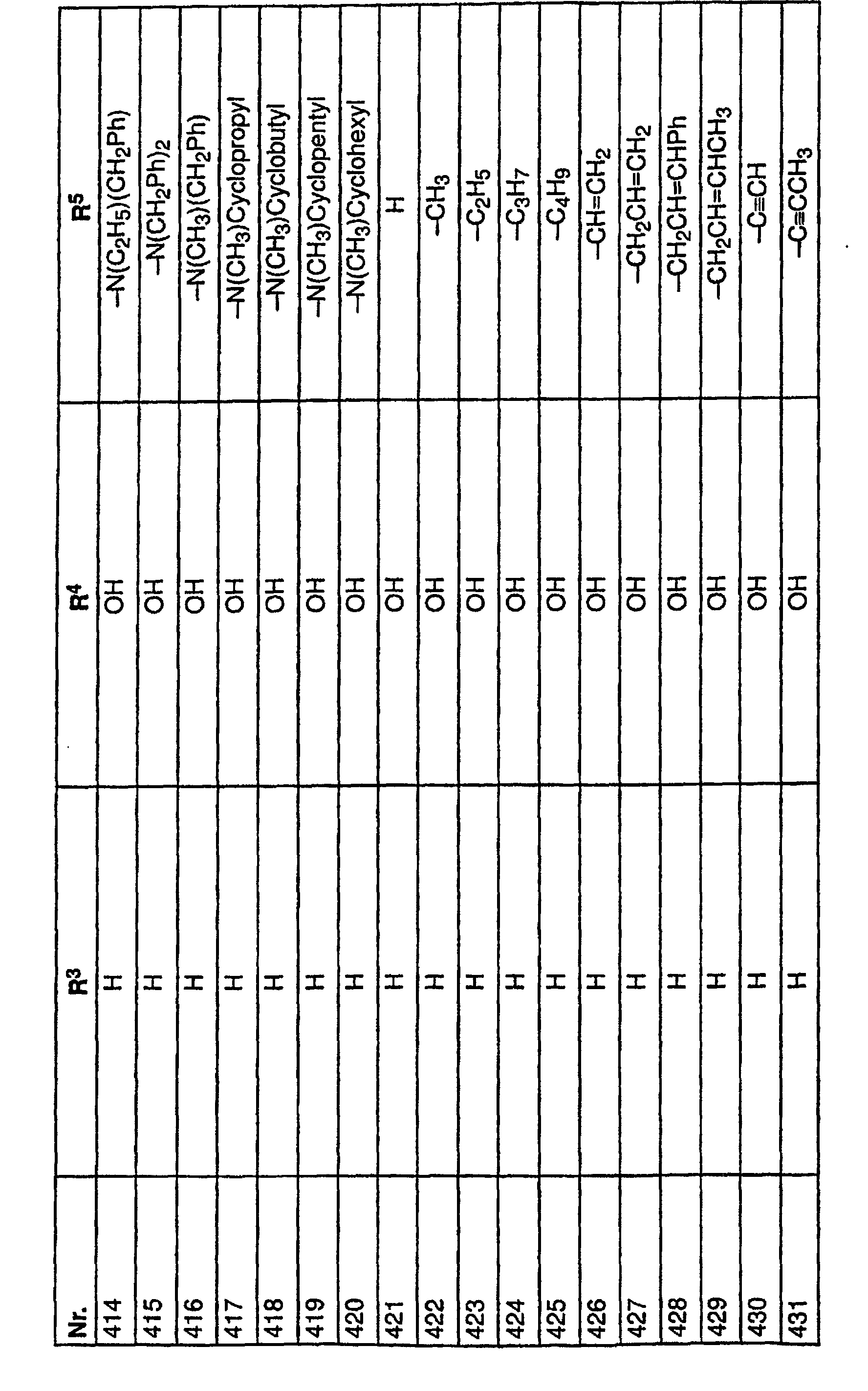

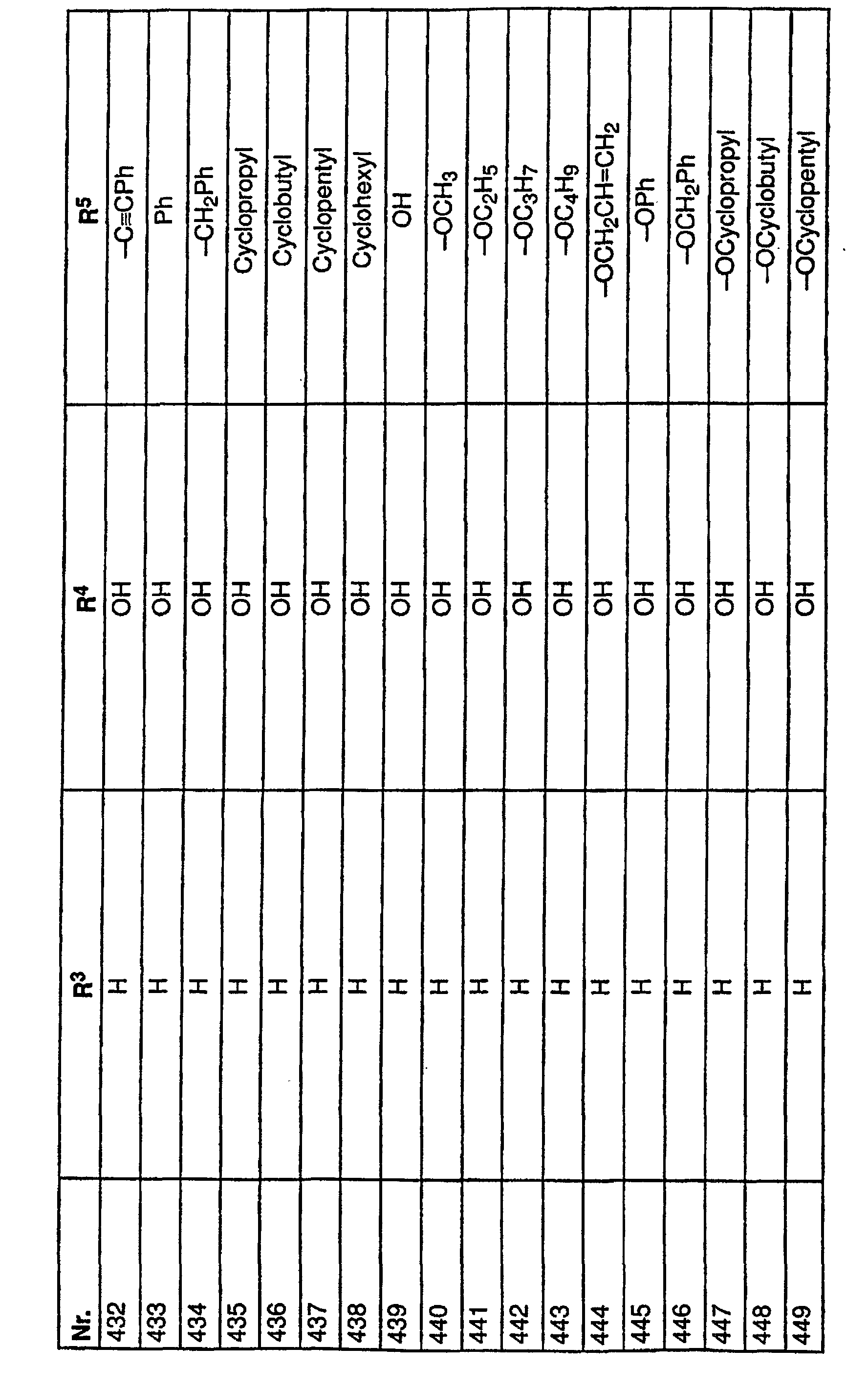

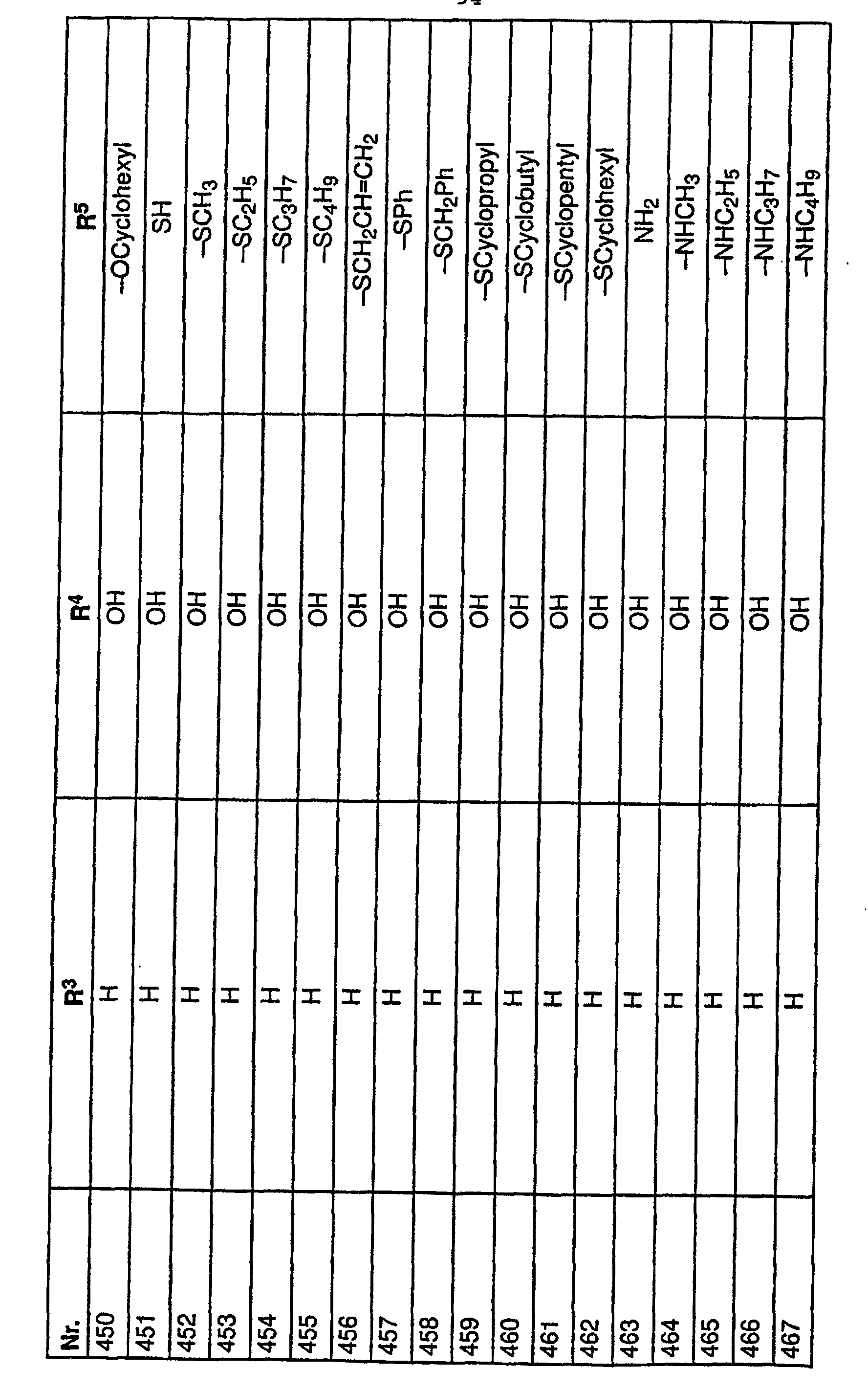

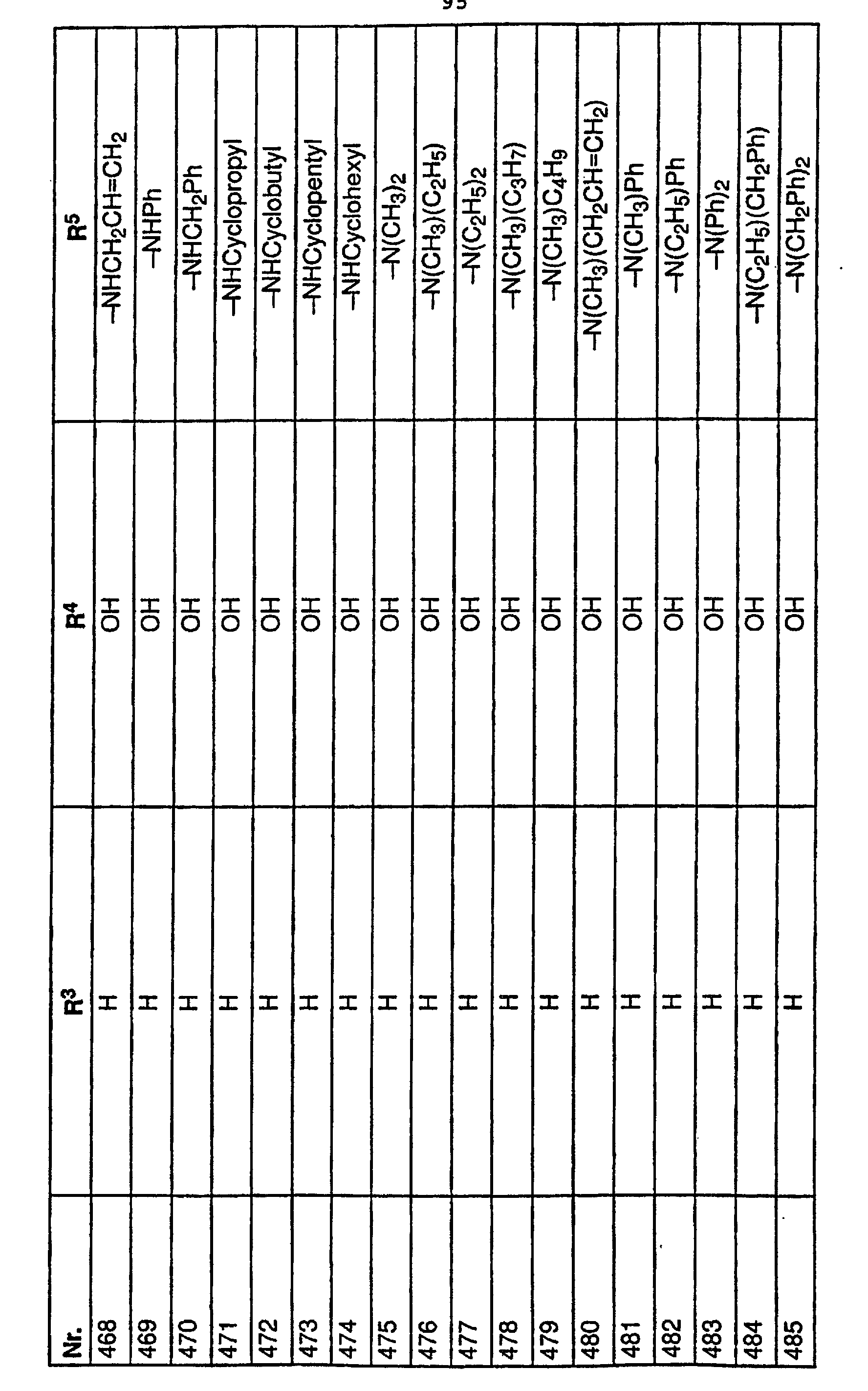

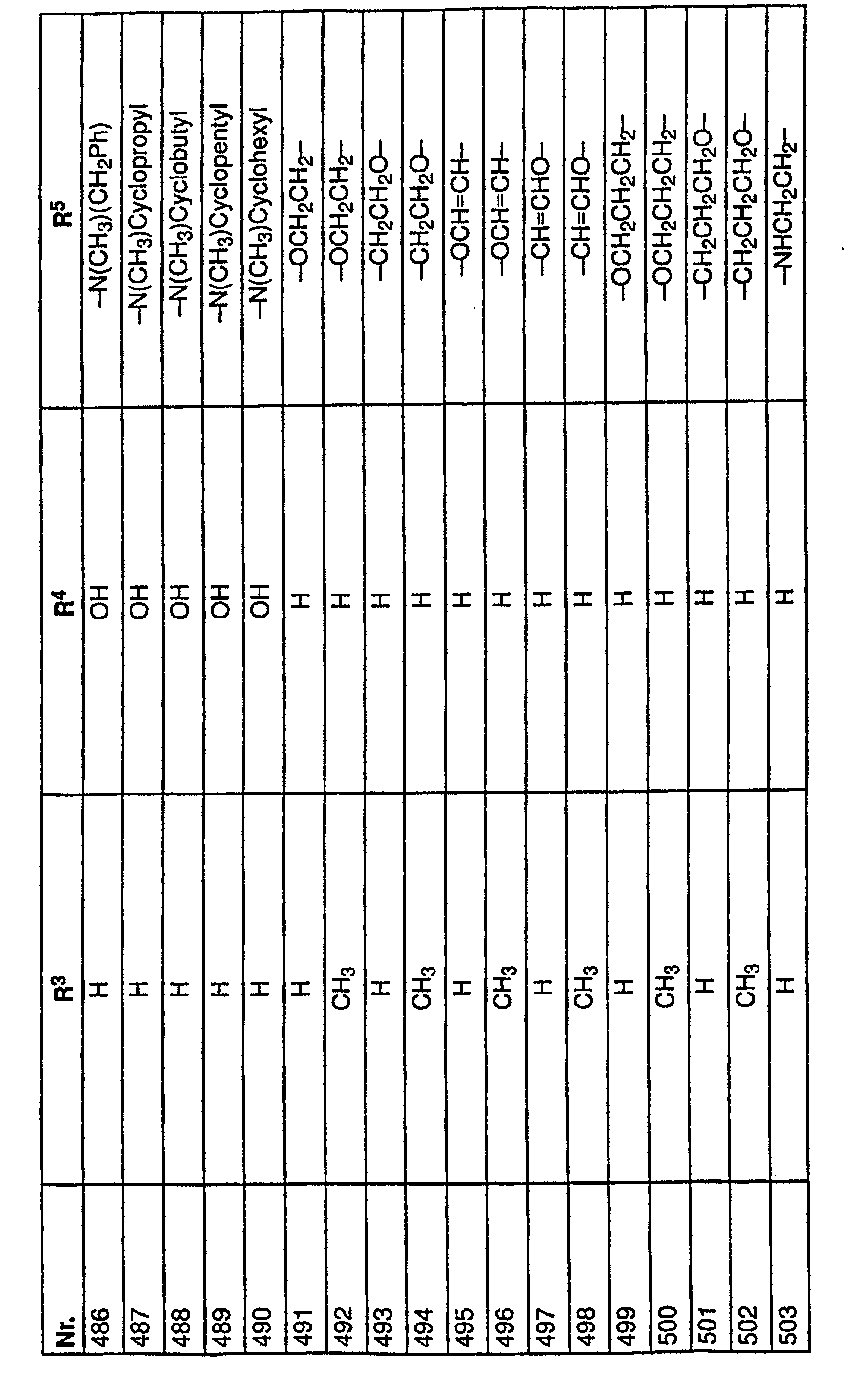

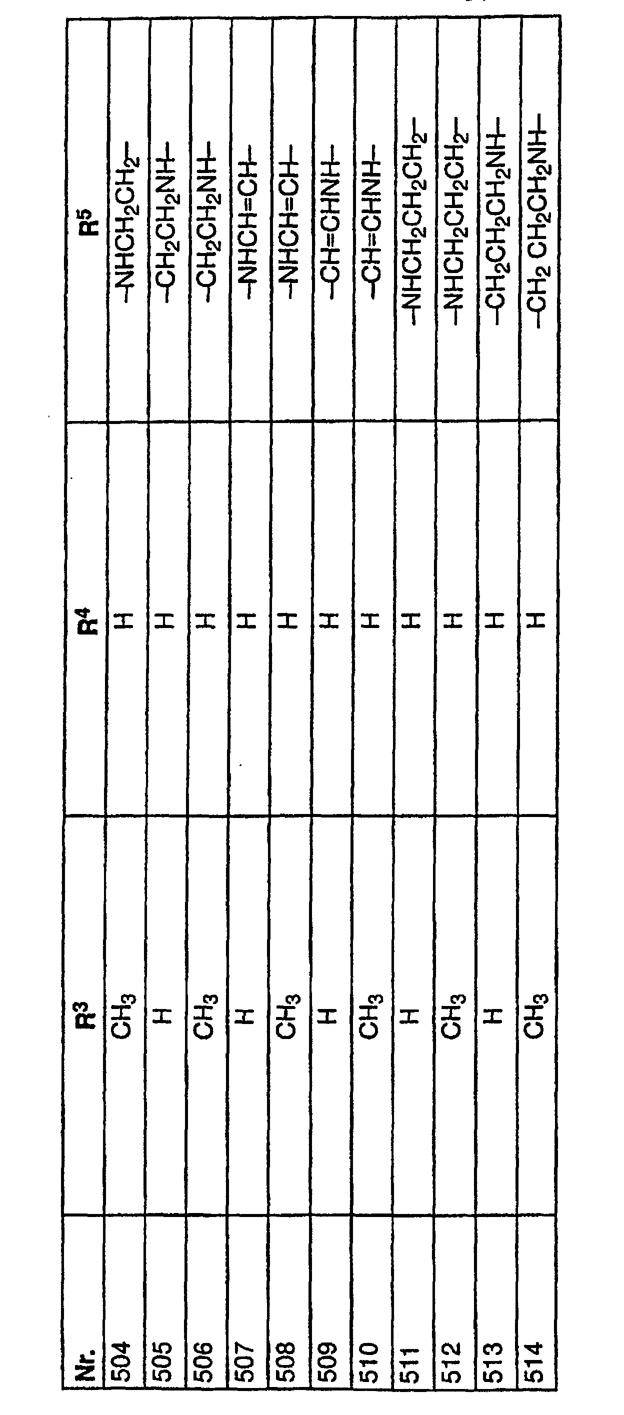

Die folgenden Tabellen 37 bis 72 basieren auf den 2-Benzoyl-cyclohexan-1,3-dionen der Formel Ic

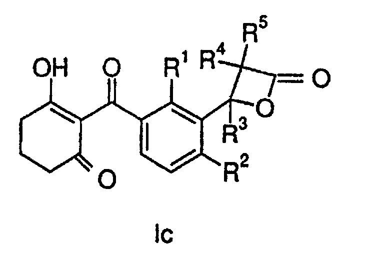

### Tabelle 37: Verbindungen 37.1-37.514

Verbindungen der allgemeinen Formel Ic, in der R¹ Chlor, R² Methylsulfonyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle B entsprechen.

### Tabelle 38: Verbindungen 38.1-38.514

Verbindungen der allgemeinen Formel Ic, in der R¹ und R² Chlor, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle B entsprechen.

### Tabelle 39: Verbindungen 39.1-39.514

Verbindungen der allgemeinen Formel Ic, in der R¹ Chlor, R² Trifluormethyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle B entsprechen.

### Tabelle 40: Verbindungen 40.1-40.514

Verbindungen der allgemeinen Formel Ic, in der R¹ Methyl, R² Chlor, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle B entsprechen.

### Tabelle 41: Verbindungen 41.1-41.514

Verbindungen der allgemeinen Formel Ic, in der R¹ Methyl, R² Methylsulfonyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle B entsprechen.

### Tabelle 42: Verbindungen 42.1-42.514

Verbindungen der allgemeinen Formel Ic, in der R¹ Methyl, R² Trifluormethyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle B entsprechen.

### Tabelle 43: Verbindungen 43.1-43.514

Verbindungen der allgemeinen Formel Ic, in der R¹ Chlor, R² Methylsulfonyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle B entsprechen.

### Tabelle 44: Verbindungen 44.1-44.514

Verbindungen der allgemeinen Formel Ic, in der R¹ und R² Chlor, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle B entsprechen.

### Tabelle 45: Verbindungen 45.1-45.514

Verbindungen der allgemeinen Formel Ic, in der R¹ Chlor, R² Trifluormethyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle B entsprechen.

### Tabelle 46: Verbindungen 46.1-46.514

Verbindungen der allgemeinen Formel Ic, in der R¹ Methyl, R² Chlor, R¹¹, R¹², R¹⁵ und R¹⁶ Wasserstoff, R¹³ und R¹⁴ Methyl bedeu-5 tet und die Substituenten R³ bis R⁷ für jede einzelne Verbindung jeweils einer Zeile der Tabelle B entsprechen.

### Tabelle 47: Verbindungen 47.1-47.514

Verbindungen der allgemeinen Formel Ic, in der R¹ Methyl, R² Methylsulfonyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle B entsprechen.

### Tabelle 48: Verbindungen 48.1-48.514

Verbindungen der allgemeinen Formel Ic, in der R¹ Methyl, R² Trifluormethyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle B entsprechen.

### Tabelle 49: Verbindungen 49.1-49.514

Verbindungen der allgemeinen Formel Ic, in der R¹ Chlor, R² Methylsulfonyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle B entsprechen.

### Tabelle 50: Verbindungen 50.1-50.514

Verbindungen der allgemeinen Formel Ic, in der R¹ und R² Chlor, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle B entsprechen.

### Tabelle 51: Verbindungen 51.1-51.514

Verbindungen der allgemeinen Formel Ic, in der R¹ Chlor, R² Trifluormethyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle B entsprechen.

### Tabelle 52: Verbindungen 52.1-52.514

Verbindungen der allgemeinen Formel Ic, in der R¹ Methyl, R² Chlor, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle B entsprechen.

### Tabelle 53: Verbindungen 53.1-53.514

Verbindungen der allgemeinen Formel Ic, in der R¹ Methyl, R² Methylsulfonyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle B entsprechen.

### Tabelle 54: Verbindungen 54.1-54.514

Verbindungen der allgemeinen Formel Ic, in der R¹ Methyl, R² Trifluormethyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle B entsprechen.

### Tabelle 55: Verbindungen 55.1-55.514

Verbindungen der allgemeinen Formel Ic, in der R¹ Chlor, R² Methylsulfonyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle B entsprechen.

### Tabelle 56: Verbindungen 56.1-56.514

Verbindungen der allgemeinen Formel Ic, in der R¹ und R² Chlor, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle B entsprechen.

### Tabelle 57: Verbindungen 57.1-57.514

Verbindungen der allgemeinen Formel Ic, in der R¹ Chlor, R² Trifluormethyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle B entsprechen.

### Tabelle 58: Verbindungen 58.1-58.514

Verbindungen der allgemeinen Formel Ic, in der R¹ Methyl, R² Chlor, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle B entsprechen.

### Tabelle 59: Verbindungen 59.1-59.514

Verbindungen der allgemeinen Formel Ic, in der R¹ Methyl, R² Methylsulfonyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle B entsprechen.

### Tabelle 60: Verbindungen 60.1-60.514

Verbindungen der allgemeinen Formel Ic, in der R¹ Methyl, R² Trifluormethyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle B entsprechen.

### Tabelle 61: Verbindungen 61.1-61.514

Verbindungen der allgemeinen Formel Ic, in der R¹ Methylsulfonyl, R¹¹, R¹², R¹³, R¹⁵ und R¹⁶ Wasserstoff, R¹⁴ Methyl bedeutet, die Substituenten R³ bis R⁷ für jede einzelne Verbindung jeweils einer Zeile der Tabelle B entsprechen.

### Tabelle 62: Verbindungen 62.1-62.514

Verbindungen der allgemeinen Formel Ic, in der R¹ und R² Chlor, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle B entsprechen.

### Tabelle 63: Verbindungen 63.1-63.514

Verbindungen der allgemeinen Formel Ic, in der R¹ Chlor, R² Trifluormethyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle B entsprechen.

### Tabelle 64: Verbindungen 64.1-64.514

Verbindungen der allgemeinen Formel Ic, in der R¹ Methyl, R² Chlor, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle B entsprechen.

### Tabelle 65: Verbindungen 65.1-65.514

Verbindungen der allgemeinen Formel Ic, in der R¹ Methyl, R² Methylsulfonyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle B entsprechen.

### Tabelle 66: Verbindungen 66.1-66.514

Verbindungen der allgemeinen Formel Ic, in der R¹ Methyl, R² Trifluormethyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle B entsprechen.

### Tabelle 67: Verbindungen 67.1-67.514

Verbindungen der allgemeinen Formel Ic, in der R¹ Chlor, R² Methylsulfonyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle B entsprechen.

### Tabelle 68: Verbindungen 68.1-68.514

Verbindungen der allgemeinen Formel Ic, in der R¹ und R² Chlor, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle B entsprechen.

### Tabelle 69: Verbindungen 69.1-69.514

Verbindungen der allgemeinen Formel Ic, in der R¹ Chlor, R² Trifluormethyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle B entsprechen.

### Tabelle 70: Verbindungen 70.1-70.514

Verbindungen der allgemeinen Formel Ic, in der R¹ Methyl, R² Chlor, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle B entsprechen.

### Tabelle 71: Verbindungen 71.1-71.514

Verbindungen der allgemeinen Formel Ic, in der R¹ Methyl, R² Methylsulfonyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle B entsprechen.

### Tabelle 72: Verbindungen 72.1-72.514

Verbindungen der allgemeinen Formel Ic, in der R¹ Methyl, R² Trifluormethyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle B entsprechen.

**Tabelle C**

| Nr. | R³ | R⁸ | R⁹ |
|---|---|---|---|
| 1 | H | H | H |
| 2 | H | CH₃ | H |
| 3 | H | C₂H₅ | H |
| 4 | H | C₃H₇ | H |
| 5 | H | C₄H₉ | H |
| 6 | H | CH (CH₃)₂ | H |
| 7 | H | cy-C₃H₅ | H |
| 8 | H | cy-C₄H₇ | H |
| 9 | H | cy-C₅H₉ | H |
| 10 | H | cy-C₆-H₁₁ | H |
| 11 | H | C₆H₅ | H |
| 12 | H | CH₂-C₆H₅ | H |
| 13 | H | 2-Furyl | H |
| 14 | H | 3-Furyl | H |
| 15 | H | 2-Thienyl | H |
| 16 | H | 3-Thienyl | H |
| 17 | H | 2-Dioxanyl | H |
| 18 | H | CHO | H |
| 19 | H | COCH₃ | H |
| 20 | H | COOCH₃ | H |
| 21 | H | COOC₂H₅ | H |
| 22 | H | OCH₃ | H |
| 23 | H | CN | H |
| 24 | H | SCH₃ | H |
| 25 | H | COCF₃ | H |
| 26 | H | COC₆H₅ | H |
| 27 | H | CH=NOCH₃ | H |
| 28 | H | CH=NOC₂H₅ | H |
| 29 | H | C(CH₃)=NOCH₃ | H |
| 30 | H | CH₃ | CH₃ |
| 31 | H | C₂H₅ | CH3 |
| 32 | H | C₃H₇ | CH₃ |
| 33 | H | C₄H₉ | CH₃ |
| 34 | H | CHO | CH₃ |
| 35 | H | COCH₃ | CH₃ |
| 36 | H | COOCH₃ | CH₃ |
| 37 | H | OCH₃ | CH₃ |
| 38 | H | C₆H₅ | CH₃ |
| 39 | H | CH₂-CH_{O} | H |
| 40 | H | COOCH₂C₆H₅ | H |
| 41 | Cl | CH₃ | H |
| 42 | CH₃ | CH₃ | H |
| 43 | C₂H₅ | CH₃ | H |
| 44 | CF₃ | CH₃ | H |
| 45 | OCH₃ | CH₃ | H |
| 46 | OC₂H₅ | CH₃ | H |
| 47 | CH₂-C≡CH | CH₃ | H |
| 48 | CH₂-CH=CH₂ | CH₃ | H |
| 49 | Cl | CH₃ | H |
| 50 | CH₃ | CH₃ | H |
| 51 | CF₃ | CH₃ | H |
| 52 | OCH₃ | CH₃ | H |
| 53 | OC₂H₅ | CH₃ | H |
| 54 | CH₂-CH=CH₂ | CH₃ | H |
| 55 | CH₂-C≡CH | CH₃ | H |
| 56 | H | CH₃ | Ph |
| 57 | H | C₂H₅ | Ph |
| 58 | H | C₃H₇ | Ph |
| 59 | H | C₄H₉ | Ph |
| 60 | H | CHO | Ph |
| 61 | H | COCH₃ | Ph |
| 62 | H | COOCH₃ | Ph |
| 63 | H | OCH₃ | Ph |
| 64 | H | C₆H₅ | Ph |
| 65 | H | CH=NOCH₃ | Ph |
| 66 | H | C(CH₃)=NOCH₃ | Ph |
| 67 | CH₃ | 2-Cl-C₆H₄ | H |
| 68 | CH₃ | 3-Br-C₆H₄ | H |
| 69 | CH₃ | 4-F-C₆H₄ | H |
| 70 | CH₃ | 2,4-Cl₂-C₆H₃ | H |
| 71 | CH₃ | 2-NO₂-C₆H₄ | H |
| 72 | CH₃ | 3-CN-C₆H₄ | H |
| 73 | CH₃ | 4-Me-C₆H₄ | H |
| 74 | CH₃ | 2-OMe-C₆H₄ | H |
| 75 | CH₃ | 3-CF₃-C₆H₄ | H |
| 76 | CH₃ | 4-OCF₃-C₆H₄ | H |
| 77 | CH₃ | 2-Me-C₆H₄ | H |
| 78 | CH₃ | 3-Me-C₆H₄ | H |
| 79 | CH₃ | 2-SMe-C₆H₄ | H |
| 80 | CH₃ | 3-COOMe-C₆H₄ | H |
| 81 | CH₃ | 4-CF₃-C₆H₄ | H |
| 82 | CH₃ | 2-CF₃-C₆H₄ | H |
| 83 | CH₃ | 3-OMe-C₆H₄ | H |
| 84 | CH₃ | 4-OMe-C₆H₄ | H |
| 85 | H | 2-Furyl | CH₃ |
| 86 | H | 3-Furyl | CH₃ |
| 87 | H | 2-Thienyl | CH₃ |
| 88 | H | 3-Thienyl | CH₃ |
| 89 | H | 2-Pyridyl | CH₃ |
| 90 | H | 3-Pyridyl | CH₃ |
| 91 | H | 4-Pyridyl | CH₃ |
| 92 | H | 2-Thiazolyl | CH₃ |
| 93 | H | 4-Thiazolyl | CH₃ |
| 94 | H | 5-Thiazolyl | CH₃ |
| 95 | H | 2-Pyrrolyl | CH₃ |
| 96 | H | 3-Pyrrolyl | CH₃ |
| 97 | H | 4-Pyrrolyl | CH₃ |
| 98 | H | 3-Isoxazolyl | CH₃ |
| 99 | H | 4-Isoxazolyl | CH₃ |
| 100 | H | 5-Isoxazolyl | CH₃ |
| 101 | H | 2-Oxazolyl | CH₃ |
| 102 | H | 4-Oxazolyl | CH₃ |

Die folgenden Tabellen 73-108 basieren auf den 2-Benzoyl-cyclohexan-1,3-dione der Formel Id

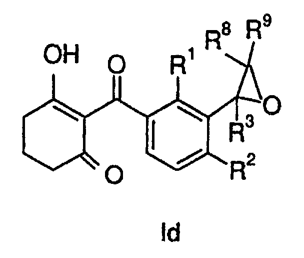

### Tabelle 73: Verbindungen 73.1-73.102

Verbindungen der allgemeinen Formel Id, in der R¹ Chlor, R² Methylsulfonyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle C entsprechen.

### Tabelle 74: Verbindungen 74.1-74.102

Verbindungen der allgemeinen Formel Id, in der R¹ und R² Chlor, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle C entsprechen.

### Tabelle 75: Verbindungen 75.1-75.102

Verbindungen der allgemeinen Formel Id, in der R¹ Chlor, R² Trifluormethyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle C entsprechen.

### Tabelle 76: Verbindungen 76.1-76.102

Verbindungen der allgemeinen Formel Id, in der R¹ Methyl, R² Chlor, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle C entsprechen.

### Tabelle 77: Verbindungen 77.1-77.102

Verbindungen der allgemeinen Formel Id, in der R¹ Methyl, R² Methylsulfonyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle C entsprechen.

### Tabelle 78: Verbindungen 78.1-78.102

Verbindungen der allgemeinen Formel Id, in der R¹ Methyl, R² Trifluormethyl, R¹¹, R¹², R¹³, R¹⁴, R¹⁵ und R¹⁶ Wasserstoff bedeutet und die Substituenten R³ bis R⁷ für jede einzelne Verbindung jeweils einer Zeile der Tabelle C entsprechen.

### Tabelle 79: Verbindungen 79.1-79.102

Verbindungen der allgemeinen Formel Id, in der R¹ Chlor, R² Methylsulfonyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle C entsprechen.

### Tabelle 80: Verbindungen 80.1-80.102

Verbindungen der allgemeinen Formel Id, in der R¹ und R² Chlor, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle C entsprechen.

### Tabelle 81: Verbindungen 81.1-81.102

Verbindungen der allgemeinen Formel Id, in der R¹ Chlor, R² Trifluormethyl, R¹¹, R¹², R¹⁵ und R¹⁶ Wasserstoff, R¹³ und R1⁴ Methyl bedeutet und die Substituenten R³ bis R⁷ für jede einzelne Verbindung jeweils einer Zeile der Tabelle C entsprechen.

### Tabelle 82: Verbindungen 82.1-82.102

Verbindungen der allgemeinen Formel Id, in der R¹ Methyl, R² Chlor, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle C entsprechen.

### Tabelle 83: Verbindungen 83.1-83.102

Verbindungen der allgemeinen Formel Id, in der R¹ Methyl, R² Methylsulfonyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle C entsprechen.

### Tabelle 84: Verbindungen 84.1-84.102

Verbindungen der allgemeinen Formel Id, in der R¹ Methyl, R² Trifluormethyl, R¹¹, R¹², R¹⁵ und R¹⁶ Wasserstoff, R¹³ und R¹⁴ Methyl bedeutet und die Substituenten R³ bis R⁷ für jede einzelne Verbindung jeweils einer Zeile der Tabelle C entsprechen.

### Tabelle 85: Verbindungen 85.1-85.102

Verbindungen der allgemeinen Formel Id, in der R¹ Chlor, R² Methylsulfonyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle C entsprechen.

### Tabelle 86: Verbindungen 86.1-86.102

Verbindungen der allgemeinen Formel Id, in der R¹ und R² Chlor, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle C entsprechen.

### Tabelle 87: Verbindungen 87.1-87.102

Verbindungen der allgemeinen Formel Id, in der R¹ Chlor, R² Trifluormethyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle C entsprechen.

### Tabelle 88: Verbindungen 88.1-88.102

Verbindungen der allgemeinen Formel Id, in der R¹ Methyl, R² Chlor, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle C entsprechen.

### Tabelle 89: Verbindungen 89.1-89.102

Verbindungen der allgemeinen Formel Id, in der R¹ Methyl, R² Methylsulfonyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle C entsprechen.

### Tabelle 90: Verbindungen 90.1-90.102

Verbindungen der allgemeinen Formel Id, in der R¹ Methyl, R² Trifluormethyl, R¹¹, R¹², R¹³ und R¹⁴ Wasserstoff, R¹⁵ und R¹⁶ Methyl bedeutet und die Substituenten R³ bis R⁷ für jede einzelne Verbindung jeweils einer Zeile der Tabelle C entsprechen.

### Tabelle 91: Verbindungen 91.1-91.102

Verbindungen der allgemeinen Formel Id, in der R¹ Chlor, R² Methylsulfonyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle C entsprechen.

### Tabelle 92: Verbindungen 92.1-92.102

Verbindungen der allgemeinen Formel Id, in der R¹ und R² Chlor, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle C entsprechen.

### Tabelle 93: Verbindungen 93.1-93.102

Verbindungen der allgemeinen Formel Id, in der R¹ Chlor, R² Trifluormethyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle C entsprechen.

### Tabelle 94: Verbindungen 94.1-94.102

Verbindungen der allgemeinen Formel Id, in der R¹ Methyl, R² Chlor, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle C entsprechen.

### Tabelle 95: Verbindungen 95.1-95.102

Verbindungen der allgemeinen Formel Id, in der R¹ Methyl, R² Methylsulfonyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle C entsprechen.

### Tabelle 96: Verbindungen 96.1-96.102

Verbindungen der allgemeinen Formel Id, in der R¹ Methyl, R² Trifluormethyl, R¹¹, R¹², R¹⁵ und R¹⁶ Methyl bedeutet, die CR¹³R¹⁴-Einheit eine Gruppe C=O bildet und die Substituenten R³ bis R⁷ für jede einzelne Verbindung jeweils einer Zeile der Tabelle C entsprechen.

### Tabelle 97: Verbindungen 97.1-97.102

Verbindungen der allgemeinen Formel Id, in der R¹ Methylsulfonyl, R¹¹, R¹², R¹³, R¹⁵ und R¹⁶ Wasserstoff, R¹⁴ Methyl bedeutet, die Substituenten R³ bis R⁷ für jede einzelne Verbindung jeweils einer Zeile der Tabelle C entsprechen.

### Tabelle 98: Verbindungen 98.1-98.102

Verbindungen der allgemeinen Formel Id, in der R¹ und R² Chlor, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle C entsprechen.

### Tabelle 99: Verbindungen 99.1-99.102

Verbindungen der allgemeinen Formel Id, in der R¹ Chlor, R² Trifluormethyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle C entsprechen.

### Tabelle 100: Verbindungen 100.1-100.102

Verbindungen der allgemeinen Formel Id, in der R¹ Methyl, R² Chlor, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle C entsprechen.

### Tabelle 101: Verbindungen 101.1-101.102

Verbindungen der allgemeinen Formel Id, in der R¹ Methyl, R² Methylsulfonyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle C entsprechen.

### Tabelle 102: Verbindungen 102.1-102.102

Verbindungen der allgemeinen Formel Id, in der R¹ Methyl, R² Trifluormethyl, R¹¹, R¹², R¹³, R¹⁵ und R¹⁶ Wasserstoff, R¹⁴ Methyl bedeutet und die Substituenten R³ bis R⁷ für jede einzelne Verbindung jeweils einer Zeile der Tabelle C entsprechen.

### Tabelle 103: Verbindungen 103.1-103.102

Verbindungen der allgemeinen Formel Id, in der R¹ Chlor, R² Methylsulfonyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle C entsprechen.

### Tabelle 104: Verbindungen 104.1-104.102

Verbindungen der allgemeinen Formel Id, in der R¹ und R² Chlor, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle C entsprechen.

### Tabelle 105: Verbindungen 105.1-105.102

Verbindungen der allgemeinen Formel Id, in der R¹ Chlor, R² Trifluormethyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle C entsprechen.

### Tabelle 106: Verbindungen 106.1-106.102

Verbindungen der allgemeinen Formel Id, in der R¹ Methyl, R² Chlor, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle C entsprechen.

### Tabelle 107: Verbindungen 107.1-107.102

Verbindungen der allgemeinen Formel Id, in der R¹ Methyl, R² Methylsulfonyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle C entsprechen.

### Tabelle 108: Verbindungen 108.1-108.102

Verbindungen der allgemeinen Formel Id, in der R¹ Methyl, R² Trifluormethyl, R¹¹, R¹², R¹⁴ und R¹⁵ Wasserstoff bedeutet, R¹³ und R¹⁶ zusammen eine Methylengruppe bilden und die Substituenten R³ bis R⁷ für jede einzelne Verbindung jeweils einer Zeile der Tabelle C entsprechen.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierten 2-Benzoyl-cyclohexan-1,3-dione als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 90 % bis 100 % (nach NMR-Sektrum) eingesetzt. Die erfindungsgemäßen Verbindungen I können beispielsweise wie folgt formuliert werden:
- I: 20 Gewichtsteile der Verbindung Nr. 110.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
- II: 20 Gewichtsteile der Verbindung Nr. 110.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- III: 20 Gewichtsteile des Wirkstoffs Nr. 110.1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- IV: 20 Gewichtsteile des Wirkstoffs Nr. 110.1 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
- V: 3 Gewichtsteile des Wirkstoffs Nr. 110.1 werden mit 97 Gewichtsteilen feinteiligem Kaolin vermischt. Man erhält auf diese Weise ein Stäubemittel, das 3 Gew.-% des Wirkstoffs enthält.
- VI: 20 Gewichtsteile des Wirkstoffs Nr. 110.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung 110.1 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII1: Gewichtsteil der Verbindung 110.1 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die substituierten 2-Benzoyl-cyclohexan-1,3-dione I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden. Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.) Nachfolgend sind die Synthesen einiger Ausgangsstoffe aufgeführt:

2,4-Dichlor-3-(3'-hydroxy-3'-methyl-2'-oxetanyl)-benzoesäure (Verbindung 5.03)
- Stufe a:: 2,4-Dichlor-3-(3'-trimethylsilyloxy-3'-methyl-2'-oxetanyl)-benzoesäuremethylester (Verbindung 5.01)
Eine Lösung aus 10 g (0,043 M) 2,4-Dichlor-3-formyl-benzoesäuremethylester und 8,4 g (0,065 M) 2-Trimethylsilyloxy-propen in 1,0 1 n-Hexan wurde 24 h bei Raumtemperatur mit einem UV-Strahler (Heraeus, TQ 150W) bestrahlt. Anschließend wurde das Lösungsmittel im Vakuum abdestilliert und der Rückstand an 100 g Kieselgel (0,04 - 0,06 mm) mit Gemischen aus Cyclohexan und Essigsäureethylester 100 : 1 bis 5 : 1 (v/v) gereinigt. Man erhält 6,8 g 2,4-Dichlor-3-(3'-trimethylsilyloxy-3'-methyl-2'-oxetanyl)-benzoesäuremethylester.
1H-NMR (CDCl₃) δ [ppm]: 1,3 (t, 3H), 3,9 (dd, 3H), 4,6 (m, 2H), 6,4 (d, 1H), 7,0 (s, 1H), 7,3 (m, 2H)
- Stufe b:: 2,4-Dichlor-3-(3'-hydroxy-3'-methyl-2'-oxetanyl)-benzoesäuremethylester (Verbindung 5.02)
Eine Lösung aus 14 g (0,039 M) 2,4-Dichlor-3-(3'-trimethylsilyloxy-3'-methyl-2'-oxetanyl)-benzoesäuremethylester und 14 g Ionenaustauscher (Dowex 50 WX2, Serva) wurde in 100 ml Methanol 12 h bei Raumtemperatur gerührt. Anschließend wurde das Lösungsmittel im Vakuum abdestilliert und der Rückstand an 100 g Kieselgel (0,04 - 0,06 mm) mit Gemischen aus Cyclohexan und Essigsäureethylester 100 : 1 bis 2 : 1 (v/v) gereinigt. Man erhält 6,1 g 2,4-Dichlor-3-(3'-hydroxy-3'-methyl-2-'oxetanyl)-benzoesäuremethylester.
1H-NMR (CDCl₃) δ [ppm]: 1,7 (s, 3H), 3,9 (s, 3H), 4,6 (d, 1H), 4,8 (d, 1H), 6,3 (s, 1H), 7,4 (d, 1H), 7,6 (d, 1H)
alternativ:
- Stufe c:: 2,4-Dichlor-3-(3'-hydroxy-3'-methyl-2'-oxetanyl)-benzoesäuremethylester (Verbindung 5.02)
Eine Lösung aus 1 g (0,003 M) 2,4-Dichlor-3-(3'-trimethylsilyloxy-3'-methyl-2'-oxetanyl)-benzoesäuremethylester und 5 ml 10 %iger methanolischer Lösung von Chlorwasserstoff wurde in 30 ml Methanol 12 h bei Raumtemperatur gerührt. Anschließend wurde das Lösungsmittel im Vakuum abdestilliert und der Rückstand in Diethylether aufgenommen. Diese etherische Lösung wurde mit Wasser neutral gewaschen, mit Natriumsulfat getrocknet, filtriert und das Lösungsmittel im Vakuum abdestilliert. Man erhält 0,7 g 2,4-Dichlor-3-(3'-hydroxy-3'-methyl-2'-oxethanyl)-benzoesäuremethylester.
1H-NMR (CDCl₃) δ [ppm] : 1,7 (s, 3H), 3,9 (s, 3H), 4,6 (d, 1H), 4,8 (d, 1H), 6,3 (s, 1H), 7,4 (d, 1H), 7,6 (d, 1H)
- Stufe d:: 2,4-Dichlor-3-(3'-hydroxy-3'-methyl-2'-oxetanyl)-benzoesäure (Verbindung 5.03)

Eine Lösung aus 4,9 g (0,013 M) 2,4-Dichlor-3-(3'-hydroxy-3'-methyl-2'-oxetanyl)-benzoesäuremethylester und 0,5 g (0,020 M) Lithiumhydroxid wurde in einem Gemisch aus 20 ml Tetrahydrofuran und 20 ml Wasser 12 h bei 0°C gerührt. Anschließend wurde die Lösung mit 10 %iger wäßriger Salzsäure auf pH 1-2 eingestellt und mit Diethylether extrahiert. Die vereinigten organischen Phasen wurden anschließend mit Natriumsulfat getrocknet, filtriert und das Lösungsmittel im Vakuum abdestilliert. Man erhält 3,5 g 2,4-Dichlor-3-(3'-hydroxy-3'-methyl-2'-oxetanyl)-benzoesäure.
1H-NMR (CDCl₃) δ [ppm]: 2,5 (s, 3H), 4,7 (d, 1H), 4,9 (d, 1H), 6,4 (s, 1H), 7,4 (d, 1H), 7,7 (d, 1H), 8,6 (breites s, 1H)

In der nachfolgenden Tabelle 109 sind neben den voranstehenden Verbindungen weitere Benzoesäurederivate der Formel Vd aufgeführt, die in analoger Weise hergestellt wurden oder herstellbar sind.

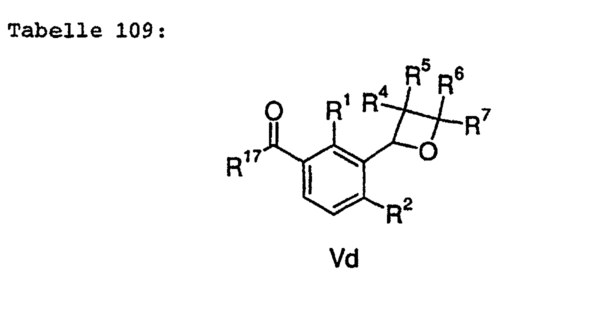

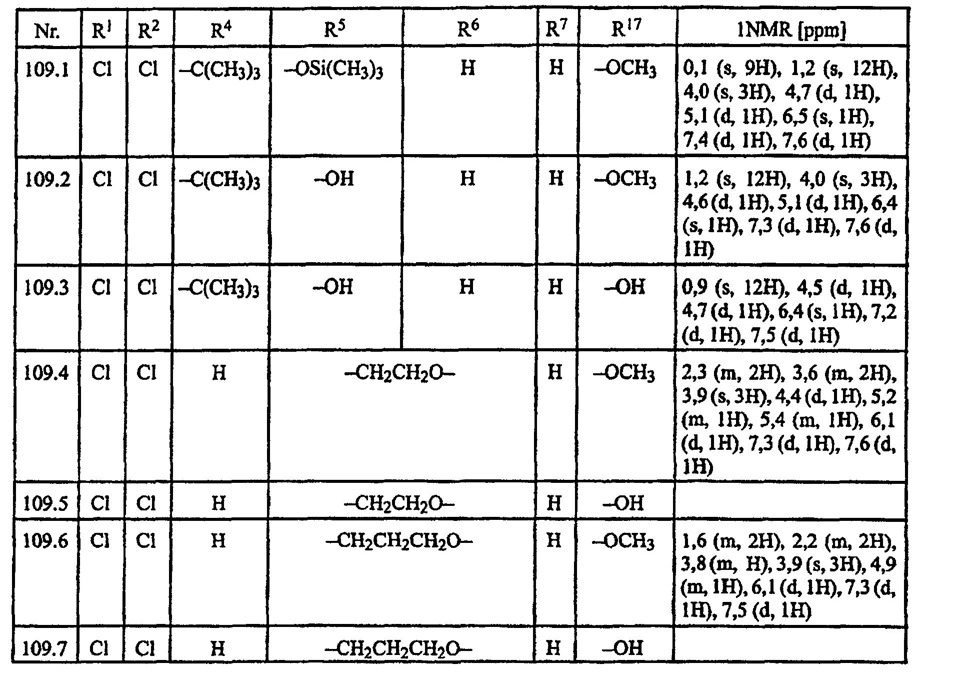

Herstellung der Endprodukte

4- (2',4'-Dichlor-3'-(3''-hydroxy-3''-methyl-2''-oxetanyl-benzoyl)-5,5-dimethyl-1,3-cyclohexandion (Verbindung 110.1)

Eine Lösung aus 0.8 g (0.003 M) 2,4-Dichlor-3-(3'-hydroxy-3'-methyl-2'-oxetanyl)-benzoesäure, 0,4 g (0,003 M) 5,5-Dimethyl-1,3-cyclohexandion und 0,7 g (0.003 M) Dicyclohexylcarbodiimid in 40 ml trockenem Acetonitril wurde 12 h bei Raumtemperatur gerührt. Anschließend wurde der Niederschlag abfiltriert und das Filtrat in wäßriger Kaliumcarbonat-Lösung aufgenommen. Nach Extraktion der wäßrigen Phase mit Essigsäureethylester wurde mit 10 %iger wäßriger Salzsäure ein pH-Wert von 2 eingestellt und nochmals mit Essigsäureethylester extrahiert. Die vereinigten organischen Phasen wurden mit Wasserneutral gewaschen, mit Natriumsulfat getrocknet, filtriert und das Lösungsmittel im Vakuum abdestilliert. Der Rückstand wurde an 50 g Kieselgel (0,04 - 0,06 mm) mit Gemischen aus Dichlormethan und Methanol 100 : 1 bis 10 : 1 (v/v) gereinigt. Man erhält 0,2 g 4-(2',4'-Dichlor-3'-(3''-hydroxy-3''-methyl-2''-oxetanyl-benzoyl) -5,5-dimethyl-1,3-cyclohexandion.

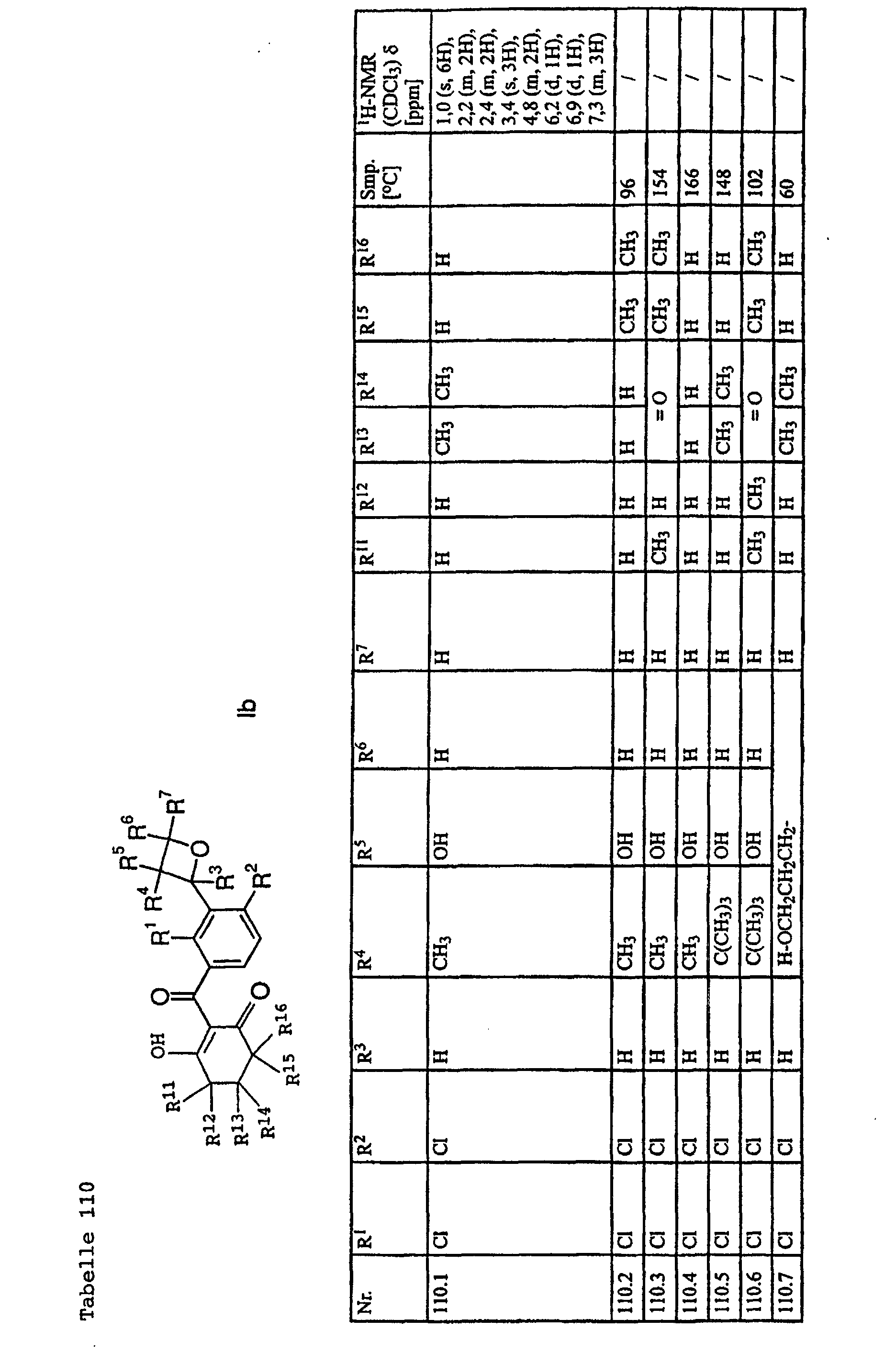

### Anwendungsbeispiele

Die herbizide Wirkung der substituierten 2-Benzoyl-cyclohexan-1,3-dione der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.5 bzw. 0.25 kg/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Die in den Gewächshausversuchen verwendeten Pflanzen setzten sich aus folgenden Arten zusammen:

| Lateinischer Name | Deutscher Name | Englischer Name | Abkürzungen |
|---|---|---|---|
| Echinochloa crus-galli | Hühnerhirse | barnyardgrass | ECHCG |
| Setaria viridis | Grüne Borstenhirse | green foxtail | SETVI |
| Chenopodium album | Weißer Gänsefuß | lambsquarters (goosefoot) | CHEAL |
| Polygonum persicaria | Flohknöterich | ladythumb | POLPE |
| Solanum nigrum | Schwarzer Nachtschatten | black nigthshade | SOLNI |

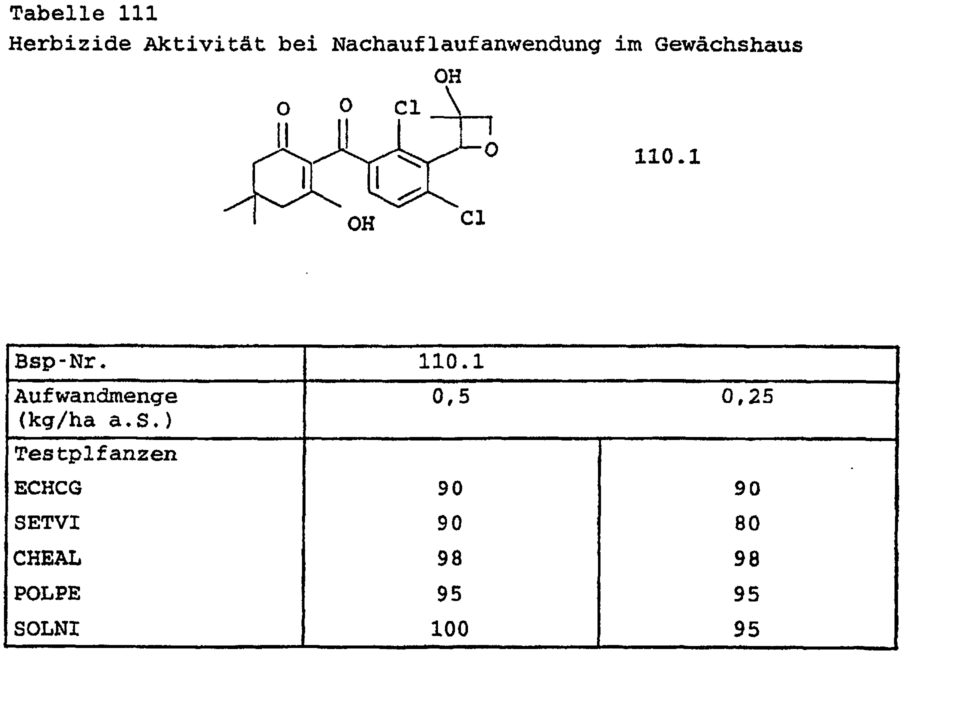

## Patentansprüche

1. 2-Benzoyl-cyclohexan-1,3-dione der Formel I 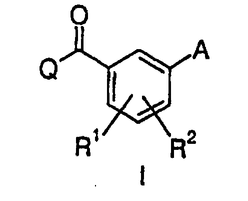 in der die Substituenten folgende Bedeutung haben:
R¹, R² Wasserstoff, Mercapto, Nitro, Halogen, Cyano, Rhodano, C₁-C₆-Alkyl, C₁-C₆-Halogenalkyl, C₁-C₆-Alkoxy, C₁-C₆-Alkyl, C₂-C₆-Alkenyl, C₂-C₆-Alkinyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ oder -NR¹⁰COR¹⁰;
Q ein gegebenenfalls substituierter, in 2-Stellung verknüpfter Cyclohexan-1,3-dion-Ring;
A eine Gruppe der Formel IIa, IIb oder III 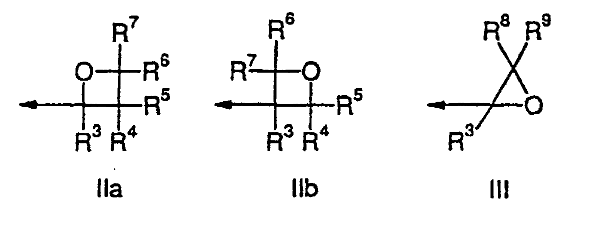 in der die Substituenten folgende Bedeutung haben:
R³ Wasserstoff, C₁-C₆-Alkyl oder Phenyl,
wobei der genannte Alkyl und Phenylrest partiell oder vollständig halogeniert sein kann und/oder eine bis drei der folgenden Gruppen tragen kann:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R⁴ - R⁷ können gleich oder verschieden sein und stehen unabhängig voneinander für:
Wasserstoff, Hydroxy, Mercapto, Amino, Halogen, C₁-C₆-Alkyl, C₃-C₆-Cycloalkyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰,
wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R⁴, R⁵ können zusammen eine gegebenenfalls durch ein Stickstoff- oder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ stehen kann;
R⁶, R⁷ können zusammen eine gegebenenfalls durch ein Stickstoff- oder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ stehen kann;
n null, eins, zwei;
R⁵, R⁶ können darüber hinaus, wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen;
R⁸, R⁹ können gleich oder verschieden sein und stehen unabhängig voneinander für:
Wasserstoff, Nitro, Halogen, Cyano, C₁-C₆-Alkyl, C₃-C₆-Cycloalkyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünf- oder sechsgliedriges Hetaryl,
wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R⁸, R⁹ können darüber hinaus zusammen eine gegebenenfalls durch ein Stickstoff- oder ein Sauerstoffatom einoder zweifach unterbrochene C₂-C₅-Alkylen- oder C₂-C₅-Alkenylenkette bilden;
R¹⁰ Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
sowie deren landwirtschaftlich brauchbaren Salze.

2. 2-Benzoyl-cyclohexan-1,3-dione der Formel I nach Anspruch 1, in der Q ein in 2-Stellung verknüpfter Cyclohexan-1,3-dion-Ring der Formel IV 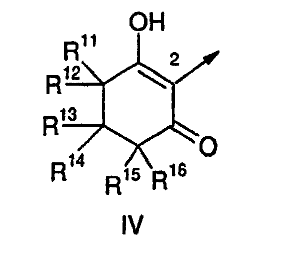 ist, wobei R¹¹, R¹², R¹⁴ und R¹⁶ für Wasserstoff oder C₁-C₄-Alkyl stehen;
R¹³ für Wasserstoff, C₁-C₄-Alkyl oder C₃-C₄-Cycloalkyl steht, wobei die beiden letztgenannten Gruppen einen bis drei der folgenden Substituenten tragen können: Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
R¹⁵ für Wasserstoff, C₁-C₄-Alkyl oder C₁-C₆-Alkoxycarbonyl steht:
oder
R¹³ und R¹⁶ gemeinsam eine π-Bindung oder einen drei- bis sechsgliedrigen carbocyclischen Ring bilden;
oder
die CR¹³R¹⁴-Einheit durch C=O ersetzt sein kann.

3. 2-Benzoyl-cyclohexan-1,3-dione der Formel I nach Anspruch 1 oder 2, in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR¹⁰ oder -S(O)ₙR¹⁰ bedeutet;
R² für Wasserstoff oder einen wie voranstehend unter R¹ genannten Rest steht.

4. 2-Benzoyl-cyclohexan-1,3-dione der Formel Ia nach einem der Ansprüche 1 bis 3, 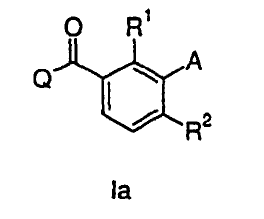 in der die Substituenten R¹, R², Q und A die unter den Ansprüchen 1 bis 3 genannte Bedeutung haben.

5. 2-Benzoyl-cyclohexan-1,3-dione der Formel Ia nach Anspruch 3, in der A eine Gruppe der Formel IIa oder IIb darstellt.

6. 2-Benzoyl-cyclohexan-1,3-dione der Formel Ia nach Anspruch 3, in der A eine Gruppe der Formel III darstellt.

7. Verfahren zur Herstellung von 2-Benzoyl-cyclohexan-1,3-dionen der Formel I gemäß den Ansprüchen 1 bis 6, **dadurch gekennzeichnet, daß** man ein gegebenenfalls substituiertes Cyclohexan-1,3-dion Q mit einer aktivierten Carbonsäure Va oder mit einer Carbonsäure Vb, 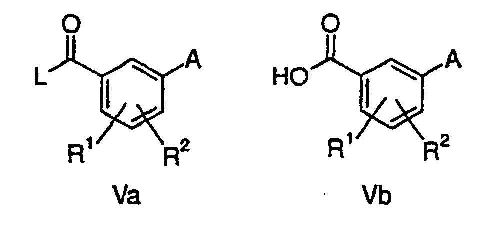 wobei die Substituenten R¹, R² und A die unter Anspruch 1 genannte Bedeutung haben und L für eine nucleophil austauschbarebare Abgangsgruppe steht, acyliert und das Acylierungsprodukt gegebenenfalls in Gegenwart eines Katalysators zu den Verbindungen I umlagert.

8. Mittel, enthaltend eine herbizid wirksame Menge mindestens einer Verbindung der Formel I oder eines landwirtschaftlich brauchbaren Salzes von I, gemäß den Ansprüchen 1 bis 6, und für die Formulierung von Pflanzenschutzmitteln übliche Hilfsmittel.

9. Verfahren zur Herstellung von herbizid wirksamen Mitteln gemäß Anspruch 8, **dadurch gekennzeichnet, daß** man eine herbizid wirksame Menge mindestens einer Verbindung der Formel I oder eines landwirtschaftlich brauchbaren Salzes von I gemäß den Ansprüchen 1 bis 6 und für die Formulierung von Pflanzenschutzmitteln übliche Hilfsmittel mischt.

10. Verfahren zur Bekämpfung von unerwünschtem Pflanzenwuchs, **dadurch gekennzeichnet, daß** man eine herbizid wirksame Menge mindestens einer Verbindung der Formel I oder eines landwirtschaftlich brauchbaren Salzes von I gemäß den Ansprüchen 1 bis 6 auf Pflanzen, deren Lebensraum und/ oder auf Samen einwirken läßt.

11. Verwendung der Verbindungen der Formel I und deren landwirtschaftlich brauchbaren Salze gemäß den Ansprüchen 1 bis 6 als Herbizide.

## Claims

1. A 2-benzoylcyclohexane-1,3-dione of the formula I 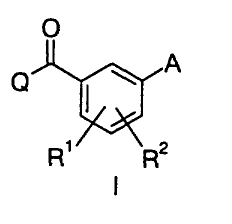 where:
R¹ and R² are each hydrogen, mercapto, nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ or -NR¹⁰COR¹⁰;
Q is a cyclohexane-1,3-dione ring with or without substitution, which is attached in position 2;
A is a group of the formula IIa, IIb or III 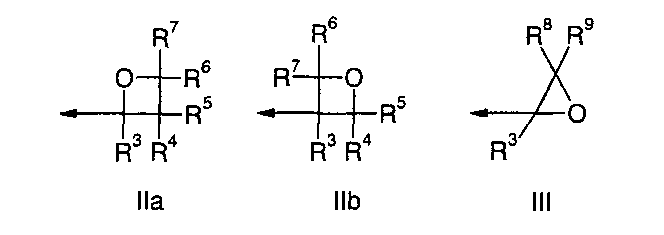
where:
R³ is hydrogen, C₁-C₆-alkyl or phenyl,
where the alkyl and phenyl radicals mentioned may be partially or fully halogenated and/or may carry one to three of the following groups: hydroxyl, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals mentioned may in turn be substituted;
R⁴- R⁷ may be identical or different and, independently of each other, are:
hydrogen, hydroxyl, mercapto, amino, halogen, C₁-C₆-alkyl, C₃-C₆-cycloalkyl, phenyl, -OR¹⁰, -S(O)ₙR¹⁰, -OS(O)ₙR¹⁰, -PO(OR¹⁰)₂, -NR³R¹⁰, -Si(R¹⁰)₃ or -OCOR¹⁰,
where the alkyl and cycloalkyl radicals mentioned and R³ and R¹⁰ of the radicals -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ may be partially or fully halogenated and/or may carry one to three of the following groups:
hydroxyl, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals mentioned may in turn be substituted;
R⁴ and R⁵ together may form a C₂-C₅-alkylene or C₂-C₅-alkenylene chain which may be interrupted once or twice by a nitrogen or an oxygen atom, or may form a group =X, where X is an oxygen atom or a group CR³R¹⁰, NR¹⁰ or NOR¹⁰;
R⁶ and R⁷ together may form a C₂-C₅-alkylene or C₂-C₅-alkenylene chain which may be interrupted once or twice by a nitrogen or an oxygen atom, or may form a group =X, where X is an oxygen atom or a group CR³R¹⁰, NR¹⁰ or NOR¹⁰;
n is zero, one or two;
R⁵ and R⁶ together may furthermore, if they are attached to adjacent carbon atoms and if R⁴ and R⁷ are each hydrogen, form a C₃-C₄-alkylene or C₃-C₄-alkenylene chain which may be interrupted by a nitrogen or an oxygen atom;
R⁸ and R⁹ may be identical or different and, independently of each other, are:
hydrogen, nitro, halogen, cyano, C₁-C₆-alkyl, C₃-C₆-cycloalkyl, C₅-C₆-heterocyclyl, -OR¹⁰, -SR¹⁰, -COR¹⁰, -COOR¹⁰, -CONR³R¹⁰, phenyl, phenyl-C₁-C₆-alkyl and five- or six-membered hetaryl,
where the alkyl and cycloalkyl radicals mentioned and R³ and R¹⁰ of the radicals -OR¹⁰, -SR¹⁰, -COR¹⁰, -COOR¹⁰, -CONR³R¹⁰ may be partially or fully halogenated and/or may carry one to three of the following groups:
hydroxyl, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals mentioned may in turn be substituted;
R⁸ and R⁹ together may furthermore form a C₂-C₅-alkylene or C₂-C₅-alkenylene chain which may be interrupted once or twice by a nitrogen or an oxygen atom;
R¹⁰ is hydrogen, C₁-C₆-alkyl, C₁-C₆-haloalkyl, C₂-C₆-alkenyl, C₂-C₆-alkynyl, phenyl or phenyl-C₁-C₆-alkyl; where the alkyl radicals mentioned may be partially or fully halogenated and/or may carry one to three of the following groups:
hydroxyl, mercapto, amino, cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals mentioned may in turn be substituted;
and agriculturally useful salts thereof.

2. A 2-benzoylcyclohexane-1,3-dione of the formula I as claimed in claim 1 in which Q is a cyclohexane-1,3-dione ring of the formula IV 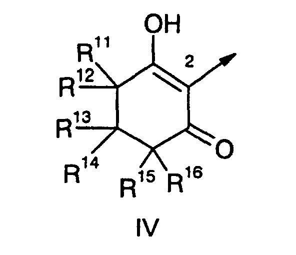 which is attached in position 2, where R¹¹, R¹², R¹⁴ and R¹⁶ are each hydrogen or C₁-C₄-alkyl;
R¹³ is hydrogen, C₁-C₄-alkyl or C₃-C₄-cycloalkyl, where the last two groups mentioned may carry one to three of the following substituents: halogen, C₁-C₄-alkylthio or C₁-C₄-alkoxy;
or
is tetrahydropyran-2-yl, tetrahydropyran-3-yl, tetrahydropyran-4-yl, tetrahydrothiopyran-2-yl, tetrahydrothiopyran-3-yl, tetrahydrothiopyran-4-yl, 1,3-dioxolan-2-yl, 1,3-dioxan-2-yl, 1,3-oxathiolan-2-yl, 1,3-oxathian-2-yl, 1,3-dithiolan-2-yl or 1,3-dithian-2-yl, where the last 6 radicals mentioned may be substituted by one to three C₁-C₄-alkyl radicals;
R¹⁵ is hydrogen, C₁-C₄-alkyl or C₁-C₆-alkoxycarbonyl;
or
R¹³ and R¹⁶ together form a π bond or a three- to six-membered carbocyclic ring;
or
the CR¹³R¹⁴ unit may be replaced by C=O.

3. A 2-benzoylcyclohexane-1,3-dione of the formula I as claimed in claim 1 or 2 in which
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR¹⁰ or -S(O)ₙR¹⁰;
R² is hydrogen or one of the radicals mentioned above under R¹.

4. A 2-benzoylcyclohexane-1,3-dione of the formula Ia as claimed in any of claims 1 to 3, 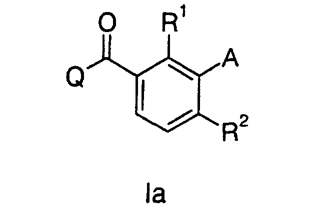 where the substituents R¹, R², Q and A are each as defined in claims 1 to 3.

5. A 2-benzoylcyclohexane-1,3-dione of the formula Ia as claimed in claim 3 in which A is a group of the formula IIa or IIb.

6. A 2-benzoylcyclohexane-1,3-dione of the formula Ia as claimed in claim 3 in which A is a group of the formula III.

7. A process for preparing the 2-benzoylcyclohexane-1,3-dione of the formula I as claimed in any of claims 1 to 6, which comprises acylating a cyclohexane-1,3-dione Q with or without substitution with an activated carboxylic acid Va or with a carboxylic acid Vb 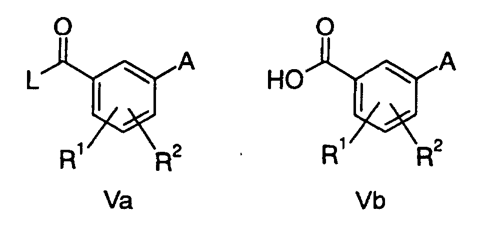 where the substituents R¹, R² and A are each as defined under claim 1 and L is a nucleophilically exchangeable leaving group, and rearranging the acylation product, if appropriate in the presence of a catalyst, to give the compounds I.

8. A composition, which comprises a herbicidally active amount of at least one compound of the formula I or an agriculturally useful salt of I as claimed in any of claims 1 to 6 and auxiliaries which are customarily used for formulating crop protection agents.

9. A process for preparing herbicidally active compositions as claimed in claim 8, which comprises mixing a herbicidally effective amount of at least one compound of the formula I or an agriculturally useful salt of I as claimed in any of claims 1 to 6 and auxiliaries which are customarily used for formulating crop protection agents.

10. A method for controlling undesirable vegetation, which comprises allowing a herbicidally effective amount of at least one compound of the formula I or an agriculturally useful salt of I as claimed in any of claims 1 to 6 to act on plants, their habitat and/or on seeds.

11. The use of the compounds of the formula I and agriculturally useful salts thereof as claimed in any of claims 1 to 6 as herbicides.

## Revendications

1. 2-benzoyl-cyclohexane-1,3-diones de formule I 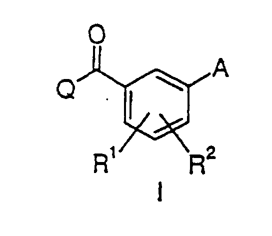 dans laquelle les substituants ont la signification suivante:
R¹, R² hydrogène, mercapto, nitro, halogène, cyano, rhodano, alkyle en C₁-C₆, halogénoalkyle en C₁-C₆, alcoxy en C₁-C₆, alkyle en C₁-C₆, alcényle en C₂-C₆, alcynyle en C₂-C₆, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ ou -NR¹⁰COR¹⁰;
Q un cycle cyclohexane-1,3-dione lié en position 2, éventuellement substitué;
A un groupe de formule IIa, IIb ou III 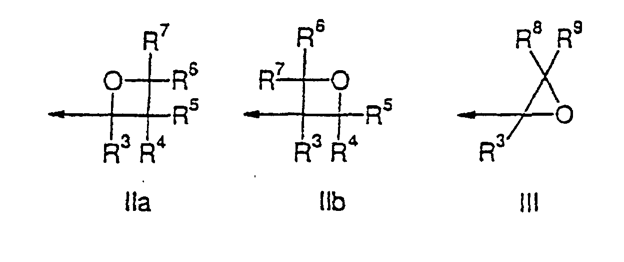 où les substituants ont la signification suivante:
R³ hydrogène, alkyle en C₁-C₆ ou phényle, tandis que le reste alkyle et phényle mentionné peut être partiellement ou complètement halogéné et/ou peut porter un à trois des groupes suivants:
hydroxy, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, alkyl(C₁-C₄)iminooxy, alcoxy(C₁-C₄)amino, alkyl(C₁-C₄)carbonyle, alcoxy(C₁-C₄)alcoxycarbonyle(C₂-C₆), alkyl(C₁-C₄)sulfonyle, hétérocyclyle, hétérocyclyloxy, phényle, benzyle, hétaryle, phénoxy, benzyloxy et hétaryloxy, tandis que les huit restes mentionnés en dernier peuvent à leur tour être substitués;
R⁴ - R⁷ peuvent être identiques ou différents et désignent, indépendamment l'un de l'autre:
hydrogène, hydroxy, mercapto, amino, halogène, alkyle en C₁-C₆, cycloalkyle en C₃-C₆, phényle, -OR¹⁰, -S(O)ₙR¹⁰, -OS(O)ₙR¹⁰, -PO(OR¹⁰)₂, -NR³R¹⁰, -Si(R¹⁰)₃ ou -OCOR¹⁰, tandis que les restes alkyle et cycloalkyle mentionnés, ainsi que R³ et R¹⁰ des restes -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ peuvent être partiellement ou complètement halogénés et/ou peuvent porter un à trois des groupes suivants: hydroxy, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, alkyl(C₁-C₄)iminooxy, alcoxy(C₁-C₄)amino, alkyl(C₁-C₄)carbonyle, alcoxy(C₁-C₄)alcoxycarbonyle(C₂-C₆), alkyl(C₁-C₄)sulfonyle, hétérocyclyle, hétérocyclyloxy, phényle, benzyle, hétaryle, phénoxy, benzyloxy, et hétaryloxy, tandis que les huit restes cités en dernier peuvent à leur tour être substitués;
R⁴, R⁵ peuvent former ensemble une chaîne alkylène en C₂-C₅ ou alcénylène en C₂-C₅ éventuellement interrompue une ou deux fois par un atome d'azote ou un atome d'oxygène, ou un groupe =X, tandis que X peut désigner un atome d'oxygène ou un groupe CR³R¹⁰, NR¹⁰ ou NOR¹⁰;
R⁶, R⁷ peuvent former ensemble une chaîne alkylène en C₂-C₅ ou alcénylène en C₂-C₅ éventuellement interrompue une ou deux fois par un atome d'azote ou un atome d'oxygène, ou un groupe =X, tandis que X peut désigner un atome d'oxygène ou un groupe CR³R¹⁰, NR¹⁰ ou NOR¹⁰;
n vaut zéro, un, deux;
R⁵, R⁶ peuvent en outre, quand ils sont liés à des atomes de carbone adjacents, former ensemble une chaîne alkylène en C₃-C₄ ou alcénylène en C₃-C₄ éventuellement interrompue par un atome d'azote ou un atome d'oxygène, quand R⁴ et R⁷ représentent l'hydrogène;
R⁸, R⁹ peuvent être identiques ou différents et désignent indépendamment l'un de l'autre:
hydrogène, nitro, halogène, cyano, alkyle en C₁-C₆, cycloalkyle en C₃-C₆, hétérocyclyle en C₅-C₆, -OR¹⁰, -SR¹⁰, -COR¹⁰, -COOR¹⁰, -CONR³R¹⁰, phényle, phényl-alkyle(C₁-C₆), et hétaryle à cinq ou six chaînons,
tandis que les restes alkyle et cycloalkyle mentionnés, ainsi que R³ et R¹⁰ des restes - OR¹⁰, -SR¹⁰, -COR¹⁰, -COOR¹⁰, -CONR³R¹⁰, peuvent être partiellement ou complètement halogénés et/ou peuvent porter un à trois des groupes suivants:
hydroxy, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, alkyl(C₁-C₄)iminooxy, alcoxy(C₁-C₄)amino, alkyl(C₁-C₄)carbonyle, alcoxy(C₁-C₄)alcoxycarbonyle-(C₂-C₆), alkyl(C₁-C₄)sulfonyle, hétérocyclyle, hétérocyclyloxy, phényle, benzyle, hétaryle, phénoxy, benzyloxy, et hétaryloxy, tandis que les huit restes cités en dernier peuvent à leur tour être substitués;
R⁸, R⁹ peuvent en outre former ensemble une chaîne alkylène en C₂-C₅ ou alcénylène en C₂-C₅ éventuellement interrompue une ou deux fois par un atome d'azote ou un atome d'oxygène;
R¹⁰ hydrogène, alkyle en C₁-C₆, halogénoalkyle en C₁-C₆, alcényle en C₂-C₆, alcynyle en C₂-C₆, phényle ou phényl-alkyle(C₁-C₆); tandis que les restes alkyle mentionnés peuvent être partiellement ou complètement halogénés et/ou peuvent porter un à trois des groupes suivants:
hydroxy, mercapto, amino, cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, alkyl(C₁-C₄)iminooxy, alcoxy(C₁-C₄)amino, alkyl(C₁-C₄)carbonyle, alcoxy(C₁-C₄)alcoxycarbonyle(C₂-C₆), alkyl(C₁-C₄)sulfonyle, hétérocyclyle, hétérocyclyloxy, phényle, benzyle, hétaryle, phénoxy, benzyloxy, et hétaryloxy, tandis que les huit restes cités en dernier peuvent à leur tour être substitués;
ainsi que leurs sels utilisables en agriculture.

2. 2-Benzoyl-cyclohexane-1,3-diones de formule I selon la revendication 1, où Q est un cycle cyclohexane-1,3-dione lié en position 2, de formule IV, 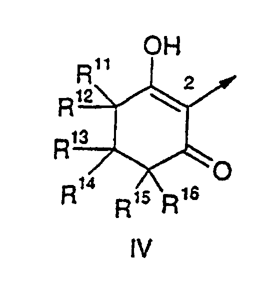 tandis que R¹¹, R¹², R¹⁴ et R¹⁶ désignent l'hydrogène ou un alkyle en C₁-C₄;
R¹³ représente l'hydrogène un alkyle en C₁-C₄ ou un cycloalkyle en C₃-C₄, tandis que les deux groupes mentionnés en dernier peuvent porter un à trois des substituants suivants: halogène, alkyl(C₁-C₄)thio ou alcoxy en C₁-C₄;
ou
désigne un groupe tétrahydropyranne-2-yle, tétrahydropyranne-3-yle, tétrahydropyranne-4-yle, tétrahydrothiopyranne-2-yle, tétrahydrothiopyranne-3-yle, tétrahydrothiopyranne-4-yle, 1,3-dioxolanne-2-yle, 1,3-dioxanne-2-yle, 1,3-oxathiolanne-2-yle, 1,3-oxathianne-2-yle, 1,3-dithiolanne-2-yle ou 1,3-dithianne-2-yle, tandis que les 6 restes mentionnés en dernier peuvent être substitués par un à trois restes alkyle en C₁-C₄;
R¹⁵ désigne l'hydrogène, un alkyle en C₁-C₄ ou un alcoxy(C₁-C₆)carbonyle;
ou
R¹³ et R¹⁶ forment ensemble une liaison πou un cycle carbocyclique ayant trois à six chaînons;
ou
l'unité CR¹³R¹⁴ peut être remplacée par C=O.

3. 2-Benzoyl-cyclohexane-1,3-diones de formule I selon la revendication 1 ou 2, où
R¹ représente un nitro, halogène, cyano, rhodano, alkyle en C₁-C₆, halogénoalkyle en C₁-C₆, alcoxy(C₁-C₆)alkyle(C₁-C₆), alcényle en C₂-C₆, alcynyle en C₂-C₆, -OR¹⁰ ou -S(O)ₙR¹⁰;
R² désigne l'hydrogène ou un reste tel qu'indiqué plus haut sous R¹.

4. 2-Benzoyl-cyclohexane-1,3-diones de formule la selon l'une des revendications 1 à 3, 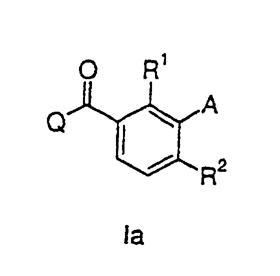 où les substituants R¹, R², Q et A ont la signification indiquée dans les revendications 1 à 3.

5. 2-Benzoyl-cyclohexane-1,3-diones de formule Ia selon la revendication 3, où A représente un groupe de formule IIa ou IIb.

6. 2-Benzoyl-cyclohexane-1,3-diones de formule Ia selon la revendication 3, où A représente un groupe de formule III.

7. Procédé pour la préparation de 2-Benzoyl-cyclohexane-1,3-diones de formule I selon les revendications 1 à 6, **caractérisé par le fait qu'**on acyle une cyclohexane-1,3-dione Q, éventuellement substituée, avec un acide carboxylique activé Va ou avec un acide carboxylique Vb, 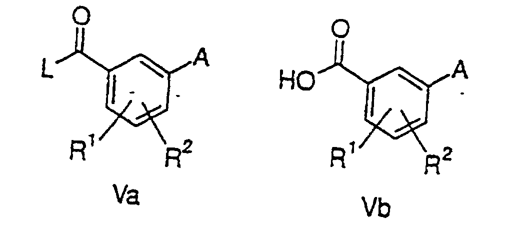 tandis que les substituants R¹, R² et A ont la signification indiquée dans la revendication 1 et L désigne un groupe séparable, échangeable par voie nucléophile, et on convertit le produit d'acylation en les composés I, éventuellement en présence d'un catalyseur.

8. Produit, contenant une quantité à activité herbicide d'au moins un composé de formule I ou d'un sel utilisable en agriculture de I, selon les revendications 1 à 6, et des adjuvants usuels pour la formulation de produits phytosanitaires.

9. Procédé pour la préparation de produits à activité herbicide selon la revendication 8, **caractérisé par le fait qu'**on mélange une quantité à activité herbicide d'au moins un composé de formule I ou d'un sel utilisable en agriculture de I, selon les revendications 1 à 6, et des adjuvants usuels pour la formulation de produits phytosanitaires.

10. Procédé pour la lutte contre la croissance non-souhaitée de plantes, **caractérisé par le fait qu'**on fait agir une quantité à activité herbicide d'au moins un composé de formule I ou d'un sel utilisable en agriculture de I, selon les revendications 1 à 6, sur des plantes, leur biotope et/ou sur des semences.

11. Utilisation des composés de formule I et de leurs sels utilisables en agriculture selon les revendications 1 à 6 comme herbicides.
